# EUROPEAN PATENT SPECIFICATION

(11) **EP 1 441 724 B1**
(45) Date of publication and mention of the grant of the patent: **05.09.2007**
(21) Application number: 02766382.2
(22) Date of filing: 27.09.2002
(51) Int. Cl.: A61K 31/415, A61K 31/4439, A61K 31/4709, A61K 31/4725, A61K 31/4545, A61K 31/4155, A61K 31/497, A61P 5/26, A61P 15/08

(54) **METHODS OF INCREASING ENDOGENOUS TESTOSTERONE LEVELS**
VERFAHREN ZUR ERHÖHUNG DES TESTOSTERONSPIEGELS
METHODES POUVANT AUGMENTER DES CONCENTRATIONS EN TESTOSTERONE ENDOGENE

(30) Priority: 27.09.2001 US 325470 P
(43) Date of publication of application: 04.08.2004
(73) Proprietor: Applied Research Systems ARS Holding N.V., Curaçao (AN)
(72) Inventor: BRONDYK, William, H., Mansfield, MA 02048 (US); MCKENNA, Sean, Duxbury, MA 02332 (US); ARKINSTALL, Stephen, J., Belmont, MA 02478 (US)
(74) Representative: Merck Serono International S.A. Intellectual Property
(86) International application number: PCT/US2002/030801
(87) International publication number: WO 2003/026649

(56) References cited:
- WO-A-00/66612
- WO-A-01/87287
- WO-A-02/18346
- WO-A-94/13661
- WO-A-95/13069
- WO-A-98/52941
- WO-A-99/23091
- DATABASE BIOSIS [Online] BIOSCIENCES INFORMATION SERVICE, PHILADELPHIA, PA, US; 1983 SANTUCCI L ET AL: "INHIBITION OF TESTOSTERONE PRODUCTION BY RAT LEYDIG CELLS WITH ETHANOL AND ACETALDEHYDE PREVENTION OF ETHANOL TOXICITY WITH 4 METHYL PYRAZOLE" Database accession no. PREV198477023774 XP002229750 & ALCOHOLISM CLINICAL AND EXPERIMENTAL RESEARCH, vol. 7, no. 2, 1983, pages 135-139, ISSN: 0145-6008
- JUNIEWICZ P E ET AL: "EFFECT OF COMBINATION TREATMENT WITH ZANOTERONE (WIN 49596), A STEROIDAL ANDROGEN RECEPTOR ANTAGONIST, AND FINASTERIDE (MK-906), A STEROIDAL 5ALPHA-REDUCTASE INHIBITOR, ON THE PROSTATE AND TESTES OF BEAGLE DOGS" ENDOCRINOLOGY, BALTIMORE, MD, US, vol. 133, no. 2, 1993, pages 904-913, XP008012035 ISSN: 0013-7227
- MAKLAD ET AL.: "Androgenic adnd anabolic activities of some newly synthetized epiandrosterone and progesterone derivatives" SCIENTIA PHARMACEUTICA, vol. 68, no. 2, 30 June 2000 (2000-06-30), pages 141-157, XP008013051

## Description

### BACKGROUND OF THE INVENTION

### 1. Field of the Invention

The present invention relates to the use of substituted pyrazole compounds to increase endogenous testosterone production. Compounds of the invention are useful for the treatment of conditions, disorders or diseases which would benefit patients by increasing endogenous testosterone levels.

### 2. Background

Testosterone is the most important representative of the male sex hormones collectively called androgens. Using cholesterol as a base, the male gonads (testes) produce between 4 and 10 mg of testosterone per day. Gonadotropin Releasing Hormone (GnRH) produced by the hypothalamus stimulates the release of gonadotropins, luteinizing hormone (LH) and follicle stimulating hormone (FSH). In the testes, Leydig cells synthesize and secrete testosterone in response to LH, which is counter-regulated by feedback influences of testosterone and its metabolites. During puberty, testosterone levels are at their lifetime peak. They begin to decline around the age of 23.

Testosterone is responsible for three major functions in animals.
1) The development of secondary male sex characteristics also called the androgenic functions of testosterone. Some examples of these characteristics are increased growth of body hair, beard growth, deep voice, increased production of sebaceous glands, development of the penis, aggressiveness, sexual behavior, libido, and the maturation of sperm.
2) Promotion of the protein biosynthesis that are responsible for the highly anabolic characteristics of testosterone. It accelerates muscle buildup, increases the formation of red blood cells, speeds up regeneration, and speeds up recovery time after injuries or illness. It also stimulates the entire metabolism which results in the burning of bodyfat.
3) Inhibition of the gonad regulating cycle, including the hypothalamohypophysial testicular axis, which regulates the amount of testosterone produced in the organism If the testosterone level in the blood is high, the testes will signal the hypothalymus to release less LHRH (leutenizing hormone releasing hormone). Thus the hypophysis releases less gonadotropin. Consequently, the Leydig's cells in the testes reduces the production of testosterone. In other words, if you have too much testosterone, your body will tell itself to reduce or even stop production of it until it is back down to its normal levels.

Male hypogonadism with a deficiency of testosterone is a relatively common disorder in clinical practice and has significant effects on the fertility, sexual function, and general health of patients. Hypogonadism can be caused by disorders of the testes (primary), pituitary (secondary), or the hypothalamus (tertiary). Testosterone deficiency may occur as a result of Leydig cell dysfunction from primary disease of the testes, or insufficient LH secretion from diseases of the pituitary, or insufficient GnRH secretion from the hypothalamus. Pinpointing the cause of testosterone deficiency with laboratory testing makes it possible to tailor successful replacement therapy for men with androgen deficiency. Some causes of this disorder are relatively common while others are rare. Klinefelter's syndrome, for example, occurs in about 1 in 500 men; it is a primary testicular disorder that results in both androgen deficiency and infertility. In men with clinical symptoms of primary or secondary hypogonadism, deficiency of testosterone can be treated with hormone replacement. Infertility in men with primary testicular disease of the seminiferous tubules, such as Klinefelter's syndrome, is irreversible.

Hormone replacement for management of male hypogonadism depends upon both the cause and the stage of sexual development of the patient. In prepubertal boys and men with either primary or secondary hypogonadism, androgen replacement therapy is indicated to stimulate and sustain normal secondary sexual characteristics, sexual function, and behavior. Several options for replacement therapy are available and these include administration by injection, oral delivery and transdermal patches. The ultimate goal is to safely normalize physiology, with comfort to the patient at the lowest possible cost.

Injection of testosterone esters--testosterone enanthate or cypionate--are effective, safe, and relatively inexpensive androgen preparations, but they produce early supraphysiologic concentrations of testosterone and may be more prone to cause gynecomastia and polycythemias than transdermal testosterone systems. Transdermal testosterone delivery results in more physiologic testosterone and estradiol concentrations with a circadian variation. The transdermal systems are more expensive than testosterone ester preparations, however. Transdermal systems allow monitoring of therapeutic response through assessment of testosterone concentrations about 12 hours after application. The scrotal patch has fewer skin reactions than the non-scrotal patch, but results in supraphysiologic concentrations of DHT. Oral and sublingual preparations produce widely fluctuating testosterone concentrations. The oral derivative of testosterone results in unpredictable concentrations of testosterone and may cause hepatic toxicity and reduce HDL-cholesterol more dramatically than pre-testosterone formulations.

When testosterone replacement therapy is indicated, a safe general principle is to mimic the normal concentrations of testosterone (350-1050 ng/dL) and its active metabolites; thus avoiding unphysiologically high testosterone serum concentrations to prevent possible side effects or low concentrations to prevent androgen deficiency. When these goals of therapy are met, physiological responses to androgen replacement therapy can be expected allowing virilization in prepuberal males and restoration or preservation of virilization in postpuberal men. The treatment should not have untoward effects on the prostate, serum lipids, or cardiovascular, liver, and lung function; should allow self-administration, be convenient, cause minimal discomfort, and be affordable. None of the currently available androgen replacement therapies achieves the ideal.

It thus would be desirable to have new agents and methods to treat conditions, disorders or diseases in patients who would benefit by increasing endogenous levels of testosterone.

### SUMMARY OF THE INVENTION

We have now found that substituted pyrazole compounds are potent agents at increasing endogenous testosterone levels. Compounds of the invention are particularly useful for treatment of male hypogonadism

Pyrazole compounds of the invention are substituted by other than hydrogen in one or more pyrazole ring positions, and preferably are substituted at the 1, 3, 4 and/or 5 ring positions by a non-hydrogen substituent. Typical pyrazole compounds of the invention are substituted at least at the 5-ring position by other than hydrogen.

For example, described are substituted pyrazole compounds of the following Formula I: 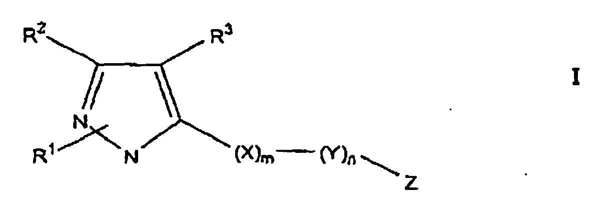 wherein
R¹ is hydrogen; optionally substituted alkyl preferably having 1 to about 20 carbons, more preferably 1 to about 12 carbons, optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms; optionally substituted aralkyl having at least about 6 ring carbon atoms ; optionally substituted heteroaromatic or heteroalicyclic group having 1 to 3 rings, 3 to 8 ring members in each ring and from 1 to 3 hetero atoms (N, O or S); or optionally substituted heteroaralkyl or heteroalicyclicalkyl group having from 1 to 3 rings, 3 to 8 ring members in each ring and from 1 to 3 hetero atoms (N, O or S);
R² and R³ are each independently hydrogen, halogen, optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted alkoxy preferably having from 1 to about 20 carbon atoms, more preferably 1 to about 12 carbon atoms; optionally substituted alkylthio preferably having from 1 to about 20 carbon atoms, more preferably 1 to about 12 carbon atoms; optionally substituted alkylsulfinyl preferably having from 1 to about 20 carbon atoms, more preferably 1 to about 12 carbon atoms; optionally substituted alkylsulfonyl preferably having from 1 to about 20 carbon atoms, more preferably 1 to about 12 carbon atoms; optionally substituted carbocyclic aryl having at least about 6 ring carbon atoms; optionally substituted aralkyl having at least about 6 ring carbon atoms; optionally substituted heteroaromatic or heteroalicyclic group having from 1 to 3 rings, 3 to 8 ring members in each ring and from 1 to 3 hetero atoms (particularly 1-3 N, O and/or S atoms); or optionally substituted heteroaralkyl or heteroalicyclicalkyl group having from 1 to 3 rings, 3 to 8 ring members in each ring and from 1 to 3 hetero atoms (particularly 1-3 N, O and/or S atoms);
wherein preferably at least one of R¹, R² and R³ are other than hydrogen and more preferably at least two of R¹, R² and R³ are other than hydrogen;
X is optionally substituted alkylene preferably having 1 to about 12 chain carbons, more preferably 2 to about 8 chain carbons, still more preferably 3 to about 6 alkylene chain carbons; optionally substituted alkenylene preferably having 2 to about 12 chain carbons, more preferably 2 to about 8 chain carbons, still more preferably 3 to about 6 alkenylene chain carbons; optionally substituted alkynylene preferably having 2 to about 12 chain carbons, more preferably 2 to about 8 chain carbons, still more preferably 3 to about 6 alkynylene chain carbons; optionally substituted heteroalkylene preferably having 1 to about 12 chain carbons, more preferably 2 to about 8 chain carbons, still more preferably 3 to about 6 heteroalkylene chain carbons and a total of 4 or 5 atoms in the heteroalkylene chain inclusive of hetero atoms (particularly N, O and/or S atoms); optionally substituted heteroalkenylene preferably having 2 to about 12 chain carbons, more preferably 2 to about 8 chain carbons, still more preferably 3 to about 6 heteroalkenylene chain carbons and a total of 4 or 5 atoms in the heteroalkenylene chain (particularly N, 0 and/or S atoms); optionally substituted heteroalkynylene preferably having 2 to about 12 chain carbons, more preferably 2 to about 8 chain carbons, still more preferably 4 or 5 heteroalkynylene chain carbons and a total of 4 or 5 atoms in the heteroalkynylene chain inclusive of hetero atoms (particularly N, O and/or S atoms); or X is optionally alicyclic, optionally substituted carbocyclic aryl; optionally substituted heteroalicyclic, optionally substituted heteroaromatic, optionally substituted heteroaralkyl, or optionally substituted heteroalicyclicalkyl group, each preferably having 3-10 carbon or hetero atoms in a ring, more preferably 5 or 6 membered ring(s), and 1-3 N, O or S atoms;
Y is optionally substituted amino; optionally substituted methylene (e.g. unsubstituted methylene, CH₂), carbonyl (C=O); or sulfonyl (SO₂);
Z is an optionally substituted alkylamine; an amino acid (natural or non-natural amino acid) including a β-amino acid; or a glycine; or a derivative thereof , attached to the rest of the molecule either by its amino or carboxylic acid residue depending on the nature ofY; m is 0 (where no X group is present) or 1, and preferably m is 1; n is 0 (where no Y group is present) or 1, and preferably n is 1; and pharmaceutically acceptable salts thereof

Example compounds of Formula I include those of the following Formula I': 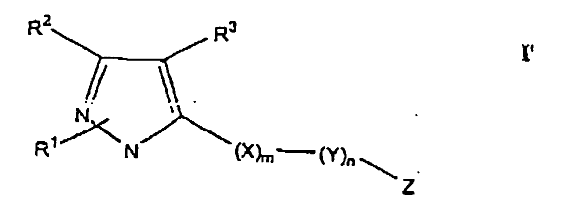 wherein
R¹, R² and R³ are each independently hydrogen; optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optional substituted alkynyl preferably having 2 to about 20 carbon atoms, more preferably 2 to about 12 carbon atoms; optionally substituted alkoxy preferably having 1 to about 20 carbon atoms, more preferably 1 to about 12 carbon atoms; optionally substituted alkylthio preferably having 1 to about 20 carbon atoms, more preferably 1 to about 12 carbon atoms ; optionally substituted alkylsulfinyl preferably having 1 to about 20 carbon atoms, more preferably 1 to about 12 carbon atoms; optionally substituted alkylsulfonyl preferably having 1 to about 20 carbon atoms, more preferably 1 to about 12 carbon atoms; optionally substituted carbocyclic aryl having at least 6 ring carbon atoms; optionally substituted aralkyl having at least 6 ring atoms; optionally substituted heteroaromatic or heteroalicyclic group having 1 to 3 rings, 3 to 8 ring members in each ring and from 1 to 3 hetero atoms (N, O or S); or optionally substituted heteroaralkyl or héteroalicyclicalkyl having 1 to 3 rings, 3 to 8 ring members in each ring and from 1 to 3 hetero atoms (particularly N, O or S);
X is optionally substituted alkylene preferably having 1 to about 12 chain carbons, more preferably 2 to about 8 chain carbons, still more preferably 3 to about 6 alkylene chain carbons; optionally substituted alkenylene preferably having 2 to about 12 chain carbons, more preferably 2 to about 8 chain carbons, still more preferably 3 to about 6 alkenylene chain carbons; optionally substituted alkynylene preferably having 2 to about 12 chain carbons, more preferably 2 to about 8 chain carbons, still more preferably 3 to about 6 alkynylene chain carbons; optionally substituted heteroalkylene preferably having 1 to about 12 chain carbons, more preferably 1 to about 8 chain carbons, still more preferably 3 to about 6 heteroalknylene chain carbons and a total of 4 or 5 atoms in the heteroalkylene chain (inclusive of N, O or S atoms); optionally substituted hcteroalkenylene preferably having 2 to about 12 chain carbons, more preferably 2 to about 8 chain carbons, still more preferably 3 to about 6 heteroalkenylene chain carbons and a total of 4 or 5 atoms in the heteroalkenylene chain (inclusive of N, O or S atoms); optionally substituted heteroalkynynylene preferably having 2 to about 12 chain carbons, more preferably 2 to about 8 chain carbons, still more preferably 3 to about 6 heteroalkynylene chain carbons and a total of 4 or 5 atoms in the heteroalkynylene chain (inclusive of N, O or S atoms);
or X is optionally substituted alicyclic; optionally substituted carbocyclic aryl ; optionally substituted aralkyl; optionally substituted heteroaromatic; optionally substituted heteroalicyclic group; optionally substituted heteroaralkyl ; or optionally substituted heteroalicyclicalkyl group, each preferably having 3 to 10 carbon or hetero (N, O or S) atoms in a ring, more preferably 5 or 6 membered ring(s), and 1-3 N, O or S atoms;
Y is optionally substituted amino; optionally substituted methylene; carbonyl, or sulfonyl;
Z is an optionally substituted alkylamine; an amino acid (natural or non-natural amino acid) including a β-amino acid; or a glycine; m is 0 (where no X group is present) or 1, and preferably m is 1; n is 0 (where no Y group is present) or 1, and preferably n is 1; and pharmaceutically acceptable salts thereof.

Example compounds of Formula I also include those where the pyrazole ring nitrogen has a non-hydrogen substituent, such as compounds of the following Formula IA: 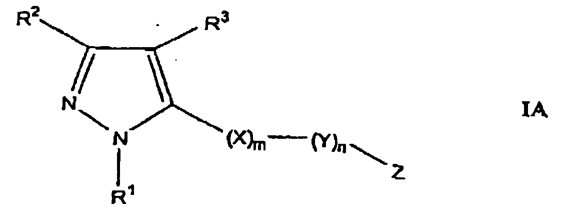 wherein R¹ is a non-hydrogen substituent selected from the same group as defined for R¹ in Formula I above; R², R³, X, Y, Z, m and n are the same as defined in Formula I above; and pharmaceutically acceptable salts thereof.

Preferred R¹ groups of compounds of Formula IA include optionally substituted alkyl ; optionally substituted alkenyl ; optionally substituted carbocyclic aryl; optionally substituted aryalkyl; optionally substituted heteroaromatic; optionally substituted hoteroalicyclic group; optionally substituted heteroarylalkyl; or optionally substituted heteroalicyclicalkyl group.

Compounds of the invention are compounds of the following Formula IB: 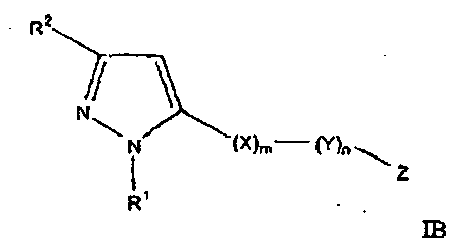 wherein
R¹ and R² are each independently optionally substituted alkyl; optionally substituted alkenyl; optionally substituted alkynyl; optionally substituted carbocyclic, aryl; optionally substituted aralkyl; optionally substituted heteroaromatic or heteroalicyclic group; or optionally substituted heteroaralkyl or heteroalicyclicalkyl group;
X is optionally substituted alkylene; optionally substituted alkenylene; optionally substituted alkynylene; optionally substituted heteroalkylene; optionally substituted heteroalkenylene; optionally substituted heteroalkenynylene; optionally substituted alicyclic; optionally substituted carbocyclic aryl; optionally substituted aralkyl; optionally substituted heteroaromatic; optionally substituted heteroalicyclic group; optionally substituted heteroaralkyl; or optionally substituted heteroalicyclicalkyl group;
Y is optionally substituted amino; optionally substituted methylene; carbonyl; or sulfonyl;
Z is an optionally substituted alkylamine; an amino acid; or a glycine; m and n are each independently 0 or 1; and pharmaceutically acceptable salts thereof; for the preparation of a pharmaceutical composition for the treatment of a condition, disorder or disease involving testosterone deficiency.

Preferred compounds of the invention are compounds of the following Formula IC: 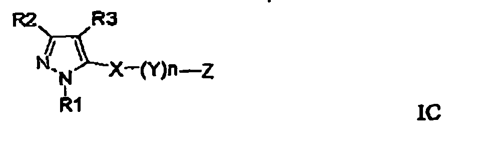 wherein
R¹ is selected from the group consisting of C₁-C₆ alkyl, aryl alkyl, aryl, heteroaryl and alkyl aryl;
R² is selected from the group consisting of aryl and heteroaryl ;
R³ is hydrogen;
X is selected from the group consisting of aryl, heteroaryl, alkyl, heteroalkyl, amino alkyl, aryl heteroalkyl, amino alkyl phenyl and heterocycloalkyl alkyl;
Y is carbonyl;
Z is selected from the group consisting of substituted alkylamine, -NH-CH(C(O)-NH₂)₀₋₁-(CH2)₀₋₂-Phe-OH, -NH-CH(C(O)-NH₂)₀₋₁-(CH₂)₀₋₂-Phe-O-alkyl, -NH-CH(C(O)-NH₂)₀₋₁-(CH2)₀₋₂-Phe- NH₂, -NH-CH(C(O)-NH₂)₀₋₁-(CH₂)₀₋₂-Phe-NH-C(O)CH₃ and -NMe-CH(C(O)-NCH₃)-CH2-Phe-OH, an amino acid derivative amino attached by the α-amino group to the rest of the molecule,
n is 1.

Preferred R¹ in compounds of formula IC are those that are selected from the group consisting of phenyl, -Phe-butyl, -Phe-propyl, -Phe-(CH₂)₂-CH ; benzylbutyl pyridinyl ; propyl, hexyl;

Preferred R² in compounds of formula IC are those selected from the group consisting of pyridinyl, isoquinolinyl, quinolinyl, furyl, dimethyl amino phenyl and pyrazinyl;

Preferred X in compounds of formula IC are those selected from the group consisting of phenylene, thienylene, ethylene, propylene, pentylene, -CH₂-NH-, -CH₂-NH-CH₂, - CH₂-NH-CH₂- CH₂, -CH2-NH-Phe, - CH₂-piperidin- and -NH-(CH₂)₃-, ;

Preferred Z in compounds of formula IC are those selected from the group consisting of tyrosinamide, threonamide, serinamide, hydroxymethyl phenylalanamide (these amino acid derivatives being attached by the α-amino group to the rest of the molecule), -NMe-CH(C(O)-NCH₃)-CH₂-Phe-OH, -NH-CH(C(O)NH₂)-(CH₂)₂-Phe-OH, -NH-CH(PheOH)-C(O)-NH₂-NH-(CH₂)₂-Phe-OH and-NH-CH(C(O)NH₂)-(CH₂)₂-Phe-O-t-bu ;

A particularly preferred embodiment of the invention is a pyrazole derivative according to formula IC, wherein R¹ is 4-t-butyl phenyl; R² is pyridinyl; R³ is hydrogen; X is propylene; Y is carbonyl; Z is tyrosinamide attached by the α-amino group to the rest of the molecule ; n is 1;

Other preferred compounds of the invention also include pyrazoles of formula ID: 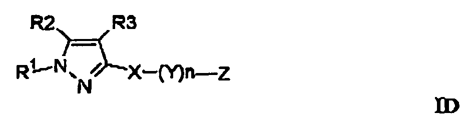 Wherein
R¹ is selected from the group consisting of C₁-C₆ alkyl, aryl alkyl, aryl and alkyl aryl, heteroaryl;
R² is selected from the group consisting of aryl and heteroaryl;
R³ is hydrogen;
X is selected from the group consisting of aryl, heteroaryl, alkyl, heteroalkyl, amino alkyl, aryl alkyl, aryl heteroalkyl, amino alkyl phenyl and heterocycloalkyl alkyl;
Y is carbonyl;
Z is selected from the group consisting of alkylamine, -NH-CH(C(O)-NH₂)₀₋₁-(CH2)₀₋₂-Phe-OH and -NH-CH(C(O)-NH₂)₀₋₁-(CH2)₀₋₂-Phe-O-alkyl, -NH-CH(C(O)-NH₂)₀₋₁-(CH₂)₀₋₂-Phe- NH₂, -NH-CH(C(O)-NH₂)₀₋₁-(CH2)₀₋₂-Phe-NH-C(O)CH₃, -NMe-CH(C(O)-NCH₃-CH2-Phe-OH, an amino acid derivative amino attached by the α-amino group to the rest of the molecule; n is 1.

Preferred R¹, Preferred R², X and Z in formula ID are those preferred defined for formula IC. A particularly preferred embodiment of the invention is a pyrazole derivative according to formula 1D, wherein R¹ is 4-t-butyl phenyl; R² is pyridinyl; R³ is hydrogen; X is propylene; Y is carbonyl; Z is tyrosinamide attached by the α-amino group to the rest of the molecule; n is 1;

Also described are compounds of the following Formula IE 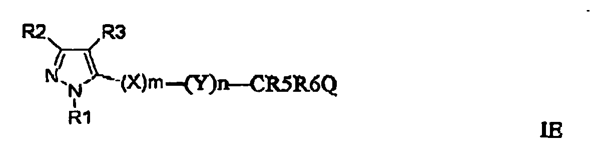 wherein
R¹ and R² are each non-hydrogen substituents independently selected from the same group as defined in Formula I above;
R³, X, Y, m and n are the same as defined in Formula I above;
R⁵ and R⁶ are independently hydrogen, optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms; optionally substituted aralkyl having at least about 6 ring carbon atoms; optionally substituted heteroaromatic or heteroalicyclic group having from 1 to 3 rings, 3 to 8 ring members in each ring and from 1 to 3 hetero atoms; or optionally substituted heteroaralkyl or heteroalicyclicalkyl group having from 1 to 3 rings, 3 to 8 ring members in each ring and from 1 to 3 hetero atoms more preferably one of R⁵ and R⁶ is hydrogen; and
Q is omitted (it does not exist) or is -(CH₂)p-CO-A-R⁷ wherein p is 0, 1 or 2, A is O or NH and R⁷ is independently hydrogen, optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms; optionally substituted aralkyl having at least about 6 ring carbon atoms; optionally substituted heteroaromatic or heteroalicyclic group 1 to 3 rings, 3 to 8 ring members in each ring and from 1 to 3 hetero atoms (particularly 1-3 N, O and/or S atoms); or optionally substituted heteroaralkyl or heteroalicyclicalkyl group having from 1 to 3 rings. 3 to 8 ring members in each ring and from 1 to 3 hetero atoms (particularly 1-3 N, O and/or S atoms); and pharmaceutically acceptable salts thereof.

Also described are compounds of the following Formula IF 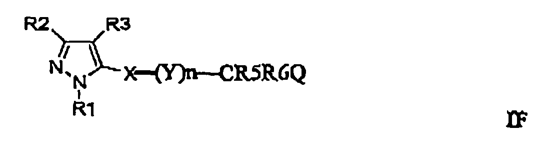 wherein
R¹ and R² are each non-hydrogen substituents independently selected from the same group as defined in Formula I above; R³, Y and n are the same as defined in Formula I above; X is optionally substituted heteroalkylene preferably having 1 to about 12 chain carbons, more preferably 2 to about 8 chain carbons, still more preferably 3 to about 6 heteroalkylene chain carbons and a total of 4 or 5 atoms in the heteroalkylene chain inclusive of N, O and/or S atoms; optionally substituted heteroalkenylene preferably having 2 to about 12 chain carbons, more preferably 2 to about 8 chain carbons, still more preferably 3 to about 6 heteroalkenylene chain carbons and a total of 4 or 5 atoms in the heteroalkenylene chain inclusive of N, O and/or S atoms; optionally substituted heteroalkynynylene preferably having 2 to about 12 chain carbons, more preferably 2 to about 8 chain carbons, still more preferably 4 or 5 heteroalkynylene chain carbons and a total of 4 or 5 atoms in the heteroalkynylene chain inclusive of N, O and/or S atoms;
R⁵ and R⁶ are independently hydrogen, optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms ; optionally substituted aralkyl having at least about 6 ring carbon atoms; optionally substituted heteroaromatic or heteroalicyclic group 1 to 3 rings, 3 to 8 ring members in each ring and from 1 to 3 hetero atoms (particularly 1-3 N, O and/or S atoms); or optionally substituted heteroaralkyl or heteroalicyclicalkyl group having from 1 to 3 rings, 3 to 8 ring members in each ring and from 1 to 3 hetero atom (particularly 1-3 N, O and/or S atoms), more preferably one of R⁵ and R⁶ is hydrogen; and
Q is omitted (i.e. it is not present) or is -(CH2)p-CO-A-R⁷ wherein p is 0, 1 or 2 ; A is O or NH and R⁷ is independently hydrogen, optionally substituted alkyl preferably having 1 to about 20 carbons, more preferably 1 to about 12 carbons; optionally substituted alkanyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms; optionally substituted aralkyl having at least about 6 ring carbon atoms; optionally substituted heteroaromatic or heteroalicyclic group 1 to 3 rings, 3 to 8 ring members in each ring and from 1 to 3 hetero atoms (particularly 1-3 N, O and/or S atoms); or optionally substituted heteroaralkyl or heteroalicyclicalkyl group having from 1 to 3 rings, 3 to 8 ring members in each ring and from 1 to 3 hetero atoms (particularly 1-3 N, O and/or S atoms); and pharmaceutically acceptable salts thereof.

Also described are compounds of the following Formula IG: 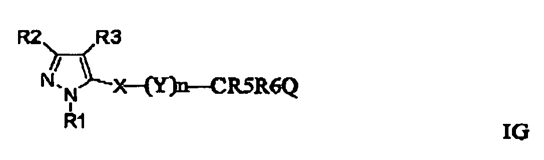 wherein
R¹ and R² are each non-hydrogen substituents independently selected from the same group as defined in Formula. I above;
R³, Y and n are the same as defined in Formula I above;
X is optionally alicyclic, optionally substituted carbocyclic aryl; optionally substituted heteroalicyclic, optionally substituted heteroaromatic, optionally substituted heteroaralkyl, or optionally substituted heteroalicyclicalkyl group, each preferably having 3-10 carbon or hetero atoms in a ring, more preferably 5 or 6 membered ring(s), and 1-3 N, O or S atoms; R⁵ and R⁶ are independently hydrogen , optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms; optionally substituted aralkyl having at least about 6 ring carbon atoms; optionally substituted heteroaromatic or heteroalicyclic group 1 to 3 rings, 3 to 8 ring members in each ring and from 1 to 3 hetero atoms (particularly 1-3 N, O and/or S atoms); or optionally substituted heteroaralkyl or heteroalicyclicalkyl group having from 1 to 3 rings, 3 to 8 ring members in each ring and from 1 to 3 hetero atoms (particularly 1-3 N, O and/or S atoms), more preferably one of R⁵ and R⁶ is hydrogen; and
Q is omitted (it does not exist) or is -(CH2)p-CO-A-R⁷ wherein p is 0, 1 or 2 ; A is O or NH and R⁷ is independently hydrogen, optionally substituted alkyl preferably having 1 to about 20 carbons, more preferably 1 to about 12 carbons; optionally substituted alkenyl preferably having 2 to about 20 carbon atoms, more preferably 2 to about 12 carbon atoms; optionally substituted alkynyl preferably having 2 to about 20 carbon atoms, more preferably 2 to about 12 carbon atoms; optionally substituted carbocyclic aryl having at least about 6 ring carbon atoms; optionally substituted aralkyl having at least about 6 ring carbon atoms; optionally substituted heteroaromatic or heteroalicyclic group 1 to 3 rings, 3 to 8 ring members in each ring and from 1 to 3 hetero atoms; (particularly 1-3 N, O and/or S atoms); or optionally substituted heteroaralkyl or heteroalicyclicalkyl group having from 1 to 3 rings, 3 to 8 ring members in each ring and from 1 to 3 hetero atoms (particularly 1-3 N, O and/or S atoms); and pharmaceutically acceptable salts thereof

Preferred R¹ groups of pyrazole compounds of Formulae I, I', IA, IB, IC, IE, IF and IG are optionally substituted alkyl, optionally substituted carbocyclic aryl, optionally substituted aralkyl (or aryalkyl) such as optionally substituted benzyl, and optionally substituted heteroaromatic, particularly optionally substituted phenyl, benzyl, pyridyl and naphthyl Generally preferred R¹ groups include C₁₋₁₆ alkyl, branched alkyl such as propyl, butyl, pentyl, and the like, particularly branched alkyl such as i-propyl, t-butyl, sec-pentyl and the like, and optionally substituted phenyl such as phenyl having one or more C₁₋₁₆ alkyl substituents e.g. propyl, butyl including t-butyl, and the like. Particularly preferred R¹ groups have more significant size (molecular volume) such as C₄₋₁₆ alkyl preferably branched such as t-butyl, isopentyl and the like, and substituted carbocyclic aryl, particularly with branched ring substituents such phenyl substituted with t-butyl, isopentyl, trifluoromethyl CF₃CH₂-, and the like.

Preferred R² groups of compounds of Formulae I, I', IA, IB, IC, IE, IF and IG include optionally substituted carbocyclic aryl, particularly optionally substituted phenyl and naphthyl, and optionally substituted heteroaromatic and heteroalicyclic, particularly nitrogen-containing heterocyclics such as pyridyl, tetrahydropyridyl, quinoline, isoquinoline, piperidine and the like.

Preferred X linker groups of compounds of Formulae I, I', IA, IB and IE are alkylene groups, particularly alkylene groups having 2, 3, 4, 5, or 6 chain carbon atoms (i.e. -CH₂-), more preferably 4 or 5 chain carbon atoms. Preferred X linkers groups of compounds of Formula I, I' IA, IB and IE include carbocyclic aryl, heteroaromatic, alicyclic and heteroalicyclic groups, particularly groups having 3 to 10 ring members, more particularly 4-6 membered rings, such as optionally substituted phenyl, thienyl, furyl, pyrrolyl and pyridyl groups. Carbonyl is a preferred Y group.

Preferred Z groups of compounds of Formulae I, I', IA, IB, IC, IE, IF and IG comprise hydroxy moieties, particularly phenolic moieties. Substituted amines and natural and non-natural amino acids are generally preferred Z groups. For example, preferred Z groups include optionally substituted C₁₋₁₂ alkylamine, preferably substituted with a phenolic group on the alkyl chain, such as -NH(CH₂)₁₋₈C₆H₄OH. Preferred Z groups include tyrosine groups and other aminophenyl groups such as -NHCH(CH₂C₆H₄OH)C(=O)NH₂; -NH{CH₂C₆H₃-(N=C-NH-)}C(=O)NH₂; NH{CH₂C₆H₃-(N=N-NH-)}C(=O)NH₂ where in such groups the phenolic moiety may be substituted at any available phenyl ring position, and preferably the hydroxyl is a meta or para substituent. Specifically preferred Z groups including tyrosine, homo-tyrosine, 4-hydroxy-α-phenylglycine, 4-amino-phenylalanine, 4-hydroxymethyl-phenylalanine, 4-acetyl-amino-phenylalanine, meta-tyrosine, β-homo-tyrosine, threonine, serine, and 4-hydroxy-phenyl-ethylamine.

Also, unless otherwise indicated herein, it is understood that the above Formulae I, I' IA, IB, IC, IE, IF and IG are inclusive of all possible regio isomers of the depicted pyrazoles, even in the case of Formulae IA, IB, IC, IE, IF and IG where the preferred regio isomer is depicted.

Particular compounds of the invention include:
N-{5-[1-(4-tert-butylphenyl)-3-pyridin-2-yl-1H-pyrazol-5-yl]pentanoyl}tyrosinamide;
N-{5-[1-(4-tert-butylphenyl)-3-pyridin-3-yl-1H-pyrazol-5-yl]pentanoyl}tyrosinamide;
N-{5-[1-(4-tert-butylphenyl)-5-pyridin-3-yl-1H-pyrazol-3-yl]pentanoyl}tyrosinamide;
N-{5-[1-(4-tert-butylphenyl)-3-pyridin-4-yl-1H-pyrazol-5-yl]peatanoyl}tyrosinamide ;
N-{5-[1-(4-tert-butylphenyl)-5-pyridm-4-yl-1H-pyrazol-3-yl]peatanoyl}tyrosmanaride ;
N-{5-[1-(4-tert-butylphenyl)-3-pyridin-3-yl-1H-pyrazol-5-yl]pentanoyl}-N,N-dimethyltyrosinamide;
N-(3-[1-(4-tert-butylphenyl)-5-pyridin-3-yl-1H-pyrazol-3-yl]propanoyl)tyrosinamide ;
N-(3-[1-(4-tert-butylphenyl)-3-pyridin-3yl-1H-pyrazol-5-yl]propanoyl)tyrosinamide N-[4-(1-butyl-3-isoquinolin-3-yl-1H-pyrazol-5-yl)benzoyl]tyrosinamide;
N-{5-[1-(4-isopropylphenyl)-3-pyridin-3-yl-1H-pyrazol-5-yl]pentanoyl}tyrosinamide;
N-{6-[1-(4-isopropylphenyl)-3-pyridin-2-yl-1H-pyrazol-5-yl] hexanoyl}tyrosinamide
N-{6-[1-(4-tert-butylphenyl)-3-pyridin-2yl-1H-pyrazol-5-yl]hexanoyl}-3-hydroxyphenylalaninamide;
N-[1-(aminocarbonyl)-3-(4-hydroxyphenyl)propyl]-5-[1-(-4-tert-butylphenyl)-3-pyridin-2-yl-1H-pyrazol-5-yl]pentanamide ;
N-[5-(1-butyl-3-isoquinolin-3-yl-1H-pyrazol-5-yl)pentainoyl]tyrosinamide ;
N-{6-[1-(4-tert-butylphenyl)-3-pyridin-2-yl-1H-pyrazol-5-yl]hexanoyl}serinamide;
N-{6-[1-(4-iso-propylphenyl)-3-pyridin-2-yl-1H-pyrazol-5-yl]hexanoyl}serinamide;
N-{6-[1-(4-iso-propylphenyl)-3-pyridin-2-yl-1H-pyrazol-5-yl]hexanoyl}threonamide ;
N-{5-[1-(4-isopropylphenyl)-3-pyridin-2-yl-1H-pyrazol-5-yl]pentanoyl}tyrosinamide;
6-[1-(4-tert-butylphenyl)-3-pyridin-2-yl-1H-pyrazol-5-yl]-N-[2-(4-hydroxyphenyl)ethyl]hexanamide;
N-{6-[1-(4-tert-butylpheayt)-3-pyridin-2-yl-1H-pyrazol-5-yl]hexanoyl}tyrosinamide;
N-{5-[1-(4-tert-butylphenyl)-3-isoquinolin-3-yl-1H-pyrazol-5-yl]pentanoyl}tyrosinamide;
N-{6-[1-(4-tert-butyphenyl)-3-isoquinolin-3-yl-1H-pyrazol-5-yl]hexanoyl}tyrosmamide;
N-{6-[1-(4-tert-butylphenyl)-3-pyridin-2-yl-1H-pyrazol-5-yl]hexanoyl}-N-methyltyrosinamide;
N-{6-[1-(4-tert-butylphenyl)-3-pyridin-2-yl-1H-pyrazol-5-yl]hexanoyl}-4-(hydroxymethyl)phenylalaninamide;
4-amino-N-{6-[1-(4-tert-butylphenyl)-3-pyridin-2-yl-1H-pyrazol-5-yl]hexanoyl}phenylalaninamide;
4-(acetylamino)-N-{6-[1-(4-tert-butylphenyl)-3-pyridin-2-yl-1H-pyrazol-5-yl]hexanoyl}phenylalaninamide;
4-(aminocarbonyl)-N-{6-[1-(4-tert-butylphenyl)-3-pyridin-2-yl-1H-pyrazol-5-yl]hexanoyl}phenylalaninamide;
N-butyl-N-{6-[1-(4-tert-butylphenyl)-3-pyridm-2-yl-1H-pyrazol-5-yl]hexanoyl}tyrosinamide;
N-{6-[1-(4-tert-butylphenyl)-3-pyridin-2-yl-1H-pyrazol-5-yl]hexanoyl}threonamide;
N-[2-amino-1-(4-hydroxyphenyl)-2-oxoethyl]-6-[1-(4-tert-butylphenyl)-3-pyridin-2-yl-1H-pyrazol-5-yl]hexanamide;
N-({5-[1-(4-isopropylphenyl)-3-pyridin-2-yl-1H-pyrazol-5-yl]thien-2-yl}carbonyl)tyrosinamide;
N-[2-amino-1-(4-hydroxyphenyl)-2-oxoethyl]-4-[1-(4-tert-butylphenyl)-3-pyridin-2-yl-1H-pyrazol-5-yl]benzamide;
4-[1-(4-tert-butylphenyl)-3-pyridin-2-yl-1H-pyrazol-5-yl]-N-[2-(4-bydroxyphenyl)ethyl]benzamide;
N-{3-[1-(4-tert-butylbenzyl)-3-isoquinolin-3-yl-1H-pyrazol-5-yl]benzoyl}tyrosinamide;
N-{5-[1-(4-tert-butylphenyl)-3-pyridin-4-yl-1H-pyrazol-5-yl]pentanoyl}tyrosinamide;
N-{3-[1-(4-tert-butylbenzyl)-3-pyridin-3-yl-1H-pyrazol-5-yl]benzoyl}tyrosinamide;
N-{3-[1-(4-tert-butylphenyl)-3-isoquinolin-3-yl-1H-pyrazol-5-yl]benzoyl}tyrosinamide,
N-[4-(1-butyl-3-isoquinolin-3-yl-1H-pyrazol-5-yl)benzoy]tyrosinamide ;
N-{3-[1-(4-tert-butylbenzyl)-3-quinolin-3-yl-1H-pyrazol-5-yl]benzoyl}tyrosinamide;
N-(3-[3-isoquinolin-3-yl-1-(4-propylphenyt)-1H-pyrazol-5-yl]benzoyl)tyrosinamide ;
N-{5-[1-(4-tert-butylphenyl)-3-pyridin-3-yl-1H-pyrazol-5-l]pentanoyl}tyrosinamide;
N-{3-[1-(4-tert-butylphenyl)-3-pyridin-3-yl-1H-pyrazol-5-yl]benzoyl}tyrosinamide ;
N-{4-[1-(4-tert-butylphenyl)-3-pyridin-3-yl-1H-pyrazol-5-yl]benzoyl}tyrosinamide;
N-{5-[1-(4-tert-butylbenzyl)-3-pyridin-4-yl-1H-pyrazol-5-yl]pentanoyl}tyrosinsmide;
N-{3-[1-(4-tert-butylbenzyl)-3-pyridin-4-yl-1H-pyrazol-5-yl]benzoyl}tyrosinamide;
N-{5-[1-(4-tert-butylbenzyl)-3-pyridin-3-yl-1H-pyrazol-5-l]pentanoyl}tyrosinamide,
N-{5-[1-(4-tert-butylphenyl)-3-pyridin-4-yl-1H-pyrazol-5-yl]pentanoyl}tyrosinamide;
N-[3-(1-butyl-3-isoquinolin-3-yl-1H-pyrazol-5-yl)benzoyl]tyrosinamide;
N-(4-{[1-(4-tert-butylphenyl)-3-pyridin-4-yl-1H-pyrazol-5-yl]amino}butanoyl)tyrosinamide;
N-[(1-butyl-3-pyridin-3-yl-1H-pyrazol-5-yl)methyl]glycyltyrosinamide,
N-{[1-(4-tert-butylphenyl)-3-pyridin-3-yl-1H-pyrazol-5-yl]methyl}glycyltyrosinamide;
N-[(1-butyl-3-pyridin-3-yl-1H-pyrazol-5-yl)methyl]-beta-alanyltyrosinamide;
N-(3-{[(1-butyl-3-pyridin-3-yl-1H-pyrazol-5-yl)methyl]amino}benzoyl)tyrosinamide;
N-[3-({[1-(4-tert-butylphenyl)-3-pyridin-4-yl-1H-pyrazol-5-yl]methyl}amino)benzoyl]tyrosinamide;
N-({1-[(1-butyl-3-pyridin-3-yl-1H-pyrazol-5-yl)methyl]piperidin-4-yl}carbonyl)tyrosinamide;
N-[(1-{[1-(4-tert-bulylphenyl)-3-pyridin-3-yl-1H-pyrazol-5 yl]methyl}piperidin-4-yl)earbonyl]tyrosinamide;
N-2--{[1-(4-tert-butylphenyl)-3-pyridin-3-yl-1H-pyrazol-5-yl]methyl}-N∼1∼-[2-(4-hydroxyphenyl)ethyl]glycinamide,
N-3-[(1-butyl-3-pyridin-3-yl-1H-pyrazol-5-yl)methyl]-N∼1∼-[2-(4-hydroxyphenyl)ethyl]-beta-alaninamide ;
4-{[(1-butyl-3-pyridin-3-yl-1H-pyrazol-5 yl)methyl]amino}-N-[2-(4-hydroxyphenyl)ethyl]benzamide ; or an optically active isomer, racemate or tautomer thereof,
and/or a pharmaceutically acceptable salts thereof

Additional preferred compound include:
N-({3-[1-(4-tert-butylphenyl)-3-pyridin-4-yl-1H-pyrazol-5-yl]propoxy}acetyl)}tyrosinamide;
4-[2-({3-[1-(4-tert-butylphenyl)-3-pyridin-4-yl-1H-pyrazol-5-yl]propyl}amino)ethyl]phenol;
N-{3-[1-(4-tert-butylphenyl)-3-pyridin-4-yl-1H-pyrazol-5-yl]propyl}tyrosinamide;
N-acetyl-N-{3-[1-(4-tert-butylphenyl)-3-pyridin-4-yl-1H-pyrazol-5-yl]propyl}tyrosinamide;
N-({3-[1-(4-tert-butylphenyl)-3-pyridin-4-yl-1H-pyrazol-5-yl]propoxy}acetyl)tyrosinamide;
4-[2-({3-[1-(4-tert-butylphenyl)-3-pyridin-4-yl-1H-pyrazol-5-yl]propyl}amino)ethyl]phenol;
N-{3-[1-(4-tert-butylphenyl)-3-pyridin-4-yl-1H-pyrazol-5-yl]propyl}tyrosinamide;
N-acetyl-N-{3-[1-(4-tert-butylphenyl)-3-pyridin-4-yl-1H-pyrazol-5-yl]propyl}tyrosinamide;
3-[(4-{[1-(4-tert-butylphenyl)-3-pyridin-4-yl-1H-pyrazol-5-yl]amino}butanoyl)amino]-4-(4-hydroxyphenyl)butanamide;
N-(4-{[1-(4-tert-butylphenyl)-3-(1-methyl-1,2,3,6-tetrahydropyridin-4-yl)-1H-pyrazol-5-yl]amino}butanoyl)tyrosinamide;
N-(3-{[1-(4-tert-butylphenyl)-3-pyridin-4-yl-1H-pyrazol-5-yl]amino}propyl)tyrosinamide;
N-acetyl-N-(3- {[1-(4-tert-butylphenyl)-3-pyridin-4-yl-1H-pyrazol-5-yl]amino}propyl)tyrosinamide;
N-(4-{[1-(4-tert-butylphenyl)-3-(1-oxidopyridin-4-yl)-1H-pyrazol-5-yl]amino} butanoyl)tyrosinamide;
4-{[1-(4-tert-butylphenyl)-3-pyridin-4-yl-1H-pyrazol-5-yl]amino}-N-[2-(4-hydroxyphenyl)ethyl]butanamide;
4-{2-[(4-{[1-(4-tert-butylphenyl)-3-pyridin-4-yl-1H-pyrazol-5-yl]amino}butyl)amino]ethyl} phenol;
N-(4-{[1-(4-tert-butylphenyl)-3-pyridin-4-yl-1H-pyrazol-5-yl]amino}butanoyl)-N-(2-hydroxyethyl)tyrosinamide;
N-(4-{[1-(4-tert-butylphenyl)-3-pyridin-4-yl-1H-pyrazol-5 yl]amino}butanoyl)-N-[2-(1-methylpyrrolidin-2-yl)ethyl]tyrosinamide;
N-(4-{[1-(4-tert-butylphenyl)-3-pyridin-3-yl-1H-pyrazol-5-yl]amino}butanoyl)tyrosinamide;
N-(4-{[1-(4-tert-butylphenyl)-3-(1-methyl-1,2,5,6-tetrahydropyridin-3-yl)-1H-pyrazol-5-yl]amino}butanoyl)tyrosinamide;
(3R)-3-[(4-{[1-(4-tert-butylphenyl)-3-pyridin-3-yl-1H-pyrazol-5-yl]amino}butanoyl)amino]-4-(4-hydroxyphenyl)butanamide;
N-(3-{[1-(4-tert-butylphenyl)-3-pyridin-3-yl-1H-pyrazol-5-yl]amino}propyl)tyrosinamide;
N-acetyl-N-(3-{[1-(4-tert-butylphenyl)-3-pyridin-3-yl-1H-pyrazol-5-yl]amino}propyl)tyrosmatmde;
N-(4-{[1-(4-tert-butylphenyl)-3-(1-oxidopyridin-3-yl)-1H-pyrazol-5-yl]amino}butanoyl)tyrosinamide;
4-{[1-(4-tert-butylphenyl)-3-pyridin-3-yl-1H-pyrazol-5-yl]amino}-N-[2-(4-hydroxyphenyl)ethyl]butanamide;
4-{2-[(4-{[1-(4-tert-butylphenyl)-3-pyridin-3-yl-1H-pyrazol-5-yl]amino} butyl)amino] ethyl} phenol;
N-(4-{[1-(4-tert-butylphenyl)-3-pyridin-3 yl-1H-pyrazol-5-yl]amino}butanoyl)-N-(2-hydroxyethyl)tyrosinamide ;
N-(4-{[1-(4-tert-butylphenyl)-3-pyridin-3-yl-1H-pyrazol-5-yl]amino}butanoyl)-N-[2-(1-methylpyrrolidin-2-yl)ethyl]tyrosinamide;
N-(4-{[1-(4-tert-butylphenyl)-3-pyridin-3-yl-1H-pyrazol-5-yl]oxy}butanoyl)tyrosinamide; or an optically active isomer, racemate or tautomer thereof, and/or a pharmaceutically acceptable salts thereof

Substituted pyrazole compounds of the invention are useful for treatment of various conditons, disorders or diseases in male mammals, particularly humans. Therapeutic methods of the invention in general comprise administering an effective amount of one or more substituted pyrazole compounds as disclosed herein to a mammal in need thereof, such as a mammal suspected of suffering from testosterone deficiency, particularly a human suspected of suffering from testosterone deficiency. Compounds of the invention will be useful for treatment of conditions, disorders or diseases currently treated with exogenous administration of testosterone preparations, including male infertility and male spermatogenesis disorders.

Preferred compounds of the invention exhibit significantly higher testosterone induction activity in a standard assay as compared to controls, such as the assay of Example 1 which follows.

The invention also provides pharmaceutical compositions that comprise one or more substituted pyrazole compounds of the invention and a suitable carrier for the compositions. Other aspects of the invention are disclosed *infra.*

### DETAILED DESCRIPTION OF THE INVENTION

We have now discovered that substituted pyrazole compounds, including compounds of the above Formulae I, I', IA, IB, IC, IE, IF and IG, are useful for treatment of conditions, disorders or diseases which benefit patients by increasing endogenous testosterone levels.

Suitable alkyl substituent groups of compounds of the invention (which includes compounds of Formulae I, r, IA, IB, IC, IE, IF and IG as defined above) typically have from 1 to about 12 carbon atoms, more preferably 1 to about 8 carbon atoms, still more preferably 1, 2, 3, 4, 5, or 6 carbon atoms. As used herein, the term alkyl unless otherwise modified refers to both cyclic and noncyclic groups, although of course cyclic groups will comprise at least three carbon ring members. Preferred alkenyl and alkynyl groups of compounds of the invention have one or more unsaturated linkages and typically from 2 to about 12 carbon atoms, more preferably 2 to about 8 carbon atoms, still more preferably 2, 3, 4, 5, or 6 carbon atoms. The terms alkenyl and alkynyl as used herein refer to both cyclic and noncyclic groups, although straight or branched noncyclic groups are generally more preferred. Preferred alkoxy groups of compounds of the invention include groups having one or more oxygen linkages and from 1 to about 12 carbon atoms, more preferably from 1 to about 8 carbon atoms, and still more preferably 1, 2, 3, 4, 5 or 6 carbon atoms. Preferred alkylthio groups of compounds of the invention include those groups having one or more thioether linkages and from 1 to about 12 carbon atoms, more preferably from 1 to about 8 carbon atoms, and still more preferably 1, 2, 3, 4, 5, or 6 carbon atoms. Preferred alkylsulfinyl groups of compounds of the invention include those groups having one or more sulfoxide (SO) groups and from 1 to about 12 carbon atoms, more preferably from 1 to about 8 carbon atoms, and still more preferably 1, 2, 3, 4, 5, or 6 carbon atoms. Preferred alkylsulfonyl groups of compounds of the invention include those groups having one or more sulfonyl (SO₂) groups and from 1 to about 12 carbon atoms, more preferably from 1 to about 8 carbon atoms, and still more preferably 1, 2, 3, 4,5 or 6 carbon atoms. Preferred aminoalkyl groups include those groups having one or more primary, secondary and/or tertiary amine groups, and from 1 to about 12 carbon atoms, more preferably 1 to about 8 carbon atoms, still more preferably 1, 2, 3, 4, 5, or 6 carbon atoms. Secondary and tertiary amine groups are generally more preferred than primary amine moieties. Suitable heteroaromatic groups of compounds of the invention contain one or more N, O or S atoms and include, e.g., coumarinyl including 8-coumarinyl, quinolinyl including 8-quinolinyl, pyridyl, pyrazinyl, pyrimidyl, furyl, pyrrolyl, thienyl, thiazolyl, oxazolyl, oxidizolyl, triazole, imidazolyl, indolyl, benzofuranyl and benzothiazole. Suitable heteroalicyclic groups of compounds of the invention contain one or more N, O or S atoms and include, e.g., tetrahydrofuranyl, thienyl, tetrahydropyranyl, piperidinyl morpholino and pyrrolidinyl groups. Suitable carbocyclic aryl groups of compounds of the invention include single and multiple ring compounds, including multiple ring compounds that contain separate and/or fused aryl groups. Typical carbocyclic aryl groups of compounds of the invention contain 1 to 3 separate or fused rings and from 6 to about 18 carbon ring atoms. Specifically preferred carbocyclic aryl groups include phenyl; naphthyl including 1-naphthyl and 2-naphthyl; biphenyl; phenanthryl; anthracyl; and acenaphthyl. Substituted carbocyclic groups are particularly suitable including substituted phenyl, such as 2-substituted phenyl, 3-substituted phenyl, 4-substituted phenyl, 2,3-substituted phenyl, 2,4-substituted phenyl, and 2,4-substituted phenyl; and substituted naphthyl, including naphthyl substituted at the 5, 6 and/or 7 positions.

Suitable aralkyl groups of compounds of the invention include single and multiple ring compounds, including multiple ring compounds that contain separate and/or fused aryl groups. Typical aralkyl groups contain 1 to 3 separate or fused rings and from 6 to about 18 carbon ring atoms. Preferred aralkyl groups include benzyl and methylenenaphthyl (-CH₂-naphthyl), and other carbocyclic aralkyl groups, as discussed above.

Suitable heteroaralkyl groups of compounds of the invention include single and multiple ring compounds, including multiple ring compounds that contain separate and/or fused heteroaromatic groups, where such groups are substituted onto an alkyl linkage. More preferably, a heteroaralkyl group contains a heteroaromatic group that has 1 to 3 rings, 3 to 8 ring members in each ring and from 1 to 3 hetero (N, O or S) atoms, substituted onto an alkyl linkage. Suitable heteroaromatic groups substituted onto an alkyl linkage include e.g., coumarinyl including 8-coumarinyl, quinolinyl including 8-quinolinyl, pyridyl, pyrazinyl, pyrimidyl, furyl, pyrrolyl, thienyl, thiazolyl, oxazolyl, oxidizolyl, triazole, imidazolyl, indolyl, benzofuranyl and benzothiazole.

Suitable heteroalicyclicalkyl groups of compounds of the invention include single and multiple ring compounds, where such groups are substituted onto an alkyl linkage. More preferably, a heteroalicylicalkyl group contains at least one ring that has 3 to 8 ring members from 1 to 3 hetero (N, O or S) atoms, substituted onto an alkyl linkage. Suitable heteroalicyclic groups substituted onto an alkyl linkage include e.g. tetrahydrofuranyl, thienyl, tetrahydropyranyl, piperidinyl, morpholino and pyrrolidinyl groups.

As discussed above, particularly preferred X linker groups are alkylene, particularly having 4 or 5 chain carbons, such as -CH₂CH₂CH₂CH₂- and -CH₂CH₂CH₂CH₂CH₂-. X linker groups may one contain one or more carbon-carbon double or triple bonds in the chain, i.e. a alkenylene, alkynylene, heteroalkenylene or heteroalkynylene linkage. Such unsaturated X groups typically contain 1, 2 or 3 carbon-carbon multiple bonds, more typically 1 or 2 carbon-carbon multiple bonds. An X group that is heteroalkylene, heteroalkenylene, or heteroalkynylene contains one or more N, O or S atoms in the linker chain, more typically 1 or 2 N, O or S atoms in the chain. An X linker group also preferably may be carbocyclic aryl or a heteroaromatic group, particularly 3-10 membered rings or fused rings, more particularly 4, 5 and 6 membered rings with zero, one or more N, O or S atoms such as optionally substituted o-, m-, p-phenyl, pyridyl, furyl, thiophenyl and pyrrolidinyl rings.

As discussed above, R, R¹, R², R³, R⁴, R⁵, R⁶, R⁷, X, Y and Z groups are optionally substituted. A "substituted" R, R¹, R², R³, R⁴, R⁵, R⁶, R⁷, X, Y and Z group or other substituent may be substituted by other than hydrogen at one or more available positions, typically 1 to 3 or 4 positions, by one or more suitable groups such as those disclosed herein. Suitable groups that may be present on a "substituted" R, R¹, R², R³, R⁴, R⁵, R⁶, R⁷, X, Y and Z group or other substituent include e.g. halogen such as fluoro, chloro, bromo and iodo; cyano; hydroxyl; nitro; azido; alkanoyl such as a C₁₋₆ alkanoyl group such as acyl and the like; carboxamido; alkyl groups including those groups having 1 to about 12 carbon atoms, or 1, 2, 3, 4, 5, or 6 carbon atoms; alkenyl and alkynyl groups including groups having one or more unsaturated linkages and from 2 to about 12 carbon, or 2, 3, 4, 5 or 6 carbon atoms; alkoxy groups having those having one or more oxygen linkages and from 1 to about 12 carbon atoms, or 1, 2, 3, 4, 5 or 6 carbon atoms; aryloxy such as phenoxy; alkylthio groups including those moieties having one or more thioether linkages and from 1 to about 12 carbon atoms, or 1, 2, 3, 4, 5 or 6 carbon atoms; alkylsulfinyl groups including those moieties having one or more sulfinyl linkages and from 1 to about 12 carbon atoms, or 1, 2, 3, 4, 5, or 6 carbon atoms; alkylsulfonyl groups including those moieties having one or more sulfonyl linkages and from 1 to about 12 carbon atoms, or 1, 2, 3, 4, 5, or 6 carbon atoms; aminoalkyl groups such as groups having one or more N atoms and from 1 to about 12 carbon atoms, or 1, 2, 3, 4, 5 or 6 carbon atoms; carbocyclic aryl having 6 or more carbons, particularly phenyl (e.g. an R group being a substituted or unsubstituted biphenyl moiety); aralkyl having 1 to 3 separate or fused rings and from 6 to about 18 carbon ring atoms, with benzyl being a preferred group; aralkoxy having 1 to 3 separate or fused rings and from 6 to about 18 carbon ring atoms, with O-benzyl being a preferred group; or a heteroaromatic or heteroalicyclic group having 1 to 3 separate or fused rings with 3 to about 8 members per ring and one or more N, O or S atoms, e.g. coumarinyl, quinolinyl, pyridyl, pyrazinyl, pyrimidyl, furyl, pyrrolyl, thienyl, thiazolyl, oxazolyl, imidazolyl, indolyl, benzofuranyl, benzothiazolyl, tetrahydrofuranyl, tetrahydropyranyl, piperidinyl, morpholino and pyrrolidinyl.

Preferred carbocyclic or heteroaromatic ring substituents of compounds of the invention include halogen (F, Cl, Br and I; hydroxyl; azido; optionally substituted alkyl having 1 to about 12 carbons such as methyl, ethyl, propyl and butyl and branched groups such as isopropyl, sec-butyl and tert-butyl, and including halogenated alkyl, particularly fluoro-alkyl having 1 to about 6 carbon atoms; optionally substituted alkoxy having 1 to about 12 carbons such as methoxy, ethoxy, propoxy and butoxy, and including halogenated alkoxy, particularly fluoro-alkoxy having 1 to about 6 carbon atoms; optionally substituted alkylthio having 1 to about 6 carbons such as methylthio and ethylthio; optionally substituted alkylsulfinyl having 1 to about 6 carbons such as methylsulfinyl (-S(O)CH₃) and ethylsulfinyl (-S(O)CH₂CH₃); optionally substituted alkylsulfonyl having 1 to about 6 carbons such as methylsulfonyl (-S(O)₂CH₃) and ethylsulfonyl (-S(O)₂CH₂CH₃); and optionally substituted arylalkoxy such as benzyloxy (C₆H₅CH₂O-).

It should be understood that alkoxy, alkylthio, alkylsulfinyl, alkylsulfonyl and aminoalkyl substituent groups described above include groups where a hetero atom is directly bonded to a ring system, such as a carbocyclic aryl group or a heteroalicyclic or heteroaromatic group, as well as groups where a hetero atom of the group is spaced from such ring system by an alkylene linkage, e.g. of 1 to about 4 carbon atoms.

Two particularly preferred compounds of the invention are the following:
5-[2-(4-tert-Butyl-phenyl)-5-pyridin-3-yl-2H-pyrazol-3-yl]-pentanoic acid [1-carbamoyl-2-(4-hydroxy-phenyl)-ethyl]-amide 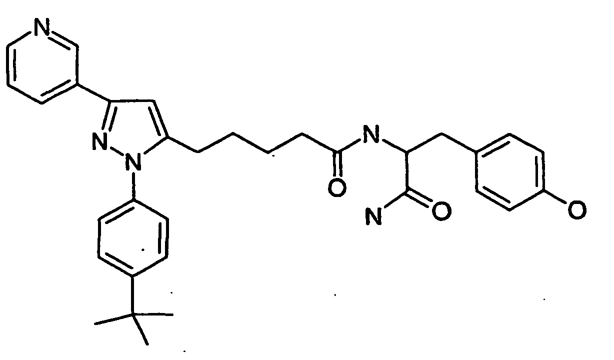
5-[2-(4-tert-Butyl-phenyl)-5-pyridin-4-yl-2H-pyrazol-3-yl]-pentanoic acid [1-carbamoyl-2-(4-hydroxy-phenyl)-ethyl]-amide

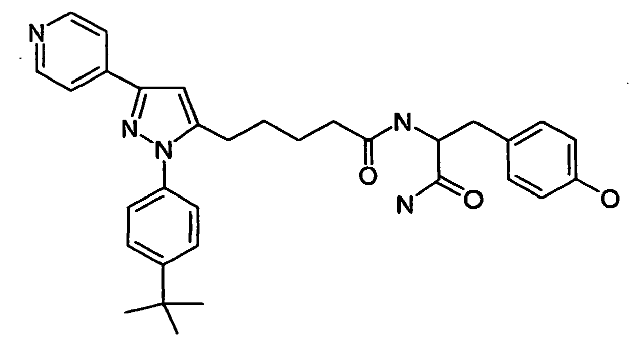

Compounds of the invention can be readily prepared. For instance, substituted pyrazoles suitable as starting reagents are commercially available. Generally preferred are pyrazoles having a 3- and/or 5-position non-hydrogen substituent that can be further functionalized to provide a desired compound of the invention. The pyrazole ring can be readily substituted with desired moieties by known ring addition reactions to provide R¹, R² and R³ substituents. For instance, non-hydrogen R² and R³ substituents can be provided by Friedel-Crafts-type reactions or Suzuki coupling reactions. See also U.S. Patents 5,986,104; 5,744,493; and 5,486,618, for procedures to prepare substituted pyrazoles. See also U.S. Patent Application 09/860,658, filed May 19, 2001; hereby incorporated by reference.

Suitable pyrazoles for use to prepare compounds of the invention also may be readily synthesized. For example, pyrazole reagents may be synthesized by e.g. reaction of a β-dicarbonyl compound with the corresponding substituted hydrazine or the reaction of the dianion of a methyl hydrazone with the corresponding acid chlorid or acid anhydride. For extensive description of pyrazoles synthesis, see Handbook of Heterocyclic Chemistry, 2nd edition, A.R. Katritzky and AF. Pozharskii, Pergamon, 2000 or Comprehensive Heterocyclic Chemistry III, A.R. Katritzky, C.W. Rees, E.F.V. Scriven, 1st Edition,1996).

Substituted pyrazole compounds of the invention also may be readily prepared by combinatorial synthetic procedures. For discussions of combinatorial procedures, see, e.g., I. Chaiken et al, Molecular Diversity and Combinatorial Chemistry, (ACS, 1996); B. Bunn, The Combinatorial Index, (Academic Press 1998). See also A. Marzinzik et al, Tetrahedron Letters, 37(7):1003-1006 (1996); Marzinzik et al., J. Org. Chem., 63: 723-727 (1998); and Marzinzik et al: WO 9815532 A1 19980416 for a solid phase combinatorial synthesis of pyrazoles. See also the examples which follow, for exemplary preferred syntheses of pyrazole compounds of the invention.

More particularly, compounds of the invention including specific regio- isomers can be suitably prepared by combinatorial synthetic procedures as outlined in the following exemplary Schemes 1, 2 (examples of mixture of isomers) and 3 (as an example of specific regio isomer). It should be appreciated that the compounds shown in the following Schemes are exemplary only, and a variety of other compounds can be employed in a similar manner as described below. Additionally, while in some instances the Schemes detail certain preferred reaction conditions, it will be understood that alternate conditions also may be suitable.

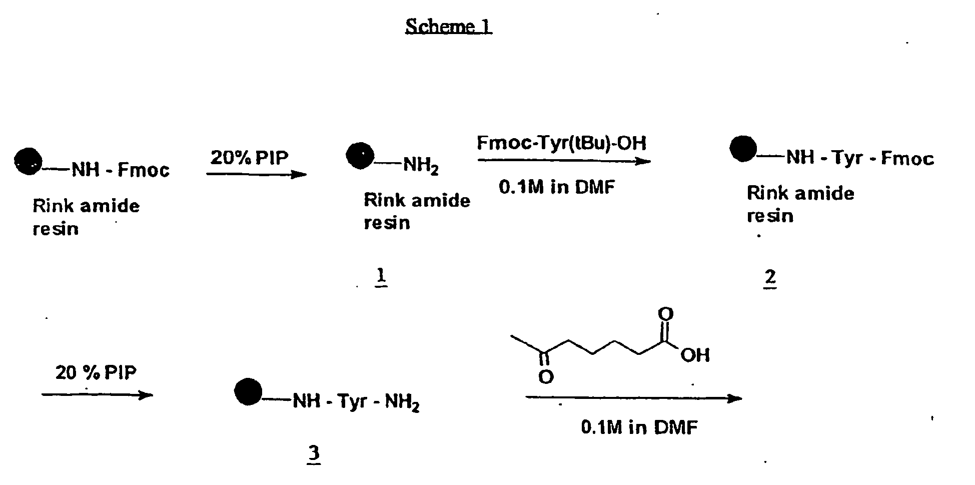

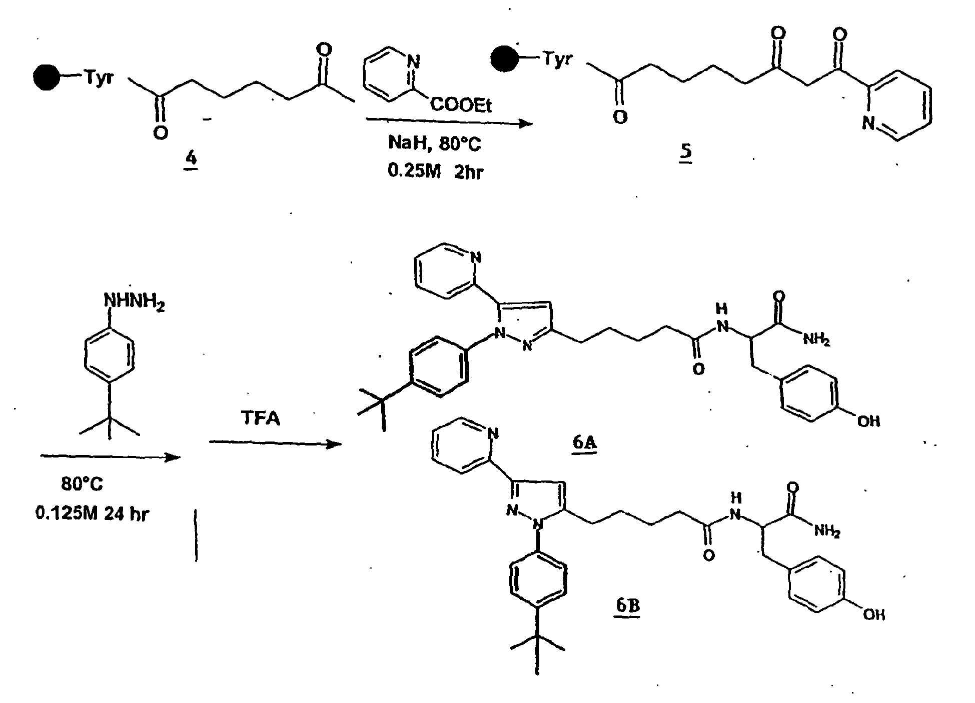

Thus, as depicted in Scheme 1 above, resin bound amine 1 is reacted with a protected amino acid to provide 2 which is reacted with a keto-acid to provide methyl ketone 4. That methyl ketone is reacted with a substituted ester (provides R² substituent of Formula I) in the presence of strong base to yield the di-ketone 5. The adjacent keto groups with interposed methylene react with a hydrazine to provide the substituted pyrazole as regio isomeric mixtures 6A and 6B.

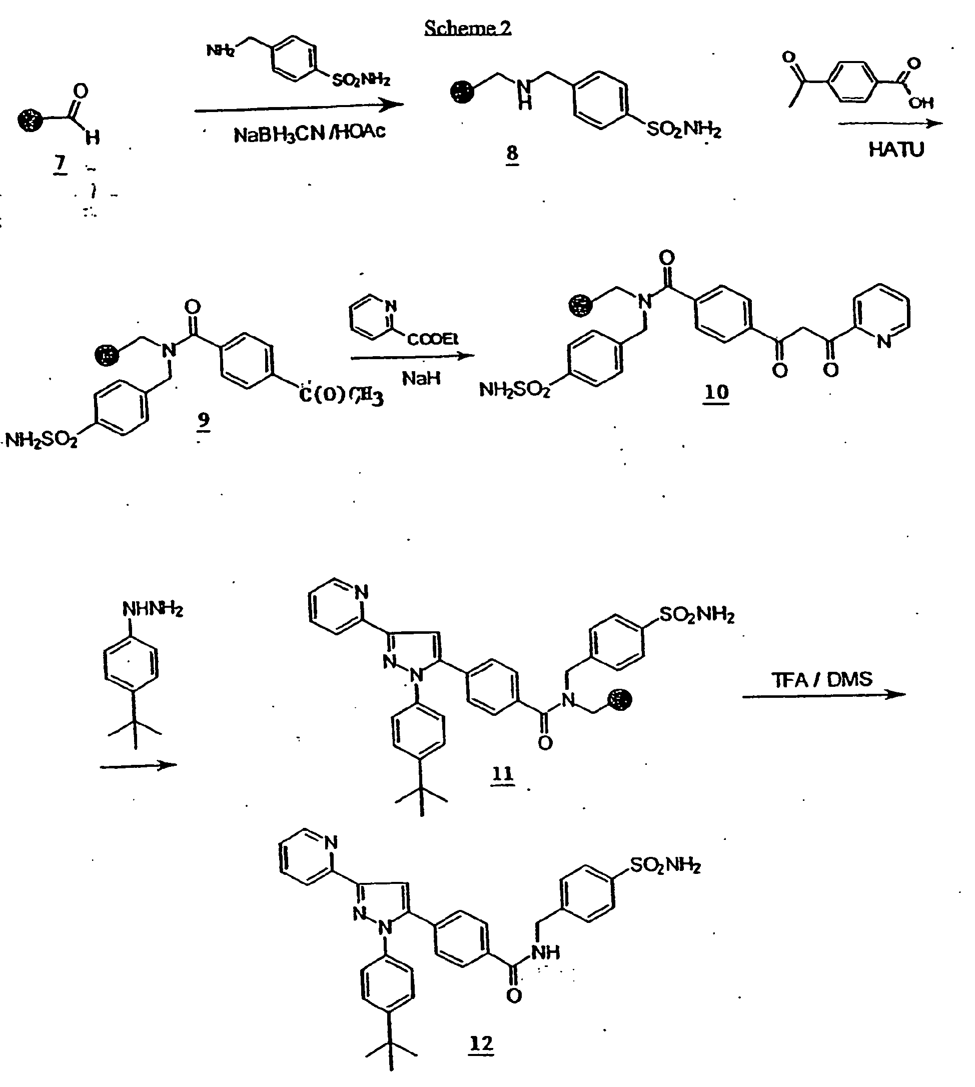

Scheme 2 above depicts another route for combinatorial synthesis of compounds of the invention. Briefly, resin bound aldehyde 1. is functionalized to give secondary amine 8, which is then reacted with a keto-acid to provide tertiary amine 9-with methyl ketone functionality. Reaction with an ester in the presence of a strong base provide compound 10 having adjacent keto groups with interposed methylene that can be reacted with a hydrazine to provide substituted pyrazole 11 and its isomer. Treatment with strong acid releases the pyrazole 12 from the resin.

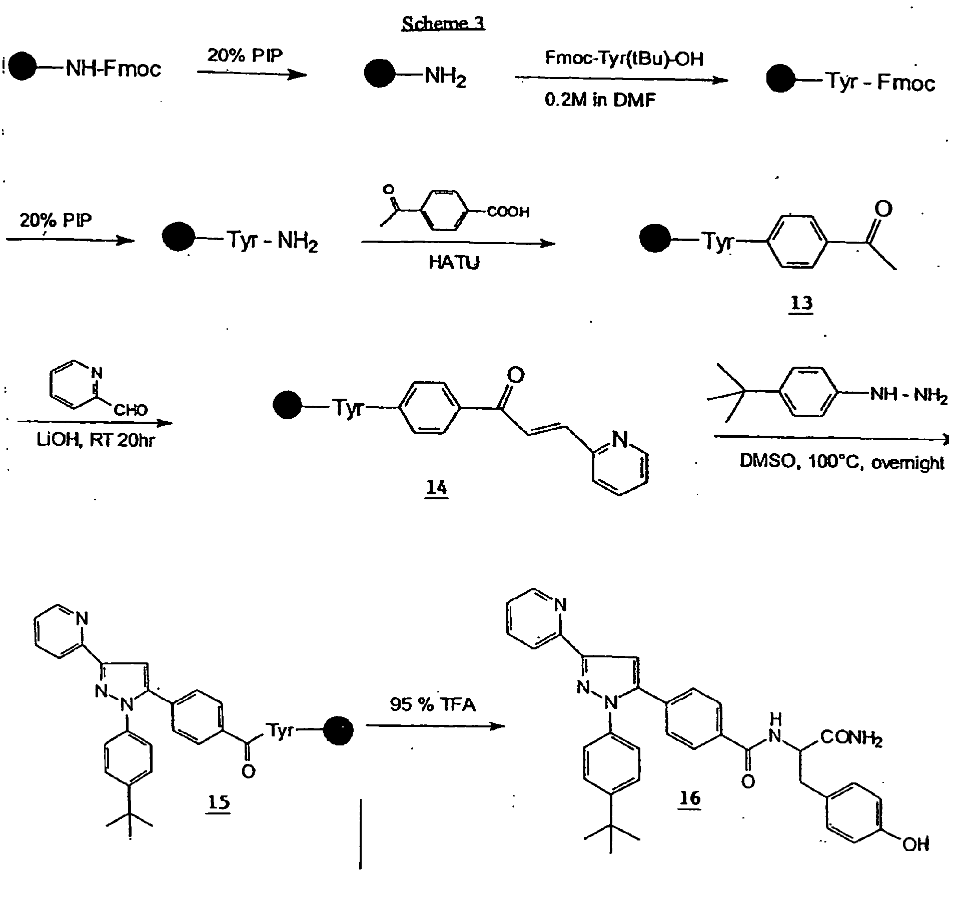

Scheme 3 above depicts a selective synthesis of specific regioisomer by combinatorial method. The resin bound methyl ketone 13 on reaction with an appropriate aldehyde in presence of Li(OH) results in α, β-unsaturated ketone 14, which on further reaction with hydrazine results in a specific regio isomer 15, which can be released from the resin support in the presence of acid to provide pyrazole 16.

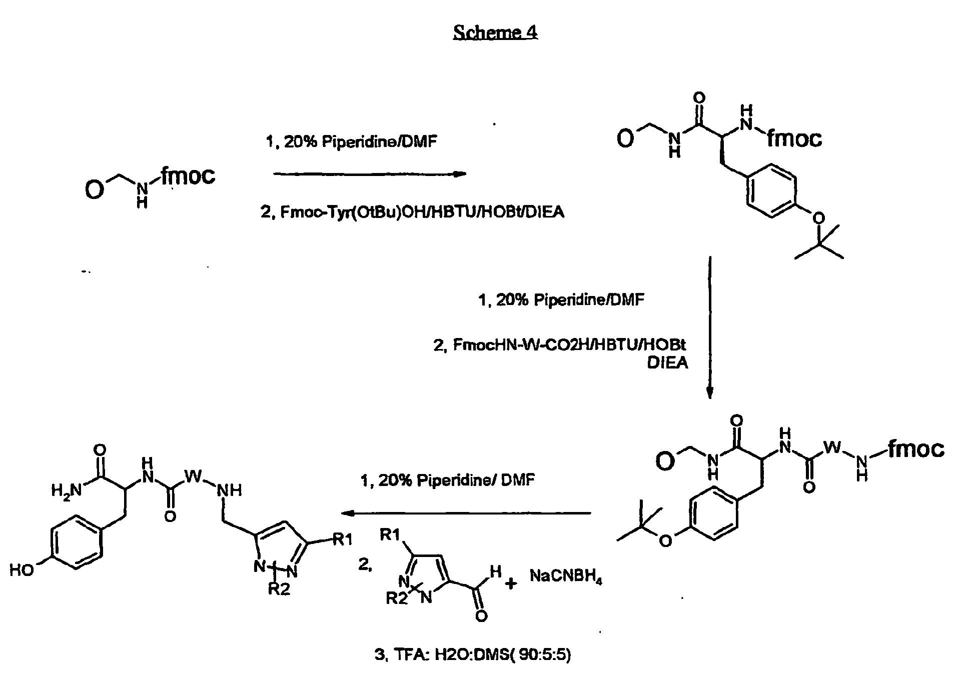

Scheme 4 above depicts the synthesis by combinatorial methods of pyrazole libraries containing a heteroatom on the side chain, particularly compounds of Formulae I, I' and IA where R¹ and R² are as defined above for Formulae I, I', and IA, R³ is H, X is heteroalkylene chain and Y is CO and Z is tyrosine. It should be recognized that while all the reactions shown depict tyrosine as the amino acid ( Z = Tyr), by using the appropriate amino acid in the first coupling step other amino acid derivatives may be prepared. In scheme 4, W is C₁-C₄ alkyl or 1,3- or 1,4 phenyl radical. All of the reactions shown depict standard coupling reactions which are known to those skilled in the art. The pyrazoles with carboxylic acid or carboxaldehyde in the 3 (or 5) position may be prepared by standard literature procedures as depicted below.

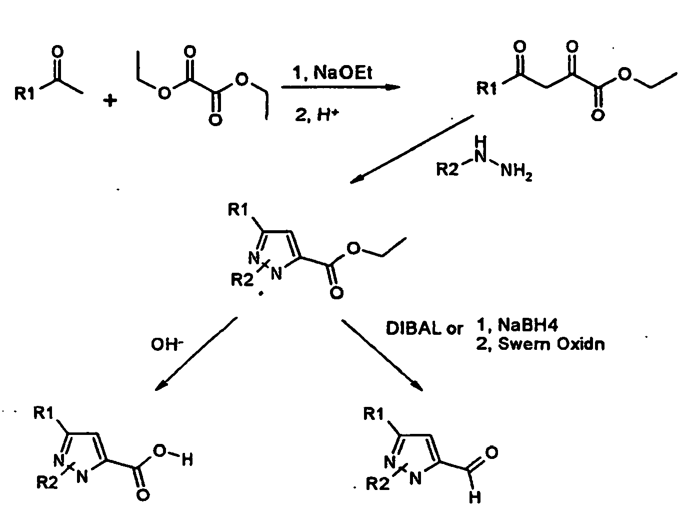

As already mentioned, compounds of the invention including specific regioisomers can be prepared by solution phase synthetic procedures as outlined in the following exemplary Schemes 5 and 6.

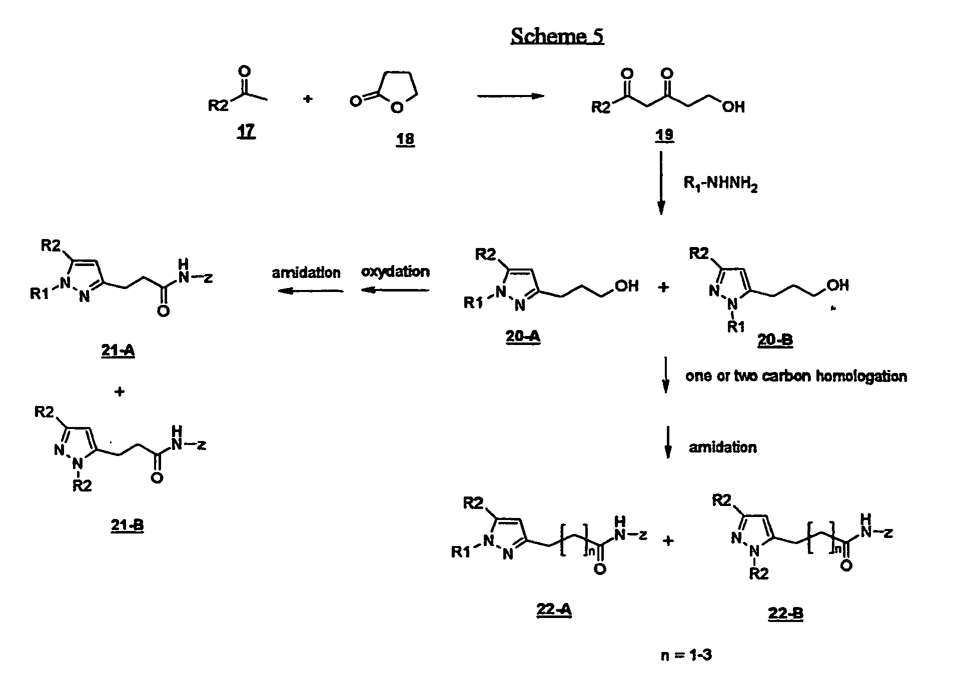

Compounds of general formula 20 where R¹ and R² have the same definition as in Formula I can be obtained by reaction of β-diketoalcohol 19 with suitable hydrazine as depicted in scheme 5. This procedure is a preferred one as it gives rise to a better regioselectivity in favor of the 20-B pyrazole regioisomer (see W.V. Murray, Tetrahedron Lett., 34, p.1863,1993). The hydroxyalkyl pyrazole thus obtained can be oxidized and reacted with an amine or amino-acid ZNH₂ where Z is defined as in Formula I using standard amidation conditions (HATU or EDC/HOAT/DIEA for example) to give compounds 21. Homologation by one or two carbons using methods well known to those skilled in the art followed by an amidation step can provide compounds 22.

As an example of such transformation, the hydroxyalkyl derivative 19 can be treated first with iodine in presence of triphenylphosphine and imidazole, then with the anion of *tert-*butyloxyacetate and finally with trifluoroacetic acid to give the corresponding carboxylic acid. Compounds of formula 22 where n=3 can be obtained from such carboxylic acid by coupling with appropriate amine or amino acid ZNH₂ using standard conditions (as HATU or EDC/HOAT/DIEA).

A second preferred synthetic method for obtaining pyrazoles of the present invention is described in scheme 6. It involves the preparation of hydrazone 24 from an acetyl derivative 23 where R¹ and R² have the same definition as in Formula I and the appropriate hydrazine. The dianion of the hydrazone is then reacted with an anhydride (RCO)₂O to give the pyrazole 25 where R is (X)ₘ-(Y)ₙ-Z defined as in Formula I or a synthetic precursor which can be easily transformed to (X)ₘ-(Y)ₙ-Z by methods well known to those skilled in the art. An example of the conditions that can be used for such transformation can be found in S.R. Stauffer, Y. Huang, C.J. Coletta, R Tedesco, J.A. Katzenellenbogen, Biorg. Med. Chem. 2001, 9, 141- 150.

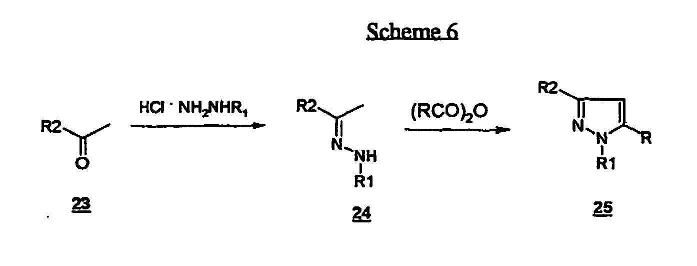

In the case of compounds of Formula IC above, suitable methods for preparation include those exemplified in the following Schemes 7 and 8.

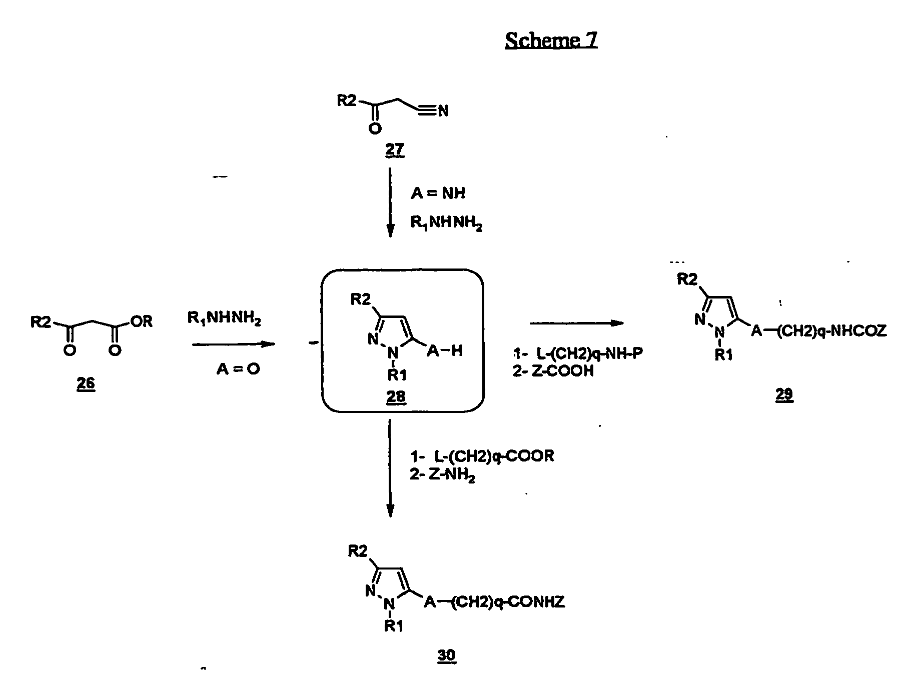

As showed in Scheme 7 above, compounds of general formula 29 and 30 wherein R¹, R² and Z are defined as in Formula I above, A is O or NH and q is a number between 1 and 6, may be obtained by alkylation of an intermediate such as 28 with a reactant such as L-(CH₂)q-NH-P wherein L is a leaving group (like OMs, OTs,CI, Br or I) and P an appropriate protecting group. After appropriate protection/deprotection steps, the intermediate thus obtained can be submitted to standard amidation conditions well known by those skilled in the art. Depending on the nature of A, intermediate 28 can be obtained by reaction with suitable hydrazine R¹NHNH₂ either from β-ketoester 26 if A is O or β-keto-nitrile if A is NH. Examples of specific conditions for those reactions can be found in PCT Int.Appl 9712884 and M.J.Fray, D.J. Bull, M.Kinns, J.ChemResearch (S), p.11 (1992).

Some compounds of Formula I-C above can be easily obtained from intermediates 20-A and B (see Scheme 5) using synthetic methods well known by those skilled in the art. Scheme 8 below illustrates one of those possible methods yielding to compounds 32-A and B wherein R¹, R² and Z are defined as in Formula I. It should be appreciated that the methods shown in scheme 7 and 8 are exemplary only and that a variety of other methods can be employed to obtained compounds of Formula I-C.

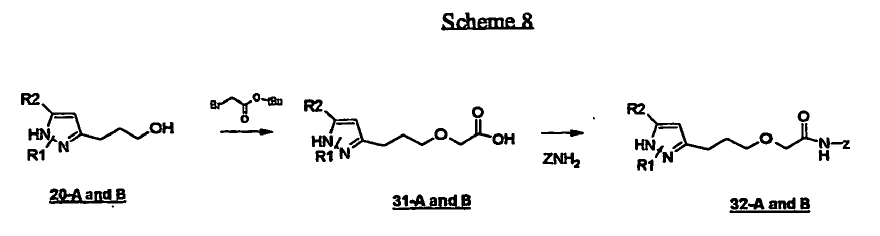

As discussed above, the present invention includes methods for treating testosterone deficiency in male mammals, such as primates, particularly humans. Compounds of the invention will be useful for treatment of infertility conditions currently treated with testosterone replacement, including male infertility and male spermatogenesis disorders.

The therapeutic methods of the invention generally comprise administration of an effective amount of one or more compounds of the invention to a subject including a mammal, such as a primate, especially a human, in need of such treatment.

Typical candidates for treatment in accordance with the methods of the invention persons suffering from or suspected of suffering from testosterone deficiency. Compounds of the invention also can be administered to males to facilitate adequate spermatogenesis.

The treatment methods of the invention also will be useful for treatment of testosterone deficiency conditions, disorders or diseases in mammals other than humans, such as horses and livestock e.g. cattle, sheep, cows and the like.

A preferred embodiment of the invention consists in the use of compounds of the invention for the manufacture of a medicament for the treatment and/or prevention of disorders or diseases requiring exogenous administration of testosterone preparation, such as AIDS wasting, muscle dystrophy, cachexia, aging, testosterone deficiency and infertility.

A further preferred embodiment of the invention consists in the use of compounds of the invention for the manufacture of a medicament for the treatment and/or prevention of fertility disorders, preferably male infertility and spermatogenesis.

An another preferred embodiment of the invention consists in the previous listed uses wherein the medicament further comprises one or more a fertility agents for simultaneous, sequential or separate use.

Compounds of the invention may be administered as a "cocktail" formulation, i.e. coordinated administration of one or more compounds of the invention together with one or more other active therapeutics, particularly one or more other known fertility agents.

The compounds of this invention can be administered by a variety of routes, such as orally or by injection, e.g., intramuscular, intraperitoneal, subcutaneous or intravenous injection, or topically such as transdermally, vaginally and the like. Compounds of the invention may be suitably administered to a subject in the protonated and water-soluble form, e.g., as a pharmaceutically acceptable salt of an organic or inorganic acid, e.g., hydrochloride, sulfate, hemi-sulfate, phosphate, nitrate, acetate, oxalate, citrate, maleate, mesylate, etc.

Compounds of the invention can be employed, either alone or in combination with one or more other therapeutic agents as discussed above, as a pharmaceutical composition in mixture with conventional excipient, i.e., pharmaceutically acceptable organic or inorganic carrier substances suitable for oral, parenteral, enteral or topical application which do not deleteriously react with the active compounds and are not deleterious to the recipient thereof Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds.

For oral administration, pharmaceutical compositions containing one or more substituted pyrazole compounds of the invention may be formulated as e.g. tablets, troches, lozenges, aqueous or oily suspensions, dispersible powders or granules, emulsions, hard or soft capsules, syrups, elixers and the like. Typically suitable are tablets, dragees or capsules having talc and/or carbohydrate carrier binder or the like, the carrier preferably being lactose and/or corn starch and/or potato starch. A syrup, elixir or the like can be used wherein a sweetened vehicle is employed. Sustained release compositions can be formulated including those wherein the active component is protected with differentially degradable coatings, e.g., by microencapsulation, multiple coatings, etc.

For parenteral application, e.g., sub-cutaneous, intraperitoneal or intramuscular, particularly suitable are solutions, preferably oily or aqueous solutions as well as suspensions, emulsions, or implants, including suppositories. Ampules are convenient unit dosages.

For topical applications, formulations may be prepared in a topical ointment or cream containing one or more compounds of the invention. When formulated as an ointment, one or more compounds of the invention suitably may be employed with either a paraffinic or a water-miscible base. The one or more compounds also may be formulated with an oil-in-water cream base. Other suitable topical formulations include e.g. lozenges and dermal patches.

It will be appreciated that the actual preferred amounts of active compounds used in a given therapy will vary according to the specific compound being utilized, the particular compositions formulated, the mode of application, the particular site of administration, etc. Optimal administration rates for a given protocol of administration can be readily ascertained by those skilled in the art using conventional dosage determination tests conducted with regard to the foregoing guidelines. See also Remington's Pharmaceutical Sciences. In general, a suitable effective dose of one or more compounds of the invention, particularly when using the more potent compound(s) of the invention, will be in the range of from 0.01 to 100 milligrams per kilogram of bodyweight of recipient per day, preferably in the range of from 0.01 to 20 milligrams per kilogram bodyweight of recipient per day, more preferably in the range of 0.05 to 4 milligrams per kilogram bodyweight of recipient per day. The desired dose is suitably administered once daily, or several sub-doses, e.g. 2 to 4 sub-doses, are administered at appropriate intervals through the day, or other appropriate schedule. Such sub-doses may be administered as unit dosage forms, e.g., containing from 0.05 to 10 milligrams of compound(s) of the invention, per unit dosage.

The entire text of all documents cited herein are incorporated by reference herein. The following non-limiting examples are illustrative of the invention.

### Example 1: In vivo Testosterone Induction Activity

Testosterone induction activity was determined as follows: On day 1, adult male Sprague-Dawley rats were implanted subcutaneously in the scalpular region with a single 7-day release pellet containing 7.5 milligrams of diethylstilbestrol (DES) in order to suppress endogenous luteinizing hormone release and subsequent testosterone production. Two days after the DES pellet implantation, rats were injected intraperitoneally with either Compound A at 0, 1, 4, or 8 mg/kg or Compound B at 0, 1, 2 and 4 mg/kg. Three rats were used in each treatment group. Blood samples removed from the rats by retro-orbital plexus disruption after two hours following the administration of the Compounds. Serum was collected from blood by centrifugation and frozen at -80 degrees Celsius until the time of assay. Serum testosterone levels were determined using a selective testosterone immunoassay. The average testosterone concentrations and standard deviations were determined using Microsoft Excel software program. As seen in Table 1 below, the results demonstrate that the test compounds induce increased testosterone concentrations in DES-treated adult male rats. Compound A is 5-[2-(4-tert-Butyl-phenyl)-5-pyridin-3-yl-2H-pyrazol-3-yl]-pentanoic acid [1-carbamoyl-2-(4-hydroxy-phenyl)-ethyl]-amide and Compound B is 5-[2-(4-tert-Butyl-phenyl)-5-pyridin-4-yl-2H-pyrazol-3-yl]-pentanoic acid [1-carbamoyl-2-(4-hydroxy-phenyl)-ethyl]-amide.

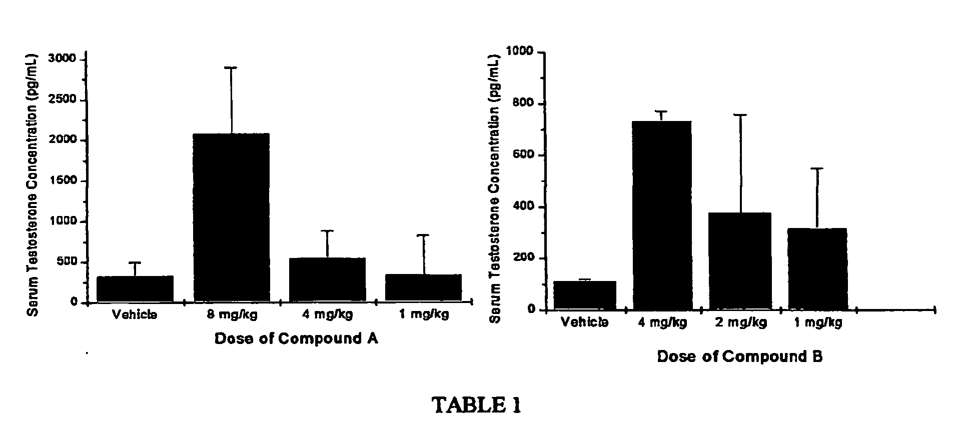

Example 2: Synthesis of N-(5-[1-(4-*tert*-butylphenyl)-5-pyridin-3-yl-1H-pyrazol-3-yl]pentanoyl)tyrosinamide (1-A) and N-(5-[1-(4-*tert*-butylphenyl)-3-pyridin-3-yl-1H-pyrazol-5-yl]pentanoyl)tyrosinamide (1-B) on solid phase

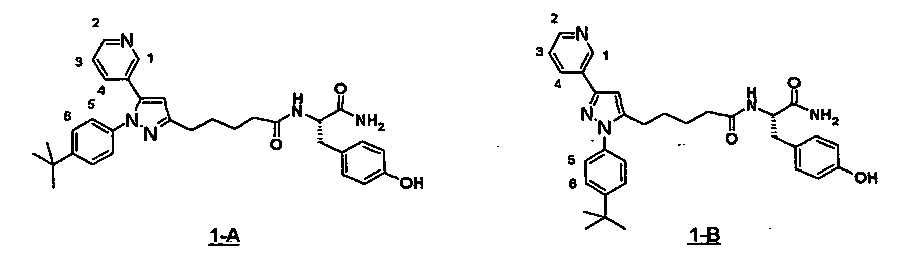

Rink-amide resin (30g, loading = 0.65mmol/g) was shaken in a large peptide vessel in a 20% piperidine solution in DMF (200mL) for 30 min. This operation was repeated 2 times. The resin was then washed with DMF (3x), NMP (2x), MeOH (2x) and DCM (2x) and dried under vacuum for 30 min The deprotected resin was transferred into a 1 L bottle and shaken for 27h in a solution containing Fmoc-Tyr(tBu)-OH (90g), HATU (74g) and DIEA (60mL) in 360 mL of DMF. After this time, the resin was transferred into a peptide vessel, washed-with DMF (3x), DCM (2x), MeOH (2x), DMF (1x),DCM (1x) and dried under vacuum for 15 min. It was then shaken in a 20% piperidine solution in DMF (200mL) for 30 min 3 times, washed with DMF (3x), NMP (2x), MeOH (2x), DCM (2x) and dried under vacuum for 15 min. For the next step, the resin was transferred again into a 1 L bottle and shaken for 48h in a solution of acetylvaleric acid (28.1g), HATU (74.1g) and DIEA (66mL) in DMF (360 mL). Same washing procedure as for the preceding coupling step was followed. The resin was dried under vacuum and divided into two portions of about 32g (portion 1) and 31g (portion 2).

Portion 1 was transferred into a 1L bottle flushed with nitrogen containing a solution of methylnicotinate (40g) in 200mL of DMA Sodium hydride 95% (70g) was then added in small portions into the mixture with constant nitrogen flushing. The bottle was placed in an ice bath and agitated by hand until the hydrogen release had stopped. It was then heated at 85°C for 2 hours with occasional hand agitation. After that time, the mixture was chilled to room temperature and poured slowly into a beaker containing ice and a solution of 15% acetic acid in water. Transfer into a peptide vessel was operated before the standard washing and drying steps. The 18g of resin thus obtained were poured into a 1L bottle containing 4-tert-butylpherrylhhydrazine hydrochloride (39.1g) and DIEA (33.1 mL) in DMA (360 mL) and heated for 24 hours at 80°C. The bottle was chilled to room temperature and the resin was washed and dried following standard procedure. Finally, it was cleaved with 100mL of DCM/TFA (2:1) and the residue was concentrated under reduced pressure. It was dissolve in ethyl acetate and washed with slightly basic solution (pH ~8.5-9 obtained with diluted NH₄OH). The organic layer was dried over magnesium sulfate, filtered and concentrated to give 3.65g of crude (yield ~70%). This crude contained a mixture of the two possible pyrazole regioisomers 1A and 1B with a ratio of about 9:1. The two regioisomers were separated by reversed phase HPLC using DELTAPAK C₁₈ column with a linear gradient of 0.1% TFA water/acetonitrile 95:5 to 60:40 in one hour.

Structures were assigned by Nuclear Overhauser experiments performed on a JEOL 400 MHz NMR apparatus. There were NOE cross peaks displayed between the proton 5 and proton 1 and 4 for isomer A, indicating that the phenyl ring is close from the pyridine ring and no NOE effect observed between those protons for isomer B.

### Isomer 1-A (more polar): trifluoroacetate salt of N-(5-[1-(4-tert-butylphenyl)-5-pyridin-3-yl-1H-pyrazol-3-yl]pentanoyl)tyrosinanude

¹H NMR (DMSO) : 1.28 (s, 9H), 1.55 (m, 4H), 2.12 (t, J = 7.3Hz, 2H), 2.63 (m, 3H), 2.86 (dd, J = 5.3 and 13.9 Hz, 1H), 4.35 (m, 1H), 5.60 ( brs, 1H), 6.61 (s, 1H), 6.62 ( d, J = 8.8 Hz, 2H), 6.98 (m, 1H), 7.01 (d, J = 8.8 Hz, 2H), 7.19 (d, J = 8.8 Hz, 2H), 7.36 (m, 1H), 7.42 (d, J = 8.8 Hz, 2H), 7.51 (dd, J = 8.1 and 5.1 Hz, 1H), 7.75 (dt, J = 8.1 and 2.2 Hz, 1H), 8.52 (dd, J = 2.2 and 0.73 Hz, 1H), 8.58 (dd, J = 4.9 and 1.5 Hz, 1H).

MS (ESI, pos.) : 540 (M+1)

### Isomer 1-B (less polar) : trifluoroacetate salt of N-(5-[1-(4-tert-butylphenyl)-3-pyridin-3-yl-1H-pyrazol-5-yl]pentanoyl)tyrosinamide

¹H NMR (DMSO): 1.34 (s, 9H), 1.47 (m, 4H), 2.05 (t, J = 9.1 Hz, 2H), 2.61 (m, 1H), 2.64 (t, J = 6.9 Hz, 2H), 2.84 (m, 1H), 4.34 (m, 1H), 6.30 (brs, 1H), 6.59 ( d, J = 8.4 Hz, 2H), 6.98 (m, 1H), 6.99 (d, J = 8.4 Hz, 2H), 7.02 (s, 1H), 7.37 (m, 1H), 7.48 (d, J = 8.8 Hz, 2H), 7.58 (d, J = 8.8 Hz, 2H), 7.86 (d, J = 8.4 Hz, 1H), 7.92 (dd, J = 8.1 and 5.5 Hz, 1H), 8.72 (dt, J=8.4 and 1.46 Hz, 1H), 8.76 (dd, J = 5.49 and 1.46 Hz, 1H), 9.24 (d, J = 2.2 Hz, 1H).

MS (ESI, pos.) : 540 (M+1)

### Example 3 : Synthesis of N-(5-[1-(4-tert-butylphenyl)-5-pyridin-4-yl-1H-pyrazol-3-yl]pentanoyl)tyrosinamide (2-A) and N-(5-[1-(4-tert-butylphenyl)-3-pyridin-4-yl-1H-pyrazol-5-yl]pentanoyl)tyrosinamide (2-B)

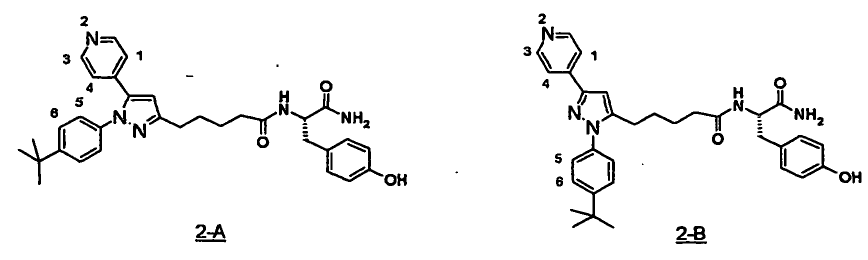

Those compounds were synthesized from the portion 2 (3 1 g) of resin obtained in Example 1 above following exactly the same procedure. All the reagents and quantities are the same except that methylnicotinate was replaced by methylisonicotinate.

2.5g of crude were obtained (48% yield) and contained a mixture of the two possible pyrazole regioisomers 2A and 2B with a ratio of about 9:1. The two regioisomers were separated using the same conditions as those described in example 1. The structures were assigned by analogy with compounds 1A and 1B.

### Isomer 2-A (more polar): trifluoroacetate salt of N-(5-[1-(4-tert-butylphenyl)-5-pyridin-4-yl-1H-pyrazol-3-yl]pentanoyl)tyrosinamide

¹H NMR (DMSO) : 1.29 (s, 9H), 1.52 (m, 4H), 2.10 (t, J = 7.3Hz, 2H), 2.55 (m, 3H), 2.83 (dd, J = 4.4 and 14.3 Hz, 1H), 4.35 (m, 1H), 6.61 ( d, J = 6.6 Hz, 2H), 6.77 (s, 1H), 7.00 (s, 1H), 7.01 (d, J = 6.9 Hz, 2H), 7.22 (d, J = 6.6 Hz, 2H), 7.38 (m, 3H), 7.45 (d, J = 6.9 Hz, 2H), 7.88 (d, J = 8.4 Hz, 1H), 8.63 (d, J = 4.8 Hz, 2H), 9.2 (brs, 1H).

MS (ESI, pos.) : 540 (M+1)

### Isomer 2-B (less polar) : trifluoroacetate salt of N-(5-[1-(4-tert-butylphenyl)-3-pyridin-4-yl-1H-pyrazol-5-yl]pentanoyl)tyrosinamide

¹H NMR (DMSO) : 1.34 (s, 9H), 1.43 (m, 4H), 2.04 (t, J = 9.1 Hz, 2H), 2.50 (m, 1H), 2.64 (m, 2H), 2.83 (dd, J = 3.3 and 9.2 Hz,1H), 4.35 (m, 1H), 6.58 ( d, J = 6.9 Hz, 2H), 6.98 (m, 3H), 7.14 (s, 1H), 7.38 (s, 1H), 7.49 (d, J = 6.9 Hz, 2H), 7.59 (d, J = 6.9 Hz, 2H), 7.86 (d, J = 8.4 Hz, 1H), 8.17 (d, J = 4.7 Hz, 2H), 8.78 (d, J = 4.1 Hz, 2H), 9.2 (brs, , 1H).

MS (ESI, pos.) : 540 (M+1)

### Elemental analysis: C₃₂H₃₇N₅O₃.TFA.1,5H₂O, theoretical: C, 59.99%; H, 6.07%: N, 10.29%; experimental: C, 60.12% ; H, 5.95% : N, 10.27%.

### Example 4: Combinatorial synthesis of pyrazole library of the invention.

A library of ~2500 pyrazoles synthesized via Fmoc/t-Butyl chemistry using either IRORI AccuTag / Robin technology. 20 microkans each in three glass bottles containing Fmoc Rink Amide MBHA resin (25mg, 0.59 mmol/g) were treated with 20% piperidine/DMF (30mL, 3 x 30min) and then rinsed with DMF and acylated with 0.1 M solution of Fmoc amino acid (3 mmlol) in DMF (30mL), HATU (3 mmol) and DIEA (6 mmol) at room temperature for 16 hours, rinsed with DMF. Fmoc was removed with 20% piperidine/DMF (100mL, 3 x 30min), rinsed with DMF. 30 microkans in two glass bottles resin were acylated with 0.1 M solution of acetyl carboxylic acid (4.5mmol) in DMF (45mL), HATU (4.5 mmol) and DIEA (9 mmol) at room temperature for 16 hours and then rinsed with DMF. Claisen condensation was carried out with 30 microkans in two glass bottles with 0.25 M solution of ester (11.25mmol), 95% NaH (11.25mmol) in DMA at 90°C in the oven for 2 hours. The microkans were washed (DMA, MeOH, DMF and CH2CI₂) and dried under reduced pressure. Cyclization was carried out with 12 microkans in 5 glass bottles with 0.125M solution of hydrazine (2.25mmol), DIEA (2.25mmol) in DMA (18 mL) at 80°C in the oven for 24 hours, rinsed and dried under reduced pressure. The final cleavage of resin was carried out with TFA at 2 hours in the IRORI cleavage block. The residue was then co-evaporated with CH₃CN under reduced pressure.

All of the crude pyrazoles were analyzed by reversed phase HPLC using DELTAPAK C₁₈, 5µM column, eluted with a linear gradient of 0.1% TFA in CH₃CN/water (0% CH₃CN/100% water) to 0.1% TFA in CH₃CN/water (100% CH₃CN/0% water) over a 30 minute period with flow rate of 1.5mL/minute. The purity of the samples were determined and were essentially found to contain the pyrazole in 60% purity as a mixture of regioisomers. The mass were confirmed by matrix-assisted laser desorption ionization time of flight mass spectral analysis (MALDI-TOF, PE Biosystem, Inc.). Generally the pyrazoles gave the mass of MH+ or M+Na+ or within experimental error of the calculated value.

### Example 5: Compound purification.

The crude pyrazoles prepared in Example 3 above were purified by reversed phase HPLC using DELTAPAK C₁₈, 5µM column, eluted with a linear gradient of 0.1% TFA in CH₃CN/water (0% CH₃CN/100% water) to 0.1% TFA in CH₃CN/water (100% CH₃CN/0% water) over a 30 minute period with flow rate of 15mL/minute. The purity of the samples were determined and were essentially found to contain one component. The mass were confirmed by matrix-assisted laser desorption ionization time of flight mass spectral analysis (MALDI-TOF, PE Biosystem, Inc.). Generally the pyrazoles gave the mass of MH+ or M+Na+ or within experimental error of the calculated value.

For example, the following compounds were prepared by this procedure:
N-{5-[1-(4-tert-butylphenyl)-3-pyridin-2-yl-1H-pyrazol-5-yl]pentanoyl}tyrosinamide, [M +H] = 540;
N-{5-[1-(4-tert-butylphenyl)-3-pyridin-3yl-1H-pyrazol-5yl]pentanoyl}tyrosinamide, [M+H] = 540;
N-{5-[1-(4-tert-butylphenyl)-5-pyridin-3 yl-1H-pyrazol-3yl]pentanoyl}tyrosinamide, [M+H] = 540;
N-{5-[1-(4-tert-butylphenyl)-3-pyridin-4-yl-1H-pyrazol-5-yl]pentanoyl}tyrosinamide, [M +H] = 540;
N-{5-[1-(4-tert-butylphenyl)-5-pyridin-4-yl-1H-pyrazol-3 yl]pentanoyl}tyrosinamide, [M +H] = 540;
N-{5-[1-(4-tert-butylphenyl)-3-pyridin-3-yl-1H-pyrazol-5-yl]pentanoyl}-N,N-dimethyltyrosinamide, [M +H] = 554;
N-[4-(1-butyl-3-isoquinolin-3-yl-1H-pyrazol-5-yl)benzoyl]tyrosinamide, [M +H] = 534;
N-{5-[1-(4-isopropylphenyl)-3-pyridin-3-yl-1H-pyrazol-5-yl]pentanoyl}tyrosinamide, [M +H] = 526;
N-{6-[1-(4-isopropylphenyl)-3-pyridin-2-yl-1H-pyrazol-5-yl]hexanoyl}tyrosinamide, [M +H] = 540;
N-{6-[1-(4-tert-butylphenyl)-3-pyridin-2-yl-1H-pyrazol-5-yl]hexanoyl}-3-hydroxyphenylalaninamide, [M +H] = 554;
N-[1-(aminocarbonyl)-3-(4-hydroxyphenyl)propyl]-5-[1-(4-tert-butylphenyl)-3-pyridin-2-yl-1H-pyrazol-5-yl]pentanamide, [M +H] = 554;
N-[5-(1-butyl-3-isoquinolin3-yl-1H-pyrazol-5-yl)pentanoyl]tyrosinamide, [M+H] = 514;
N-{6-[1-(4-tert-butylphenyl)-3-pyridin-2-yl-1H-pyrazol-5-yl]hexanoyl}serinamide, [M +H] = 478; N-{6-[1-(4-iso-propylphenyl)-3-pyridin-2-yl-1H-pyrazol-5-yl]hexanoyl}serinamide, [M +H]= 464;
N-{6-[1-(4-iso-propylphenyl)-3-pyridin-2-yl-1H-pyrazol-5-yl]hexanoyl}threonamide, [M+H] = 478;
N-{5-[1-(4-isopropylphenyl)-3-pyridia-2-yl-1H-pyrazol-5-yl]pentanoyl}tyrosinamide, [M +H] = 526;
6-[1-(4-tert-butylphenyl)-3-pyridin-2-yl-1H-pyrazol-5-yl]-N-[2-(4-hydroxyphenyl)ethyl]hexanamide, [M +H] = 511;
N-{6-[1-(4-tert-butylphenyl)-3-pyridin-2-yl-1H-pyrazol-5-yl]hexanoyl}tyrosinamide, [M +H] = 554;
N-{5-[1-(4-tert-butylphenyl)-3-isoquinolin-3-yl-1H-pyrazol-5-yl]pentanoyl}tyrosinamide, [M +H] = 590;
N-{6-[1-(4-tert-butylphenyl)-3-isoquinolin-3-yl-1H-pyrazol-5 yl]hexanoyl}tyrosinamide, [M +H] = 604;
N-{6-[1-(4-tert-bulylphenyl)-3-pyridin-2-yl-1H-pyrazol-5-yl]hexanoyl}-N-methyltyrosinamide, [M+H] = 568;
N-{6-[1-(4-tert-butylphenyl)-3-pyridin-2-yl-1H-pyrazol-5-yl]hexanoyl}-4-(hydroxymethyl)phenylalaninamide, [M +H] = 568;
4-amino-N-{6-[1-(4-tert-butylphenyl)-3-pyridin-2-yl-1H-pyrazol-5-yl]hexanoyl}phenylalaninamide, [M +H] = 553;
4-(acetylamino)-N-{6-[1-(4-tert-butylphenyl)-3-pyridin-2-yl-1H-pyrazol-5-yl]hexanoyl}phenylalaninamide, [M +H] = 595;
4-(aminocarbonyl)-N-{6-[1-(4-tert-butylphenyl)-3-pyridin-2-yl-1H-pyrazol-5-yl]hexanoyl}phenylalaninamide, [M +H] = 581;
N-butyl-N-{6-[1-(4-tert-butylphenyl)-3-pyridin-2-yl-1H-pyrazol-5-yl]hexanoyl}tyrosinamide, [M +H] = 610;
N-{6-[1-(4-tert-butylphenyl)-3-pyridin-2-yl-1H-pyrazol-5-yl]hexanoyl}threonamide, [M+H]= 492;
N-[2-amino-1-(4-hydroxyphenyl)-2-oxoethyl]-6-[1-(4-tert-butylphenyl)-3-pyridin-2-yl-1H-pyrazol-5-yl]hexanamide, [M+H] = 540;
N-({5-[1-(4-isopropylphenyl)3-pyridin-2-yl-1H-pyrazol-5-yl]thien-2-yl}carbonyl)tyrosinamide, [M +H] = 552;
N-[2-amino-1-(4-hydroxyphenyl)-2-oxoethyl]-4-[1-(4-tert-butylphenyl)-3-pyridin-2-yl-1H-pyrazol-5-yl]benzamide, [M +H] = 546;
4-[1-(4-tert-butylphenyl)-3-pyridin-2-yl-1H-pyrazol-5-yl]-N-[2-(4-hydroxyphenyl)ethyl]benzamide, [M +H] = 517;
N-{3-[1-(4-tert-butylbenzyl)-3-isoquinolin-3-yl-1H-pyrazol-5-yl]benzoyl}tyrosinamide, [M+H] = 608;
N-{5-[1-(4-tert-butylphenyl)-3-pyridin-4-yl-1H-pyrazol-5-yl]pentanoyl}tyrosinamide, [M+H] = 540;
N-{3-[1-(4-tert-butylbenzyl)-3-pyridin-3-yl-1H-pyrazol-5-yl]benzoyl}tyrosinamide, [M+H] = 574;
N-{3-[1-(4-tert-butylphenyl)-3-isoquinolin-3-yl-1H-pyrazol-5 yl]benzoyl}tyrosinamide, [M+H] = 610;
N-[4-(1-butyl-3-isoquinolin-3-yl-1H-pyrazol-5-yl)benzoyl]tyrosinamide, [M +H] = 534;
N-{3-[1-(4-tert-butylbenzyl)-3-quinolin-3-yl-1H-pyrazol-5-yl]benzoyl}tyrosinamide, [M +H] = 624;
N-{3-[3-isoquinolin 3-yl-1-(4-propylphenyl)-1H-pyrazol-5-yl]benzoyl}tyrosinamide, [M +H] = 610;
N-{3-[1-(4-tert-butylphenyl)-3-pyridin-3-yl-1H-pyrazol-5-yl]benzoyl}tyrosinamide, [M +H] = 560;
N-{4-[1-(4-tert-butylphenyl)-3-pyridin-3-yl-1H-pyrazol-5-yl]benzoyl}tyrosinamide, [M+H] = 560;
N-{5-[1-(4-tert-butylbenzyl)-3-pyridin-4-yl-1H-pyrazol-5-yl]pentanoyl}tyrosinamide, [M+H] = 554;
N-{3-[1-(4-tert-butylbenzyl)-3-pyridin-4-yl-1H-pyrazol-5-yl]benzoyl}tyrosinamide, [M+H] = 574;
N-{5-[1-(4-tert-butylbenzyl)-3-pyridin-3-yl-1H-pyrazol-5-l]pentanoyl}tyrosinamide, [M+H] = 554;
N-[3-(1-butyl-3-isoquinolin-3-yl-1H-pyrazol-5-yl)benzoyl]tyrosinamide, [M +H] = 534;
N-{4-[1-butyl-3-(2-furyl)-1H-pyrazol-5-yl]benzoyl}tyrosioamide, {M+H] = 473;
N-{5-[1-butyl-3-(2-furyl)-1H-pyrazol-5-yl]pentanoyl}tyrosinamide, [M+H] = 453;
N-{3-[3-(2-furyl)-1-(4-isopropylphenyl)-1H-pyrazol-5-yl]propanoyl}tyrosinamide, {M+H] = 487;
N-(4-{1-(4-tert-butylphenyl)-3-[3-(dimethylamino)phenyl]-1H-pyrazol-5-yl}benzoyl)tyrosinamide, [M+H] = 602;
N-[1-(aminocarbonyl)-3-(4-hydroxyphenyl)propyl]-4-[3-(2-furyl)-1-(4-isopropylphenyl)-1H-pyrazol-5-yl]benzamide, [M+H] = 549;
N-[1-(aminocarbonyl)-3-(4-hydroxyphenyl)propyl]-5-{1-(4-tert-butylphenyl)-3-[3-(dimethylamino)phenyl]-1H-pyrazol-5-yl}pentanamide, [M+H] = 596;
N-{5-[1-(4-tert-butylphenyl)-3-(2-furyl)-1H-pyrazol-5-yl]pentanoyl}tyrosinamide, [M+H] = 529;
N-[1-(aminocarbonyl)-3-(4-hydroxyphenyl)propyl]-4-[3-(2-finyl)-1-pyridin-2-yl-1H-pyrazol-5-yl]benzamide, [M+H] = 508; ,
N-(4-{3-[3-(dimethylamino)phenyl]-1-pyridin-2-yl-1H-pyrazol-5-yl}benzoyl)tyrosinamide, [M+H] = 547;
N-(5-{1-butyl-3-[3-(dimethylamino)phenyl]-1H-pyrazol-5-yl}pentanoyl) tyrosinamide, [M+H] = 506;
N-(5-{1-(4-tert-butylphenyl)-3-[3-(dimethylamino)phenyl]-1H-pyrazol-5-yl}peatanoyl)tyrosinamide, [M+H] = 582;
N-[5-(1-butyl-3-quinolin-3-yl-1H-pyrazol-5-yl)pentanoyl]tyrosinamide, [M+H] = 514;
N-{5-[1-(4-tert-butylphenyl)3-quinolin-3-yl-1H-pyrazol-5-yl]pentanoyl} tyrosinamide, [M+H} = 590;
N-[5-(1-butyl-3-quinolin-6-yl-1H-pyrazol-5-yl)pentanoyl]tyrosinamide, [M+H] = 514;
N-{5-[1-(4-tert-butylphenyl)-3-quinolin-6-yl-1H-pyrazol-5-yl]pentanoyl} tyrosinamide, [M+H] = 590;
N-{5-[1-(4-tert-butylbenzyl)-3-quolin-6-yl-1H-pyrazol-5-yl]pentanoyl} tyrosinamide, [M+H] = 604;
N-[5-(1-hexyl-3-pyrazin-2-yl-1H-pyrazol-5 yl)pentanoyl]tyrosinamide, [M+H] = 493;
N-[(1-butyl-3-pyridin-3-yl-1H-pyrazol-5-yl)methyl]glycyltyrosinamide; [M+H] = 451;
N-{[1-(4-tert-butylphenyl)-3-pyridin-3-yl-1H-pyrazol-5-yl]methyl} glycyltyrosinamide, [M+H] = 527;
N-[(1-butyl-3-pyridin-3-yl-1H-pyrazol-5-yl)methyl]-beta-alanyltyrosinamide, [M+H] = 465;
N-(3-{[(1-butyl-3-pyridin-3-yl-1H-pyrazol-5-yl)methyl]amino}benzoyl)tyrosinamide, [M+H] = 513;
N-[3-({[1-(4-tert-butylphenyl)-3-pyridin-4-yl-1H-pyrazol-5-yl]methyl}amino)benzoyl]tyrosinamide, [M+H] = 589;
N-({1-[(1-butyl-3-pyridin-3-yl-1H-pyrazol-5-yl)methyl]piperidin-4-yl}carbonyl) tyrosinamide, [M+H] = 505;
N-[(1-{[1-(4-tert-butylphenyl)-3-pyridin-3yl-1H-pyrazol-5yl]methyl}piperidin-4-yl)carbonyl]tyrosinamide, [M+H] = 581;
N-2--{[1-(4-tert-butylphenyl)-3-pyridin-3-yl-1H-pyrazol-5-ylmethyl}-N~1∼-[2-(4-hydroxyphenyl)ethyl]glycinamide; [M+H] = 484;
N-3-[(1-butyl-3-pyridin-3-yl-1H-pyrazol-5-yl)methyl]-N~1~-[2-(4-hydroxyphenyl)ethyl]-beta-alaninamide, [M+H] = 422;
4-{[(1-butyl-3-pyridin-3-yl-1H-pyrazol-5-yl)methyl]amino} N-[2-(4-hydroxyphenyl)ethyl]benzamide, [M+H] = 470.

### Example 6: Regioselective synthesis of pyrazoles

Commercially available Fmoc-protected rink amide resin (0.7g) was deprotected with 20% piperidine in DMF, rinsed and acylated with 0.5M solution of Fmoc-amino acid (10 equiv)/HATU (10 equiv)/DIEA (20equiv) overnight in DMF at room temperature, rinsed with DMF. Fmoc group was removed with 20% piperidine/DMF and resin was rinsed with DMF, acylated with 0.5M solution of acetyl carboxylic acid (10 equiv)/HATU (10 equiv)/DIEA (20equiv) overnight at room temperature, rinsed with DMF. The resin was added with LiOH.H2O (40 equivalents) in anhydrous DME and 40 equivalents of aldehyde was added. The resin was shaken for 16 hrs and filtered and washed with glacial acetic acid, DMA, I-PrOH, DCM. The resulted α,β-unsaturated ketone was cyclized to pyrazole by adding the 0.5M solution of 4-t-butyl phenyl hydrazine in DMSO and allowing the reaction to proceed for 16 hrs. Resin was washed with DMA, i-PrOH, DCM and dried before treating with TFA release the desired pyrazole product from the resin. The crude product was purified by preparative HPLC.

### Example 7: Solution phase synthesis

### Synthesis of N-(3-[1-(4-tert-butylphenyl)-5-pyridin-3-yl-1H-pyrazol-3-yl]propanoyl)tyrosinamide (3-A) and N-(3-[1-(4-tert-butylphenyl)-3-pyridin-3-yl-1H-pyrazol-5-yl]propanoyl)tyrosinamide (3-B)

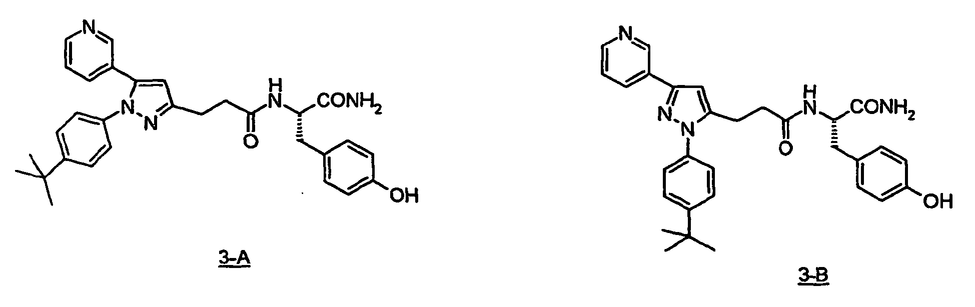

### Compound 3-A: 6-Hydroxy-1-(3-pyridinyl)-hexan-1,3-dione

A solution of y-butyrolactone (1.92 mL, 2.15 g, 25.0 mmol), 60% suspension of sodium hydride in mineral oil (300 mg, 7.50 mmol) and 3-acetylpyridine (551 µL, 606 mg, 5.00 mmol) in dimethylsulfoxyde (4 mL) and tetrahydrofuran (36 mL) was stirred overnight at 25 °C. Ethanol (4 mL) was added to destroy excess sodium hydride and the reaction mixture was adsorbed on silica gel (10 g). Flash chromatography on silica gel (75 g), eluting with DCM/MeOH/NH₄OH (96/4/1), gave 1.14 g of a yellow oil containing compound 3-A (0.62 g, 60%) .
¹H NMR (CDCl₃) : δ 9.00 (d, J =1.8 Hz, 1H), 8.65 (dd, J = 4.8, 1.5 Hz, 1H), 8.09 (dt, J = 8.1, 2.2 Hz, 1H), 7.34 (dd, J = 8.1, 4.8 Hz, 1H), 6.17 (s, 1H), 3.64 (t, J = 6.2, 2H), 2.5 (m, 2H), 1.88 (t, J = 6.2, 2H).
MS (ESI, +) : 208 (M + 1)

### Compound 3-B: 3-{1-(4-tert-butylphenyl)-5-pyridin-3-yl-1H-pyrazol-3-yl}propan-1-ol and 3-{1-(4-tert-butylphenyl)-3-pyridin-3-yl-1H-pyrazol-5-yl}propan-1-ol.

A solution of compound 3-A (370 mg, 1.79 mmol) and 4-*tert*-butylphenylhydrazine hydrochloride (359 mg, 1.79 mmol) in absolute methanol (10 mL) was stirred overnight at 25 °C. The reaction mixture was then adsorbed on silica gel (5 g) and purified by flash chromatography on silica gel (60 g), eluting with DCM/MeOH/NH₄OH (96/4/1), to afford 709 mg of a yellow oil containing the pyrazole 3-B (two regioisomers, 452 mg, 75%, ratio 24/76) and DMSO. An aliquot of this mixture was purified with preparative HPLC deltapack C18 column, using a gradient of water/acetonitrile from 95/5 to 50/50 in 60 mm, to give pure samples of the two regioisomers.

### More polar regioisomer :

¹H NMR (CDCl₃) : δ 8.80 (s, 1H), 8.76 (d, J = 5.0 Hz, 1H), 7.95 (d, J = 8.1), 7.70 (dd, J = 8.1, 5.4 Hz, 1H), 7.43 (d, J = 7.7 Hz, 2H), 7.16 (d, J = 8.1 Hz, 2H),6.60 (s, 1H), 4.48 (t, J = 6.0 Hz, 2H), 2.88 (t, J = 7.3 Hz, 2H), 2.23 (pent., J = 7.0 Hz, 2H), 1.32 (s, 9H).
MS (ESI, +) : 336 (M + 1)

### Less polar regioisomer (partially salified with trifluoroacetate) :

¹H NMR (CDCl₃): δ 9.30 (d, J = 9.2 Hz, 1H), 8.87 (d, J = 8.4 Hz, 1H), 8.73 (t, J = 5.8 Hz, 1H), 7.9 (m, 1H), 7.5 (m, 2H), 7.3 (m, 2H), 6.81 (s, 0.6H), 6.77 (s, 0.4H), 4.36 (t, J = 6.0 Hz, 1.2H), 3.71 (t, J = 6.0 Hz, 0.8H),2.83 (t,J=7.7Hz, 1.2H), 2.81 (t, J = 8.0 Hz), 2.10 (pent., J = 7.7 Hz, 1.2H), 1.92 (pent., J = 7.3 Hz, 0.8H), 1.36 (s, 9H).
MS (ESI, +) : 336 (M + 1)

### Compound 3-C: 3-{1-(4-tert-butylphenyl)-5-pyridin-3-yl-1H-pyrazol-3-yl}propanoic acid and 3-{1-(4-tert-butylphenyl)-3-pyridin-3-yl-1H-pyrazol-5-yl}propanoic acid

To a solution of pyrazoles 3-B (168 mg, 0.500 mmol) in acetone (4 mL) was added a solution of 2N H₂Cr₂O₇ (0.75 mL) at 10 °C. The mixture was stirred for 1 h, and the solution was decanted away from solid, concentrated and filtrated through a C18 column with a mixture 95/5 of water/acetonitrile. Once concentrated, the residue was engaged directly into the next step.
MS (ESI, +) : 350 (M + 1)

### Compound 3: N-(3-[1-(4-tert-butylphenyl)-5-pyridin-3-yl-1H-pyrazol-3-yl]propanoyl)tyrosinamide (3-A) and N-(3-[1-(4-tert-butylphenyl)-3-pyridin-3-yl-1H-pyrazol-5-yl]propanoyl)tyrosinamide (3-B)

To a solution of compounds 3-C (150 mg, 0.43 mmol), 1-ethyl-3-(3'-dimethylaminopropyl)carbodiimide hydrochloride (104 mg, 0.54 mmol), HOAT (73 mg, 0.54 mmol) and *N,N* diisopropylethylamine (229 µL, 174 mg, 1.35 mmol) in *N,N*-dimethylformamide (3 mL) was added *O-(tert*-butyl)tyrosinamide (128 mg, 0.54 mmol). The reaction mixture was stirred overnight at 25 °C, then diluted with a solution of 0.1N ammonia (30 mL) and extracted with ethyl acetate (3 x 25 mL). The combined organic phases are dried over magnesium sulfate, filtrated and concentrated. The crude thus obtained was dissolved in dichloromethane (5 mL) and stirred overnight at room temperature in presence of trifluoroacetic acid (2 mL). The reaction mixture was concentrated and injected on HPLC deltapack C18 column for purification using a linear gradient of 0.1% TFA water/acetonitrile from 95/5 to 60/40 in 60 min to give the two possible regioisomers whose structures were assigned by analogy with compounds 1-A and 1-B. N-(3-[1-(4-*tert*-butylphenyl)-5-pyridin-3-yl-1H-pyrazol-3-yl]propanoyl)tyrosinamide (3-A):
¹H NMR (DMSO): δ 8.54 (s, 1H), 8.45 (s, 1H), 8.03 (d, J = 8.4 Hz, 1H), 7.66 (d, J = 8.1 Hz, 1H), 7.42 (m, 3H), 7.38 (s, 1H), 7.16 (d, J = 7.3 Hz, 2H), 7.01 (d, J = 8.1 Hz, 2H), 7.00 (s, 1H), 6.61 (d, J = 7.0 Hz, 2H), 6.56 (s, 1H), 4.38 (m, 1H), 2.90 - 2.75 (m, 3H), 2.64 (dd, J = 13.9, 9.9 Hz, 1H), 2.50 (m, 2H), 1.28 (s, 9H).
MS (ESI, +): 512 (M + 1)

### N-(3-[1-(4-tert-butylphenyl)-3-pyridin-3-yl-1H-pyrazol-5-yl]propanoyl)tyrosinamide (3-B) :

¹H NMR (DMSO): δ 9.15 (s, 1H), 8.68 (s, 1H), 8.51 (d, J = 7.3 Hz, 1H), 8.13 (d, J = 8.5 Hz, 1H), 7.76 (m, 1H), 7.58 (d, J = 8.4 Hz, 2H), 7.47 (d, J = 8.4 Hz, 2H), 7.44 (s, 1H), 7.02 (s, 1H), 6.99 (d, J = 8.4 Hz, 2H), 6.93 (s, 1H), 6.58 (d, J = 8.4 Hz, 2H), 4.34 (m, 1H), 2.88 - 2.79 (m, 3 H), 2.63 (dd, J = 14.3, 10.2 Hz, 1H), 2.50 (m, 3H) 1.34 (s, 9H).
MS (ESI, +): 512 (M+1)

### Example 8: Synthesis of the trifluoroacetate salt of N-(4-{[1-(4-tert-butylphenyl)-3-pyridin-4-yl-1H-pyrazol-5-yl]amino}butanoyl)tyrosinamide 7

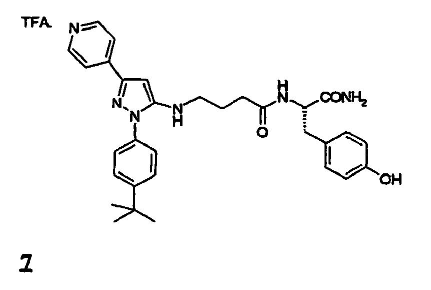

### Compound 7a : : N-[1-(4-tert-butylphenyl)-3-pyridin-4-yl-1H-pyrazol-5-yl]- trifluoroacetamide

N-[1-(4-tert-butylphenyl)-3-pyridin-4-yl-1H-pyrazol-5-yl]-amine (1.05g, 3.60mmol) obtained following literature procedure (J.ChemResearch(S), 10,1992) was dissolved in pyridine, trifluoroacetic anhydride (530 µl, 3.78 mmol) was then slowly added and the mixture was stirred under nitrogen atmosphere at room temperature for 12 h Pyridine was concentrated under reduced pressure and the resulting brown oil was dissolved in Ethyl acetate and washed twice with water and twice with brine. The organic layer was dried over magnesium sulfate, filtered and concentrated to give 1.17 g of crude material. This crude was purified by flash chromatography with DCM/MeOH/NH₄OH (95/5/1 then 85/5/1). 387 mg (yield = 28%) of pure material was isolated beside degradations products.
¹H NMR (CDCl₃) : 1.34 (s, 9H), 7.21 (s, 1H), 7.41 (d, J = 8.8 Hz, 2H), 7.56 (d, J = 8.7 Hz, 2H), 7.72 (m, 2H), 8.47 (m, 1H), 8.58 (m, 2H).
MS (ESI, +) : 389 (M+1)

### Compound 7b : 4-{[1-(4-tert-butylphenyl)-3-pyridin-4-yl-1H-pyrazol-5-yl]( trifluoroacetyl)amino}butanoic acid

A solution of compound 7a (387mg, 1.0 mmol) and ethyl-4-bromobutyrate (780mg, 4 mmol) in acetone (15 mL) was heated under reflux in presence of potassium carbonate (553mg, 4.0mmol) and potassium iodide (183mg, 1.1mmol). After 48h, the reaction was completed, acetone was removed under reduced pressure and the crude was dissolved in Ethyl acetate, washed with water and brine (2x) and finally dried over magnesium sulfate, filtrated and concentrated.
The crude (930mg) was purified by flash chromatography with DCM/MeOH/NH₄OH 90/10/1. 354 mg of a nice yellow powder were isolated (yield = 70%).
1H NMR (CDCl3) : 1.25 (t, J = 6.2 Hz, 3H). 1.35 (s, 9H), 2.12 (m, 2H), 2.35 (t, J = 6.6 Hz, 2H), 4.12 (q, J = 6.2 Hz, 2H), 4.34 (t, J = 7.3 Hz, 2H), 7.21 (s, 1H), 7.45 (d, J = 7.7 Hz, 2H), 8.03 (m, 4H), 8.31 (d, J = 6.2 Hz, 2H).
MS (ESI, +) : 503 (M+1)

### Compound 7c: Potassium salt of 4-{[1-(4-tert-butylphenyl)-3-pyridin-4-yl-1H-pyrazol-5-yl]amino}butanoic acid

A solution of ester 7b ( 234mg, 0.47mmol) in methanol/water, (1:1, 10mL) was heated under reflux for 8h in presence of potassium carbonate (195 mg, 1.41mmol). The mixture was then concentrated under reduced pressure, toluene was added and removed under reduced pressure. The crude thus obtained (398mg) was analyzed (see below) and directly engaged in the next step. 1H NMR (DMSO) : 1.34 (s, 9H), 1. 82 (t, J = 6.2 Hz, 2H), 2.01 (t, J = 6.3 Hz, 2H), 4.55 (t, J = 6.6 Hz, 2H), 5.75, s, 1H), 6.32 (s, 1H), 7.58 (s, 4H), 8.33 (d, J = 5.1 Hz, 2H), 8.98 (d, J = 5.5 Hz, 2H).
MS (ESI, +) : 379 (M+1)

### Compound 7d : O-(tert-butyl)tyrosinamide

N-Fmoc-O-*tert*-butyl-tyrosine (3.27g, 7.12mmol) was dissolved in dioxane (15 mL). Ammonium hydrogenocarbonate (732mg, 9.26mmol), di-tert-butyl-dicarbonate (2.02g, 9.26 mmol) and pyridine (0.4 mL) were added and the mixture was stirred under nitrogen atmosphere at room temperature for 12h. It was then diluted with ethyl acetate and the organic phase was washed with brine (2x), 5% sulfuric acid (1x) and brine again. It was dried over magnesium sulfate and concentrated. The crude thus obtained (3.17g) was dissolved in dichloromethane (50 mL) and diethylamine (10 mL). The mixture was stirred at room temperature for 12h, concentrated under reduced pressure and purified by flash chromatography with DCM/MeOH/NH₄OH (95/5/1 then 90/10/1) to give 1.37 g of the desired compound (yield = 81%).
¹H NMR (CDCl₃) : 1.32 (s, 9H), 2.66 (dd, J = 9.5 and 13.6 Hz, 1H), 3.20 (dd, J = 3.7 and 13.9 Hz, 1H), 3.57 (dd, J = 4.0 and 9.5 Hz, 1H), 5.40 (m, 1H), 6.93 (d, J = 7.0 Hz, 2H), 7.06 (m, 1H), 7.10 (d, J = 7.3 Hz, 2H).
MS (ESI, +) : 237 (M+1)

### Compound 7e: N-(4-{[1-(4-tert-butylphenyl)-3-pyridin-4-yl-1H-pyrazol-5-yl]amino}butanoyl) - O-(tert-butyl)tyrosinamide

Compound 7c (170 mg, 0.45mmol) was reacted with 1-ethyl-3-(3'-dimethylaminopropyl)carbodiimide hydrochloride (104 mg, 0.54mmol), HOAT (73mg, 0.54mmol) and diisopropylamine (230ul, 1.35mmol) in dimethylformamide (6 mL). O*-(tert-*butyl)tyrosinamide 7d (118mg, 0.50mmol) was then added and the reaction mixture was stirred at room temperature under nitrogen atmosphere. After completion of the reaction (48h), it was diluted with ethyl acetate and water. The two layers were separated and it appeared that the product was essentially in the aqueous phase. This latter was concentrated and injected on HPLC deltapack C18 column for purification using a gradient of water/acetonitrile from 95/5 to 40/60 in 60min. 96 mg of pure compound was isolated after lyophilisation (yield = 36%).
¹H NMR (CDCl₃) : 1.25 (s, 9H), 1.35 (s, 9H), 2.20-2.43 (m, 4H), 2.90 (t, J = 9.9 Hz, 1H), 3.18 (brd, J =10.6 Hz, 1H), 4.27-4.52 (m, 7H), 6.20 (s, 1H), 6.84 (d, J = 8.0 Hz, 2H), 7.15 (d, J = 8.0 Hz, 2H), 7.50 (m, 4H), 8.18 (m, 2H), 8.29 (m, 1H), 8.53 (m, 1H).
MS (ESI, +): 597 (M+1)

### Compound 7: Compound 7e (89 mg, 0.15 mmol) was dissolved in dichloromethane and trifluoroacetic acid was added The mixture was stirred overnight and concentrated to give 103 mg of a brown solid (quantitative yield).

1H NMR (DMSO) : 0.82 (m, 2H), 1.33 (s, 9H), 2.13 (m, 2H), 2.60 (m, 1H), 2.86 (m, 1H), 4.19-4.38 (m, 3H), 5.81 (m, 1H), 6.32 (s, 1H), 6.63 (d, J = 6.6 Hz, 2H), 7.00 (d, J = 6.6 Hz, 2H), 7.01 (s, 1H), 7.45 (s, 1H), 7.58 (brs, 4H), 7.69 (m, 2H), 8.02 (d, J = 8.4 Hz, 1H), 8.34 (d, J = 5.1 Hz, 2H), 8.85 (d, J = 5.1 Hz, 2H), 9.16 (s, 1H).

The invention has been described in detail with reference to preferred embodiments thereof However, it will be appreciated that those skilled in the art, upon consideration of this disclosure, may make modifications and improvements within the spirit and scope of the invention.

The invention has been described in detail with reference to preferred embodiments thereof. However, it will be appreciated that those skilled in the art, upon consideration of this disclosure, may make modifications and improvements within the spirit and scope of the invention.

## Claims

1. Use of a pyrazole compound of the following Formula IB: 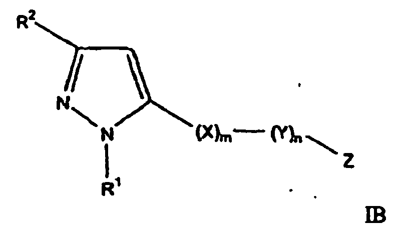 wherein
R¹ and R² are each independently optionally substituted alkyl; optionally substituted alkenyl; optionally substituted alkynyl; optionally substituted carbocyclic aryl; optionally substituted aralkyl; optionally substituted heteroaromatic or heteroalicyclic group; or optionally substituted heteroaralkyl or heteroalicyclicalkyl group;
X is optionally substituted alkylene; optionally substituted alkenylene; optionally substituted alkynylene; optionally substituted heteroalkylene; optionally substituted heteroalkenylene; optionally substituted heteroalkynylene; optionally substituted alicyclic; optionally substituted carbocyclic aryl; optionally substituted aralkyl; optionally substituted heteroaromatic; optionally substituted heteroalicyclic group; optionally substituted heteroaralkyl; or optionally substituted heteroalicyclicalkyl group;
Y is optionally substituted amino; optionally substituted methylene; carbonyl; or sulfonyl;
Z is an optionally substituted alkylamine; an amino acid; or a glycine; m and n are each independently 0 or 1; and pharmaceutically acceptable salts thereof; for the preparation of a pharmaceutical composition for the treatment of a condition, disorder or disease involving testosterone deficiency.

2. The use according to claim 1 wherein the pyrazole compound is of the following Formula IC: 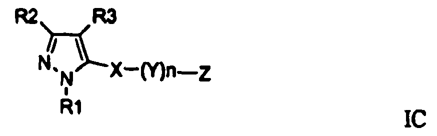 Wherein R¹ is C₁-C₆ alkyl, aryl alkyl, aryl, alkyl aryl, heteroaryl; R² is aryl or heteroaryl; R³ is hydrogen; X is aryl, heteroaryl, alkyl, heteroalkyl, amino alkyl, aryl heteroalkyl, amino alkyl phenyl, heterocycloalkyl alkyl ; Y is carbonyl; Z is a substituted alkylamine, - NH-CH(C(O)-NH₂)₀₋₁-(CH₂)₀₋₂-Phe-OH and -NH-CH(C(O)-NH₂)₀₋₁-(CH2)₀₋₂-Phe-O-alkyl, -NH-CH(C(O)-NH₂)₀₋₁-(CH2)₀₋₂-Phe- NH₂, -NH-CH(C(O)-NH₂)₀₋₁-(CH2)₀₋₂-Phe-NH-C(O)CH₃, -NMe-CH(C(O)-NCH₃)-CH2-Phe-OH, an amino acid derivative amino attached by the α-amino group to the rest of the molecule; n is 1.

3. The use according to claim 2 wherein R¹ is phenyl, -Phe-butyl, -Phe-propyl, -Phe-(CH₂)₂-CH, -benzyl-butyl, pyridinyl, propyl, hexyl; R² is pyridinyl isoquinolinyl, quinolinyl, furyl, dimethyl amino phenyl, pyrazinyl; X is phenylene, thienylene, ethylene, propylene, pentylene, -CH₂-NH-, -CH₂-NH-CH₂, - CH₂-NH- CH₂- CH₂, - CH2-NH-Phe, - CH₂-piperidin-, -NH-(CH₂)₃-; Z is tyrosinamide, threonamide, serinamide, hydroxymethyl phenylalanamide (these amino acid derivatives being attached by the α-amino group to the rest of the molecule), -NMe-CH(C(O)-NCH₃)-CH₂-Phe-OH, -NH-CH(C(O)NH₂)-(CH₂)₂-Phe-OH, -NH-CH(PheOH)-C(O)-NH₂,-NH-(CH₂)₂-Phe-OH and-NH-CH(C(O)NH₂)-(CH₂)₂-Phe-O-t-bu; n is 1.

4. The use according to claim 3 wherein R¹ is 4-t-butyl phenyl; R² is pyridinyl; R³ is hydrogen; X is propylene; Y is carbonyl; Z is tyrosinamide attached by the α-amino group to the rest of the molecule; n is 1.

5. The use according to claim 1 wherein the pyrazole compound is of the following Formula ID: 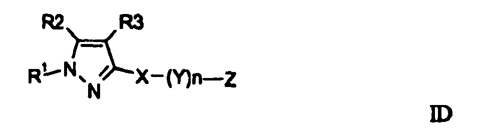 Wherein R¹ is C₁-C₆ alkyl, aryl alkyl, aryl, alkyl aryl, heteroaryl; R² is aryl or heteroaryl; R³ is hydrogen; X is aryl, heteroaryl, alkyl, heteroalkyl, amino alkyl, aryl alkyl, aryl heteroalkyl, amino alkyl phenyl, heterocycloalkyl alkyl; Y is carbonyl; Z is a substituted alkylamine, -NH-CH(C(O)-NH₂)₀₋₁-(CH2)₀₋₂Phe-OH and -NH-CH(C(O)-NH₂)₀₋₁-(CH2)₀₋₂-Phe-O-alkyl, -NH-CH(C(O)NH₂)₀₋₁-(CH2)₀₋₂-Phe- NH₂, -NH-CH(C(O)-NH₂)₀₋₁-(CH₂)₀₋₂-Phe-NH-C(O)CH₃, -NMe-CH(C(O)-NCH₃)-CH2-Phe-OH, an amino acid derivative amino attached by the α-amino group to the rest of the molecule; n is 1;

6. The use according to claim 5 wherein R¹ is phenyl, -Phe-butyl, -Phe-propyl, -Phe-(CH₂)₂-CH, -benzyl-butyl, pyridinyl, propyl, hexyl; R² is pyridinyl, isoquinolinyl, quinolinyl, furyl, dimethyl amino phenyl, pyrazinyl; X is phenylene, thienylene, ethylene, propylene, pentylene, -CH₂-NH-, -CH₂-NH-CH₂, - CH₂-NH- CH₂- CH₂, - CH2-NH-Phe, - CH₂-piperidin-, -NH-(CH₂)₃-; Z is tyrosinamide, threonamide, serinamide, hydroxymethyl phenylalanamide (these amino acid derivatives being attached by the α-amino group to the rest of the molecule), -NMe-CH(C(O)-NCH₃)-CH₂-Phe-OH, -NH-CH(C(O)NH₂)-(CH₂)₂-Phe-OH, -NH-CH(PheOH)-C(O)-NH₂,-NH-(CH₂)₂-Phe-OH and-NH-CH(C(O)NH₂)-(CH₂)₂-Phe-O-t-bu; n is 1.

7. The use according to claim 6 wherein R¹ is t-butyl phenyl; R² is pyridinyl; R³ is hydrogen; X is propylene; Y is carbonyl; Z is tyrosinamide attached by the α-amino group to the rest of the molecule; n is 1.

8. The use according to any one of claims 1 to 7 wherein R² is other than hydrogen.

9. The use according to any one of claims 1 to 8 wherein R¹ is optionally substituted alkyl or optionally substituted carbocyclic aryl or optionally substituted aryalkyl.

10. The use according to any one of claims 1 to 8 wherein R² is optionally substituted carbocyclic aryl or optionally substituted heteroaromatic and heteroalicyclic.

11. The use according to any one of claims 1 to 10 wherein X is an optionally substituted alkylene.

12. The use according to any one of claims 1 to 10 wherein X is an optionally substituted carbocyclic aryl or an optionally substituted heteroaromatic group.

13. The use according to any one of claims 1 to 10 wherein X is an optionally substituted carbocyclic aryl or an optionally substituted heteroalkylene group.

14. The use according to any one of claims 1 to 11 wherein X is -CH₂CH₂CH₂CH₂- or-CH₂CH₂CH₂CH₂CH₂-.

15. The use according to any one of claims 1 to 10 wherein X is optionally substituted phenyl, optionally substituted phenoxy; or optionally substituted phenylamino.

16. The use according to any one of claims 1 to 15 wherein Z is a phenolic moiety.

17. The use according to any one of claims 1 to 15 wherein Z is a tyrosine group.

18. The use according to any one of claims 1 to 17 wherein the compound is selected from the group consisting of:
N-{5-[1-(4-tert-butylphenyl)-3-pyridin-2-yl-1H-pyrazol-5-yl]pentanoyl}tyrosinamide;
N-{5-[1-(4-tert-butylphenyl)-3-pyridin-3-yl-1H-pyrazol-5-yl]pentanoyl}tyrosinamide;
N-{5-[1-(4-tert.butylphenyl)-5-pyridin-3-yl-1H-pyrazol-3-yl]pentanoyl}tyrosinamide;
N-{5-[1-(4-tert-butylphenyl)-3-pyridin-4-yl-1H-pyrazol-5-yl]pentanoyl}tyrosinamide;
N-{5-[1-(4-tert-butylphenyl)-5-pyridin-4-yl-1H-pyrazol-3-yl]pentanoyl}tyrosinamide;
N-{5-[1-(4-tert-butylphenyl)-3-pyridin-3-yl-1H-pyrazol-5-yl]pentanoyl}-N,Ndimethyl tyrosinamide;
N-(3-[1-(4-tert-butylphenyl)-5-pyridin-3-yl-1H-pyrazol-3-yl]propanoyl)tyrosinamide;
N-(3-[1-(4-tert-butylphenyl)-3-pyridin-3-yl-1H-pyrazol-5-yl]propanoyl)tyrosinamide;
N-[4-(1-butyl-3-isoquinolin-3-yl-1H-pyrazol-5-yl)benzoyl]tyrosinamide;
N-{5-[1-(4-isopropylphenyl)-3-pyridin-3-yl-1H-pyrazol-5-yl]pentanoyl}tyrosinamide;
N- {6-[1-(4-isopropylphenyl)-3-pyridin-2-yl-1H-pyrazol-5-yl]hexanoyl}tyrosinamide
N-{6-[1-(4-tert-butylphenyl)-3-pyridin-2-yl-1H-pyrazol-5-yl]hexanoyl}-3-hydroxy phenylalaninamide;
N-[1-(aminocarbonyl)-3-(4-hydroxyphenyl)propyl]-5-[1-(4-tert-butylphenyl)-3-pyridin-2-yl-1H-pyrazol-5-yl]pentanamide;
N-[5-(1-butyl-3-isoquinolin-3-yl-1H-pyrazol-5-yl)pentanoyl]tyrosinamide;
N-{6-[1-(4-tert-butylphenyl)-3-pyridin-2-yl-1H-pyrazol-5-yl]hexanoyl}serinamide;
N-{6-[1-(4-iso-propylphenyl)-3-pyridin-2-yl-1H-pyrazol-5-yl]hexanoyl}serinamide;
N-{6-[1-(4-iso-propylphenyl)-3-pyridin-2-yl-1H-pyrazol-5-yl]hexanoyl}threonamide;
N-{5-[1-(4-isopropytphenyl)-3-pyridin-2-yl-1H-pyrazol-5-yl]pentanoyl}tyrosinamide;
6-[1-(4-tert-butylphenyl)-3-pyridin-2-yl-1H-pyrazol-5-yl]-N-[2-(4-hydroxyphenyl) ethyl]hexanamide;
N- {6-[1-(4-tert-butytphenyl)-3-pyridin-2-yl-1 H-pyrazol-5-yl]hexanoyl} tyrosinamide;
N-{5-[1-(4-tert-butylphenyl)3-isoquinolin-3-yl-1H-pyrazol-5-yl]pentanoyl} tyrosinamide;
N-{6-[1-(4-tert-butylphenyl)-3-isoquinolin-3-yl-1H-pyrazol-5-yl]hexanoyl} tyrosinamide;
N-{6-[1-(4-tert-butylphenyl)-3-pyridin-2-yl-1H-pyrazol-5-yl]hexanoyl}-N-methyl tyrosinamide;
N-{6-[1-(4-tert-butylphenyl)-3-pyridin-2-yl-1H-pyrazol-5-yl]hexanoyl}-4-(hydroxymethyl)phenylalaninamide;
4-amino-N-{6-[1-(4-tert-butylphenyl)-3-pyridin-2-yl-1H-pyrazol-5-yl]hexanoyl} phenylalaninamide;
4-(acetylamino)-N-{6-[1-(4-tert-butylphenyl)-3-pyridin-2-yl-1H-pyrazol-5-yl] hexanoyl}phenylalaninamide;
4-(aminocarbonyl)-N-{6-[1-(4-tert-butylphenyl)-3-pyridin-2-yl-1H-pyrazol-5-yl] hexanoyl}phenylaninamide ;
N-butyl-N-{6-[1-(4-tert-butylphenyl)-pyridin-2-yl-1H-pyrazol-5-yl] hexanoyl}tyrosinamide;
N-{6-[1-(4-tert-butylphenyl)-3-pyridin-2-yl-1H-pyrazol-5-yl]hexanoyl}threonamide;
N-[2-amino-1-(4-hydroxyphenyl)-2-oxoethyl]-6-[1-(4-tert-butylphenyl)-3-pyridin-2-yl-1H-pyrazol-5-yl]hexanamide;
N-({5-[1-(4-isopropylphenyl)-3-pyridin-2-yl-1H-pyrazol-5-yl]thien-2-yl}carbonyl) tyrosinamide;
N-[2-amino-1-(4-hydroxyphenyl)-2-oxoethyl]-4-[1-(4-tert-butylphenyl)-3-pyridin-2-yl-1H-pyrazol-5-yl]benzamide;
4-[1-(4-tert-butylphenyl)-3-pyridin-2-yl-1H-pyrazol-5-yl]-N-[2-(4-hydroxyphenyl) ethyl]benzamide;
N-{3-[1-(4-tert-butylbenzyl)-3-isoquinolin-3-yl-1H-pyrazol-5-yl]benzoyl}tyrosinamide;
N-{5-[1-(4-tert-butylphenyl)-3-pyridin-4-yl-1H-pyrazol-5-yl]pentanoyl}tyrosinamide;
N-{3-[1-(4-tert-butylbenzyl)-3-pyridin-3-yl-1H-pyrazol-5-yl]benzol}tyrosinamide;
N-{3-[1-(4-tert-butylphenyl)-3-isoquinolin-3-yl-1H-pyrazol-5-yl]benzoyl}tyrosinamide;
N-[4-(1-butyl-3-isoquinolin-3-yl-1H-pyrazol-5-yl)benzoyl]tyrosinamide;
N-{3-[1-(4-tert-butybenzyl)-3-quinolin-3-yl-1H-pyrazol-5-yl]benzoyl}tyrosinamide;
N-{3-[3-isoquinolm-3-yl-1-(4-propylphenyl)-1H-pyrazol-5-yl]benzoyl}tyrosinamide;
N-{5-[1-(4-tert-butylphenyl)-3-pyridin-3-yl-1H-pyrazol-5-l]pentanoyl} tyrosinamide;
N-{3-[1-(4-tert-butylphenyl)-3-pyridin-3-yl-1H-pyrazol-5-yl]benzoyl}tyrosinamide;
N-{4-[1-(4-tert-butylphenyl)-3-pyridin-3-yl-1H-pyrazol-5-yl]benzoyl}tyrosinamide;
N-{5-[1-(4-tert-butylbenzyl)-3-pyridin-4-yl-1H-pyrazol-5-yl]pentanoyl}tyrosinamide;
N-{3-[1-(4-tert-butylbenzyl)-3-pyridin-4-yl-1H-pyrazol-5-yl]benzoyl}tyrosinamide;
N-{5-[1-(4-tert-butylbenzyl)-3-pyridin-3-yl-1H-pyrazol-5-l]pentanoyl}tyrosinamide;
N-{5-[1-(4-tert-butylphenyl)-3-pyridin-4-yl-1H-pyrazol-5-yl]pentanoyl}tyrosinamide;
N-[3-(1-butyl-3-isoquinolin-3-yl-1H-pyrazol-5-yl)benzoyl]tyrosinamide;
N-(4-{[1-(4-tert-butylphenyl)-3-pyridin-4-yl-1H-pyrazol-5-yl]amino}butanoyl) tyrosinamide;
N-({3-[1-(4-tert-butylphenyl)-3-pyridin-4-yl-1H-pyrazol-5-yl]propoxy}acetyl) tyrosinamide;
4-[2-({3-[1-(4-tert-butylphenyl)-3-pyridin-4-yl-1H-pyrazol-5-yl]propyl}amino)ethyl] phenol;
N-{3-[1-(4-tert-butylpbenyl)-3-pyridin-4-yl-1H-pyrazol-5-yl]propyl}tyrosinamide;
N-acetyl-N-{3-[1-(4-tert-butylphenyl)-3-pyridin-4-yl-1H-pyrazol-5-yl]propyl} tyrosinamide;
3-[(4-{[1-(4-tert-butylphenyl)-3-pyridin-4-yl-1H-pyrazol-5-yl]amino}butanoyl)amino]-4-(4-hydroxyphenyl)butanamide;
N-(4-{[1-(4-tert-butylphenyl)-3-(1-methyl-1,2,3,6-tetrahydropyridin-4-yl)-1H-pyrazol-5-yl]amino}butanoyl)tyrosinamide;
N-(3- {[1-(4-tert-butylphenyl)-3-pyridin-4-yl-1H-pyrazol-5-yl]amino}propyl) tyrosinamide;
N-acetyl-N-(3-{[1-(4-tert-butylphenyl)-3-pyridin-4-yl-1H-pyrazol-5-yl]amino}propyl) tyrosinamide;
N-(4-{[1-(4-tert-butylphenyl)-3-(1-oxidopyridin-4-yl)-1H-pyrazol-5-yl]amino} butanoyl)tyrosinamide;
4-{[1-(4-tert-butylphenyl)-3-pyridin-4-yl-1H-pyrazol-5-yl]amino}-N-[2-(4-hydroxy phenyl)ethyl]butanamide;
4- {2-[(4-{[1-(4-tert-butylphenyl)-3-pyridin-4-yl-1H-pyrazol-5-yl]amino}butyl)amino] ethyl}phenol;
N-(4-{[1-(4-tert-butylphenyl)-3-pyridin-4-yl-1H-pyrazol-5-yl]amino}butanoyl)-N-(2-hydroxyethyl)tyrosinamide;
N-(4-{[1-(4-tert-butylphenyl)-3-pyridin-4-yl-1H-pyrazol-5-yl]amino}butanoyl)-N-[2-(1-methylpyrrolidin-2-yl)ethyl]tyrosinamide;
N-(4-{[1-(4-tert-butylphenyl)-3-pyridin-3-yl-1H-pyrazol-5-yl]amino}butanoyl) tyrosinamide;
N-(4-{[1-(4-tert-butylphenyl)-3-(1-methyl-1,2,5,6-tetrahydropyridin-3-yl)-1H-pyrazol-5-yl]amino}butanoyl)tyrosinamide;
(3R)-3-[(4-{[1-(4-tet-butytphenyl)-3-pyhdin-3-yl-1H-pyrazol-5-yl]amino}butanoyl) amino]-4-(4-hydroxyphenyl)butanamide;
N-(3-{[1-(4-tert-butylphenyl)-3-pyridin-3-yl-1H-pyrazol-5-yl]amino}propyl) tyrosinamide;
N-acetyl-N-(3-{[1-(4-tert-butylphenyl)-3-pyridin-3-yl-1H-pyrazol-5-yl]amino} propyl) tyrosinamide ;
N-(4-{[1-(4-tert-butylphenyl)-3-(1-oxidopyridin-3-yl)-1H-pyrazol-5-yl]amino}butanoyl) tyrosinamide;
4- {[1-(4-tert-butylphenyl)-3-pyridin-3-yl-1H-pyrazol-5-yl]amino}-N-[2-(4-hydroxy phenyl)ethyl] butanamide;
4-{2-[(4-{[1-(4-tert-butylphenyl)-3-pyridin-3-yl-1H-pyrazol-5-yl]amino}butyl)amino] ethyl}phenol;
N-(4-{[1-(4-tert-butylphenyl)-3-pyridin-3-yl-1H-pyrazol-5-yl]amino}butanoyl)-N-(2-hydroxyethyl)tyrosinamide;
N-(4-{[1-(4-tert-butylphenyl)-3-pyridin-3-yl-1H-pyrazol-5-yl]amino}butanoyl)-N-[2-(1-methylpyrrolidin-2-yl)ethyl]tyrosinamide;
N-(4-{(1-(4-tert-butylphenyl)-3-pyrldin-3-yl-1H-pyrazol-5-yl)oxy}butanoyl) tyrosinamide; or an optical isomer, racemate or tautomer of any one thereof or a pharmaceutically acceptable salt of any one thereof.

19. The use according to any one of claims 1 to 17 wherein the compound is 5-[2-(4-tert-Butyl-phenyl)-5-pyridin-3-yl-2H-pyrazol-3-yl]-pentanoic acid [1-carbamoyl-2-(4-hydroxy-phenyl)-ethyl]-amide or 5-[2-(4-tert-Butyl-phenyl)-5-pyridin-4-yl-2H-pyrazol-3-yl]-pentanoic acid [1-carbamoyl-2-(4-hydroxy-phenyl)-ethyl]-amide.

20. The use according to any one of claims 1 to 19 wherein the disorder treated is a spermatogenesis disorder.

21. The use according to any one of claims 1 to 19 wherein the disorder or disease is selected from AIDS Wasting, muscle dystrophy or cachexia.

## Patentansprüche

1. Verwendung einer Pyrazolverbindung der folgenden Formel IB: 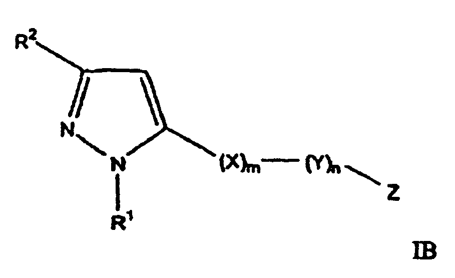 worin
R¹ und R² jeweils unabhängig voneinander gegebenenfalls substituiertes Alkyl; gegebenenfalls substituiertes Alkenyl; gegebenenfalls substituiertes Alkinyl; gegebenenfalls substituiertes carbocyclisches Aryl; gegebenenfalls substituiertes Aralkyl; eine gegebenenfalls substituierte heteroaromatische oder heteralicyclische Gruppe; oder eine gegebenenfalls substituierte Heteroaralkylgruppe oder heteroalicyclische Alkylgruppe darstellen;
X gegebenenfalls substituiertes Alkylen; gegebenenfalls substituiertes Alkenylen; gegebenenfalls substituiertes Alkinylen, gegebenenfalls substituiertes Heteroalkylen; gegebenenfalls substituiertes Heteroalkenylen; gegebenenfalls substituiertes Heteroalkinylen; gegebenenfalls substituierter Alicyclus; gegebenenfalls substituiertes carbocyclisches Aryl; gegebenenfalls substituiertes Aralkyl; gegebenenfalls substituierter Heteroaromat; eine gegebenenfalls substituierte heteroalicyclische Gruppe; gegebenenfalls substituiertes Heteroaralkyl; oder eine gegebenenfalls substituierte heteroalicyclische Alkylgruppe ist;
Y gegebenenfalls substituiertes Amino; gegebenenfalls substituiertes Methylen; Carbonyl; oder Sulfonyl ist;
Z ein gegebenenfalls substituiertes Alkylamin; eine Aminosäure oder ein Glycin ist; m und n jeweils unabhängig voneinander 0 oder 1 sind; und pharmazeutisch verträgliche Salze davon; für die Herstellung einer pharmazeutischen Zusammensetzung für die Behandlung eines Zustands, einer Störung oder einer Krankheit, die eine Testosteron-Defizienz einschließen.

2. Verwendung nach Anspruch 1, wobei die Pyrazolverbindung die folgende Formel IC aufweist: 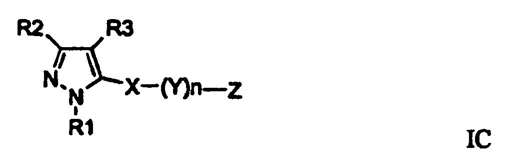 worin R¹ C₁-C₆-Alkyl, Arylalkyl, Aryl, Alkylaryl, Heteroaryl ist; R² Aryl oder Heteroaryl ist; R³ Wasserstoff ist; X Aryl, Heteroaryl, Alkyl, Heteroalkyl, Aminoalkyl, Aryl-heteroalkyl, Aminoalkylphenyl, Heterocycloalkylalkyl ist; Y Carbonyl ist; Z ein substituiertes Alkylamin, -NH-CH(C(O)-NH₂)₀₋₁-(CH₂)₀₋₂-Phe-OH und -NH-CH(C(O)-NH₂)₀₋₁-(CH₂)₀₋₂-Phe-O-alkyl, -NH-CH(C(O)-NH₂)₀₋₁-(CH₂)₀₋₂-Phe-NH₂, - NH-CH(C(O)-NH₂)₀₋₁-(CH₂)₀₋₂-Phe-NH-C(O)CH₃, -NMe-CH(C(O)-NCH₃)-CH₂-Phe-OH, ein Aminosäurederivatamino, verbunden mit dem Rest des Moleküls über die α-Aminogruppe ist; n 1 ist.

3. Verwendung nach Anspruch 2, wobei R¹ Phenyl, -Phe-butyl, -Phe-propyl, -Phe-(CH₂)₂-CH, -Benzyl-butyl, Pyridinyl, Propyl, Hexyl ist; R² Pyridinyl, Isochinolinyl, Chinolinyl, Furyl, Dimethylaminophenyl, Pyrazinyl ist; X Phenylen, Thienylen, Ethylen, Propylen, Pentylen, -CH₂-NH-, -CH₂-NH-CH₂, -CH₂-NH-CH₂-CH₂, -CH₂-NH-Phe, -CH₂-piperidin-, -NH-(CH₂)₃ ist; Z Tyrosinamid, Threonamid, Serinamid, Hydroxymethylphenylalanamid (diese Aminosäurederivate sind über die α-Aminogruppe mit dem Rest des Moleküls verbunden), -NMe-CH(C(O)-NCH₃)-CH₂-Phe-OH, -NH-CH(C(O)NH₂)-(CH₂)₂-Phe-OH, -NH-CH(PheOH)-C(O)-NH₂, -NH-(CH₂)₂-Phe-OH und -NH-CH(C(O)NH₂)-CH₂)₂-Phe-O-t-bu ist; n 1 ist.

4. Verwendung nach Anspruch 3, wobei R¹ 4-t-Butylphenyl ist; R² Pyridinyl ist; R³ Wasserstoff ist; X Propylen ist; Y Carbonyl ist; Z Tyrosinamid ist, verbunden mit dem Rest des Moleküls über die α-Aminogruppe; n 1 ist.

5. Verwendung nach Anspruch 1, wobei die Pyrazolverbindung die folgende Formel ID aufweist: 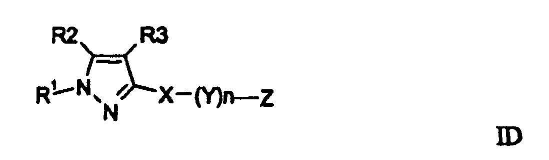 worin R¹ C₁-C₆-Alkyl, Arylalkyl, Aryl, Alkylaryl, Heteroaryl ist; R² Aryl oder Heteroaryl ist; R³ Wasserstoff ist; X Aryl, Heteroaryl, Alkyl, Heteroalkyl, Aminoalkyl, Arylalkyl, Arylheteroalkyl, Aminoalkylphenyl, Heterocycloalkylalkyl ist; Y Carbonyl ist; Z ein substituiertes Alkylamin ist, -NH-CH(C(O)-NH₂)₀₋₁-(CH₂)₀₋₂-Phe-OH und - NH-CH(C(O)-NH2)₀₋₂-(CH2)₀₋₂-Phe-O-alkyl, -NH-CH(C(O)-NH₂)₀₋₁-(CH2)₀₋₂-Phe-NH₂, -NH-CH(C(O)-NH₂)₀₋₁-(CH₂)₀₋₂-Phe-NH-C(O)CH₃, -NMe-CH(C(O)-NCH₃)-CH₂-Phe-OH, ein Aminosäurederivatamino, verbunden mit dem Rest des Moleküls über die α-Aminogruppe ist; n 1 ist.

6. Verwendung nach Anspruch 5, wobei R¹ Phenyl, -Phe-butyl, -Phe-propyl, -Phe-(CH₂)₂-CH, -Benzyl-butyl, Pyridinyl, Propyl, Hexyl ist; R² Pyridinyl, Isochinolinyl, Chinolinyl, Furyl, Dimethylaminophenyl, Pyrazinyl ist; X Phenylen, Thienylen, Ethylen, Propylen, Pentylen, -CH₂-NH-, -CH₂-NH-CH₂, -CH₂-NH-CH₂-CH₂, -CH₂-NH-Phe, -CH₂-piperidin-, -NH-(CH₂)₃ ist; Z Tyrosinamid, Threonamid, Serinamid, Hydroxymethylphenylalanamid (diese Aminosäurederivate sind mit dem Rest des Moleküls über die α-Aminogruppe verbunden), -NMe-CH(C(O)-NCH₃)-CH₂-Phe-OH, -NH-CH(C(O)NH₂)-(CH₂)₂-Phe-OH, -NH-CH(PheOH)-C(O)-NH₂, -NH-(CH₂)₂-Phe-OH und -NH-CH(C(O)NH₂)-(CH₂)₂-Phe-O-t-bu ist; n 1 ist.

7. Verwendung nach Anspruch 6, wobei R¹ t-Butylphenyl ist; R² Pyridinyl ist; R³ Wasserstoff ist; X Propylen ist; Y Carbonyl ist; Z Tyrosinamid ist, das mit dem Rest des Moleküls über die α-Aminogruppe verbunden ist; n 1 ist.

8. Verwendung nach einem der Ansprüche 1 bis 7, wobei R² sich von Wasserstoff unterscheidet.

9. Verwendung nach einem der Ansprüche 1 bis 8, wobei R¹ gegebenenfalls substituiertes Alkyl oder gegebenenfalls substituiertes carbocyclisches Aryl oder gegebenenfalls substituiertes Arylalkyl ist.

10. Verwendung nach einem der Ansprüche 1 bis 8, wobei R² gegebenenfalls substituiertes carbocyclisches Aryl oder gegebenenfalls substituierter Heteroaromat und Heteroalicyclus ist.

11. Verwendung nach einem der Ansprüche 1 bis 10, wobei X ein gegebenenfalls substituiertes Alkylen ist.

12. Verwendung nach einem der Ansprüche 1 bis 10, wobei X ein gegebenenfalls substituiertes carbocyclisches Aryl oder eine gegebenenfalls substituierte heteroaromatische Gruppe ist.

13. Verwendung nach einem der Ansprüche 1 bis 10, wobei X ein gegebenenfalls substituiertes carbocyclisches Aryl oder eine gegebenenfalls substituierte Heteroalkylengruppe ist.

14. Verwendung nach einem der Ansprüche 1 bis 11, wobei X -CH₂CH₂CH₂CH₂- oder -CH₂CH₂CH₂CH₂CH₂- ist.

15. Verwendung nach einem der Ansprüche 1 bis 10, wobei X gegebenenfalls substituiertes Phenyl, gegebenenfalls substituiertes Phenoxy; oder gegebenenfalls substituiertes Phenylamino ist.

16. Verwendung nach einem der Ansprüche 1 bis 15, wobei Z ein Phenolrest ist.

17. Verwendung nach einem der Ansprüche 1 bis 15, wobei Z eine Tyrosingruppe ist.

18. Verwendung nach einem der Ansprüche 1 bis 17, wobei die Verbindung ausgewählt ist aus der Gruppe, die besteht aus:
N-{5-[1-(4-tert-Butylphenyl)-3-pyridin-2-yl-1H-pyrazol-5-yl]pentanoyl}tyrosinamid;
N-{5-[1-(4-tert-Butylphenyl)-3-pyridin-3-yl-1H-pyrazol-5-yl]pentanoyl}tyrosinamid;
N-{5-[1-(4-tert-Butylphenyl)-5-pyridin-3-yl-1H-pyrazol-3-yl]pentanoyl}tyrosinamid;
N-{5-[1-(4-tert-Butylphenyl)-3-pyridin-4-yl-1H-pyrazol-5-yl]pentanoyl}tyrosinamid;
N-{5-[1-(4-tert-Butylphenyl)-5-pyridin-4-yl-1H-pyrazol-3-yl]pentanoyl}tyrosinamid;
N-{5-[1-(4-tert-Butylphenyl)-3-pyridin-3-yl-1H-pyrazol-5-yl]pentanoyl}-N,N-dimethyltyrosinamid;
N-(3-[1-(4-tert-Butylphenyl)-5-pyridin-3-yl-1H-pyrazol-3-yl]propanoyl)tyrosinamid;
N-(3-[1-(4-tert-Butylphenyl)-3-pyridin-3-yl-1H-pyrazol-5-yl]propanoyl)tyrosinamid;
N-[4-(1-Butyl-3-isochinolin-3-yl-1H-pyrazol-5-yl)benzoyl]tyrosinamid;
N-{5-[1-(4-lsopropylphenyl)-3-pyridin-3-yl-1H-pyrazol-5-yl]pentanoyl}tyrosinamid;
N-{6-[1-(4-lsopropylphenyl)-3-pyridin-2-yl-1H-pyrazol-5-yl]hexanoyl}tyrosinamid;
N-{6-[1-(4-tert-Butylphenyl)-3-pyridin-2-yl-1H-pyrazol-5-yl]hexanoyl}-3-hydroxyphenylalaninamid;
N-[1-(Aminocarbonyl)-3-(4-hydroxyphenyl)propyl]-5-[1-(4-tert-Butylphenyl)-3-pyridin-2-yl-1 H-pyrazol-5-yl]pentanamid;
N-[5-(1-Butyl-3-isochinolin-3-yl-1H-pyrazol-5-yl)pentanoyl]tyrosinamid;
N-{6-[1-(4-tert-Butylphenyl)-3-pyridin-2-yl-1H-pyrazol-5-yl]hexanoyl}serinamid;
N-{6-[1-(4-lso-propylphenyl)-3-pyridin-2-yl-1H-pyrazol-5-yl]hexanoyl}serinamid;
N-{6-[1-(4-lso-propylphenyl)-3-pyridin-2-yl-1H-pyrazol-5-yl]hexanoyl}threonamid;
N-{5-[1-(4-Isopropylphenyl)-3-pyridin-2-yl-1H-pyrazol-5-yl]pentanoyl}tyrosinamid;
6-[1-(4-tert-Butylphenyl)-3-pyridin-2-yl-1H-pyrazol-5-yl]-N-[2-(4-hydroxyphenyl)-ethyl]hexanamid;
N-{6-[1-(4-tert-Butylphenyl)-3-pyridin-2-yl-1H-pyrazol-5-yl]hexanoyl}tyrosinamid;
N-{5-[1-(4-tert-Butylphenyl)-3-isochinolin-3-yl-1H-pyrazol-5-yl]pentanoyl}tyrosinamid;
N-{6-[1-(4-tert-Butylphenyl)-3-isochinolin-3-yl-1H-pyrazol-5-yl]hexanoyl}tyrosinamid;
N-{6-[1-(4-tert-Butylphenyl)-3-pyridin-2-yl-1H-pyrazol-5-yl]hexanoyl}-N-methyltyrosinamid;
N-{6-[1-(4-tert-Butylphenyl)-3-pyridin-2-yl-1H-pyrazol-5-yl]hexanoyl}-4-(hydroxymethyl)phenylalaninamid;
4-Amino-N-{6-[1-(4-tert-butylphenyl)-3-pyridin-2-yl-1H-pyrazol-5-yl]hexanoyl}phenylalaninamid;
4-(Acetylamino)-N-{6-[1-(4-tert-butylphenyl)-3-pyridin-2-yl-1H-pyrazol-5-yl]hexanoyl}phenylalaninamid;
4-(Aminocarbonyl)-N-{6-[1-(4-tert-butylphenyl)-3-pyridin-2-yl-1H-pyrazol-5-yl]hexanoyl}phenylalaninamid;
N-Butyl-N-{6-[1-(4-tert-butylphenyl)-3-pyridin-2-yl-1H-pyrazol-5-yl]hexanoyl}tyrosinamid;
N-{6-[1-(4-tert-Butylphenyl)-3-pyridin-2-yl-1H-pyrazol-5-yl]hexanoyl}threonamid;
N-[2-Amino-1-(4-hydroxyphenyl)-2-oxoethyl]-6-[1-(4-tert-butylphenyl)-3-pyridin-2-yl-1H-pyrazol-5-yl]hexanamid;
N-({5-[1-(4-Isopropylphenyl)-3-pyridin-2-yl-1H-pyrazol-5-yl]thien-2-yl}carbonyl)tyrosinamid;
N-[2-Amino-1-(4-hydroxyphenyl)-2-oxoethyl]-4-[1-(4-tert-butylphenyl)-3-pyridin-2-yl-1H-pyrazol-5-yl]benzamid;
4-[1-(4-tert-Butylphenyl)-3-pyridin-2-yl-1H-pyrazol-5-yl]-N-[2-(4-hydroxyphenyl)ethyl]benzamid;
N-{3-[1-(4-tert-Butylbenzyl)-3-isochinolin-3-yl-1H-pyrazol-5-yl]benzoyl}tyrosinamid;
N-{5-[1-(4-tert-Butylphenyl)-3-pyridin-4-yl-1H-pyrazol-5-yl]pentanoyl}tyrosinamid;
N-{3-[1-(4-tert-Butylbenzyl)-3-pyridin-3-yl-1H-pyrazol-5-yl]benzoyl}tyrosinamid;
N-{3-[1-(4-tert-Butylphenyl)-3-isochinolin-3-yl-1H-pyrazol-5-yl]benzoyl}tyrosinamid;
N-[4-(1-Butyl-3-isochinolin-3-yl-1H-pyrazol-5-yl)benzoyl]tyrosinamid;
N-{3-[1-(4-tert-Butylbenzyl)-3-chinolin-3-yl-1H-pyrazol-5-yl]benzoyl}tyrosinamid;
N-{3-[3-Isochinolin-3-yl-1-(4-propylphenyl)-1H-pyrazol-5-yl]benzoyl}tyrosinamid;
N-{5-[1-(4-tert-Butylphenyl)-3-pyridin-3-yl-1H-pyrazol-5-yl]pentanoyl}tyrosinamid;
N-{3-[1-(4-tert-Butylphenyl)-3-pyridin-3-yl-1H-pyrazol-5-yl]benzoyl}tyrosinamid;
N-{4-[1-(4-tert-Butylphenyl)-3-pyridin-3-yl-1H-pyrazol-5-yl]benzoyl}tyrosinamid;
N-{5-[1-(4-tert-Butylbenzyl)-3-pyridin-4-yl-1H-pyrazol-5-yl]pentanoyl}tyrosinamid;
N-{3-[1-(4-tert-Butylbenzyl)-3-pyridin-4-yl-1H-pyrazol-5-yl]benzoyl}tyrosinamid;
N-{5-[1-(4-tert-Butylbenzyl)-3-pyridin-3-yl-1H-pyrazol-5-yl]pentanoyl}tyrosinamid;
N-{5-[1-(4-tert-Butylphenyl)-3-pyridin-4-yl-1H-pyrazol-5-yl]pentanoyl}tyrosinamid;
N-[3-(1-Butyl-3-isochinolin-3-yl-1H-pyrazol-5-yl)benzoyl]tyrosinamid;
N-(4-{[1-(4-tert-Butylphenyl)-3-pyridin-4-yl-1H-pyrazol-5-yl]amino}butanoyl)-tyrosinamid;
N-({3-[1-(4-tert-Butylphenyl)-3-pyridin-4-yl-1H-pyrazol-5-yl]propoxy}acetyl)tyrosinamid;
4-[2-({3-[1-(4-tert-Butylphenyl)-3-pyridin-4-yl-1H-pyrazol-5-yl]propyl}amino)ethyl]-phenol;
N-{3-[1-(4-tert-Butylphenyl)-3-pyridin-4-yl-1H-pyrazol-5-yl]propyl}tyrosinamid;
N-Acetyl-N-{3-[1-(4-tert-butylphenyl)-3-pyridin-4-yl-1H-pyrazol-5-yl]propyl}tyrosinamid;
3-[(4-{[1-(4-tert-Butylphenyl)-3-pyridin-4-yl-1H-pyrazol-5-yl]amino}butanoyl)amino]-4-(4-hydroxyphenyl)butanamid;
N-(4-{[1-(4-tert-Butylphenyl)-3-(1-methyl-1,2,3,6-tetrahydropyridin-4-yl)-1H-pyrazol-5-yl]amino}butanoyl)tyrosinamid;
N-(3-{[1-(4-tert-Butylphenyl)-3-pyridin-4-yl-1H-pyrazol-5-yl]amino}propyl)-tyrosinamid;
N-Acetyl-N-(3-{[1-(4-tert-butylphenyl)-3-pyridin-4-yl-1H-pyrazol-5-yl]amino}propyl)-tyrosinamid;
N-(4-{[1-(4-tert-Butylphenyl)-3-(1-oxidopyridin-4-yl)-1H-pyrazol-5-yl]amino}butanoyl)tyrosinamid;
4-{[1-(4-tert-Butylphenyl)-3-pyridin-4-yl-1H-pyrazol-5-yl]amino}-N-[2-(4-hydroxyphenyl)ethyl]butanamid;
4-{2-[(4-{[1-(4-tert-Butylphenyl)-3-pyridin-4-yl-1H-pyrazol-5-yl]amino}butyl)amino]ethyl}phenol;
N-(4-{[1-(4-tert-Butylphenyl)-3-pyridin-4-yl-1H-pyrazol-5-yl]amino}butanoyl)-N-(2-hydroxyethyl)tyrosinamid;
N-(4-{[1-(4-tert-Butylphenyl)-3-pyridin-4-yl-1H-pyrazol-5-yl]amino}butanoyl)-N-[2-(1-methylpyrrolidin-2-yl)ethyl]tyrosinamid;
N-(4{[1-(4-tert-Butylphenyl)-3-pyridin-3-yl-1H-pyrazol-5-yl]amino}butanoyl)tyrosinamid;
N-(4-{[1-(4-tert-Butylphenyl)-3-(1-methyl-1,2,5,6-tetrahydropyridin-3-yl)-1H-pyrazol-5-yl]amino}butanoyl)tyrosinamid;
(3R)-3-[(4-{[1-(4-tert-Butylphenyl)-3-pyridin-3-yl-1H-pyrazol-5-yl]amino}butanoyl)amino]-4-(4-hydroxyphenyl)butanamid;
N-(3-{[1-(4-tert-Butylphenyl)-3-pyridin-3-yl-1H-pyrazol-5-yl]amino}propyl)-tyrosinamid;
N-Acetyl-N-(3-{[1-(4-tert-butylphenyl)-3-pyridin-3-yl-1H-pyrazol-5-yl]amino}propyl)-tyrosinamid;
N-(4-{[1-(4-tert-Butylphenyl)-3-(1-oxidopyridin-3-yl)-1H-pyrazol-5-yl]amino}butanoyl)tyrosinamid;
4-{[1-(4-tert-Butylphenyl)-3-pyridin-3-yl-1H-pyrazol-5-yl]amino}-N-[2-(4-hydroxyphenyl)ethyl]butanamid;
4-{2-[(4-{[1-(4-tert-Butylphenyl)-3-pyridin-3-yl-1H-pyrazol-5-yl]amino}butyl)amino]-ethyl}phenol;
N-(4-{[1-(4-tert-Butylphenyl)-3-pyridin-3-yl-1H-pyrazol-5-yl]amino}butanoyl)-N-(2-hydroxyethyl)tyrosinamid;
N-(4-{[1-(4-tert-Butylphenyl)-3-pyridin-3-yl-1H-pyrazol-5-yl]amino}butanoyl)-N-[2-(1-methylpyrrolidin-2yl)ethyl]tyrosinamid;
N-(4-{[1-(4-tert-Butylphenyl)-3-pyridin-3-yl-1H-pyrazol-5-yl]oxy}butanoyl)-tyrosinamid;
oder ein optisches Isomer, Racemat oder Tautomer von irgendeinem davon oder ein pharmazeutisch verträgliches Salz von irgendeinem davon.

19. Verwendung nach einem der Ansprüche 1 bis 17, wobei die Verbindung 5-[2-(4-tert-Butylphenyl)-5-pyridin-3-yl-2H-pyrazol-3-yl]-pentansäure[1-carbamoyl-2-(4-hydroxy-phenyl)-ethyl]-amid oder 5-[2-(4-tert-Butyl-phenyl)-5-pyridin-4-yl-2H-pyrazol-3-yl]-pentansäure[1-carbamoyl-2-(4-hydroxy-phenyl)-ethyl]-amid ist.

20. Verwendung nach einem der Ansprüche 1 bis 19, wobei die behandelte Störung eine Spermatogenese-Störung ist.

21. Verwendung nach einem der Ansprüche 1 bis 19, wobei die Störung oder Krankheit ausgewählt ist aus AIDS-Wasting, Muskeldystrophie oder Kachexie.

## Revendications

1. Emploi d'un dérivé de pyrazole de formule IB suivante : 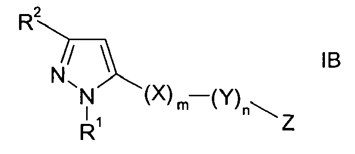 dans laquelle
- R¹ et R² représentent chacun, indépendamment, un groupe alkyle éventuellement porteur de substituant(s), alcényle éventuellement porteur de substituant(s), alcynyle éventuellement porteur de substituant(s), aryle carbocyclique éventuellement porteur de substituant(s), aralkyle éventuellement porteur de substituant(s), hétéroaromatique ou hétéroalicyclique, éventuellement porteur de substituant(s), ou hétéroaryl-alkyle ou hétéroalicyclyl-alkyle, éventuellement porteur de substituant(s) ;
- X représente un groupe alcanediyle éventuellement porteur de substituant(s), alcènediyle éventuellement porteur de substituant(s), alcyne-diyle éventuellement porteur de substituant(s), hétéroalcanediyle éventuellement porteur de substituant(s), hétéroalcènediyle éventuellement porteur de substituant(s), alicyclique éventuellement porteur de substituant(s), aryle carbocyclique éventuellement porteur de substituant(s), aralkyle éventuellement porteur de substituant(s), hétéroaromatique éventuellement porteur de substituant(s), hétéroalicyclique éventuellement porteur de substituant(s), hétéroaryl-alkyle éventuellement porteur de substituant(s), ou hétéroalicyclyl-alkyle éventuellement porteur de substituant(s) ;
- Y représente un groupe amino éventuellement porteur de substituant, méthanediyle éventuellement porteur de substituant(s), carbonyle ou sulfonyle ;
- Z représente un groupe alkylamino éventuellement porteur de substituant(s), ou un reste d'acide aminé ou de glycine ;
- et les indices m et n valent chacun, indépendamment, 0 ou 1 ;
ou d'un sel pharmacologiquement admissible d'un tel composé, pour la préparation d'une composition pharmaceutique conçue pour le traitement d'un état, d'un trouble ou d'une maladie impliquant un déficit en testostérone.

2. Emploi conforme à la revendication 1, pour lequel le dérivé de pyrazole a pour formule la suivante IC : 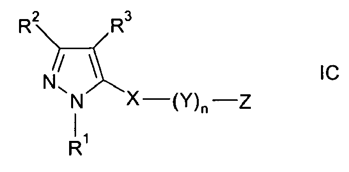 dans laquelle
- R¹ représente un groupe alkyle en C₁₋₆, aryl-alkyle, aryle, alkyl-aryle ou hétéroaryle ;
- R² représente un groupe aryle ou hétéroaryle ;
- R³ représente un atome d'hydrogène ;
- X représente un groupe aryle, hétéroaryle, alkyle, hétéroalkyle, amino-alkyle, aryl-hétéroalkyle, amino-alkyl-phényle ou hétérocycloalkyl-alkyle ;
- Y représente un groupe carbonyle;
- Z représente un groupe alkylamino à substituant(s), un groupe de formule -NH-CH(C(O)-NH₂)₀₋₁-(CH₂)₀₋₂-Phe-OH, -NH-CH(C(O)-NH₂)₀₋₁-(CH₂)₀₋₂-Phe-O-alkyle, -NH-CH(C(O)-NH₂)₀₋₁-(CH₂)₀₋₂-Phe-NH₂, -NH-CH(C(O)-NH₂)₀₋₁-(CH₂)₀₋₂-Phe-NH-C(O)CH₃ ou -NMe-CH(C(O)-NH-CH₃)-(CH₂)-Phe-OH, ou un reste de dérivé d'acide aminé lié au reste de la molécule par l'intermédiaire du groupe α-amino ;
- et l'indice n vaut 1.

3. Emploi conforme à la revendication 2, pour lequel :
- R¹ représente un groupe phényle, -phényl-butyl, -phényl-propyl,
- Phe-(CH₂)₂-CH, -benzyl-butyl, pyridyle, propyle ou hexyle ;
- R² représente un groupe pyridyle, isoquinolyle, quinolyle, furyle, diméthylamino-phényle ou pyrazinyle ;
- X représente un groupe phénylène, thiénylène, éthanediyle, propanediyle, pentanediyle, -CH₂-NH-, -CH₂-NH-CH₂-, -CH₂-NH-CH₂-CH₂-,
- CH₂-NH-Phe-, -CH₂-pipéridylène- ou -NH-(CH₂)₃- ;
- Z représente un reste de tyrosinamide, de thréoninamide, de sérinamide ou d'hydroxyméthyl-phénylalaninamide, ces restes de dérivés d'acides aminés étant liés au reste de la molécule par l'intermédiaire du groupe α-amino, ou un groupe de formule -NMe-CH(C(O)-NH-CH₃)-(CH₂)-Phe-OH, -NH-CH(C(O)-NH₂)-(CH₂)₂-Phe-OH, -NH-CH(PheOH)-C(O)-NH₂), -NH-(CH₂)₂-Phe-OH ou -NH-CH(C(O)-NH₂)-(CH₂)₂-Phe-O-t-Bu ;
- et l'indice n vaut 1.

4. Emploi conforme à la revendication 3, pour lequel R¹ représente un groupe 4-tertiobutyl-phényle, R² représente un groupe pyridyle, R³ représente un atome d'hydrogène, X représente un groupe propanediyle, Y représente un groupe carbonyle, Z représente un reste de tyrosinamide lié au reste de la molécule par l'intermédiaire du groupe α-amino, et l'indice n vaut 1.

5. Emploi conforme à la revendication 1, pour lequel le dérivé de pyrazole a pour formule la suivante ID : 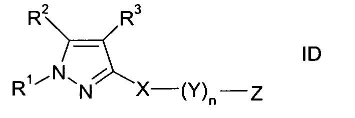 dans laquelle
- R¹ représente un groupe alkyle en C₁₋₆, aryl-alkyle, aryle, alkyl-aryle ou hétéroaryle ;
- R² représente un groupe aryle ou hétéroaryle ;
- R³ représente un atome d'hydrogène ;
- X représente un groupe aryle, hétéroaryle, alkyle, hétéroalkyle, amino-alkyle, aryl-alkyle, aryl-hétéroalkyle, amino-alkyl-phényle ou hétéro-cycloalkyl-alkyle ;
- Y représente un groupe carbonyle;
- Z représente un groupe alkylamino à substituant(s), un groupe de formule -NH-CH(C(O)-NH₂)₀₋₁-(CH₂)₀₋₂-Phe-OH₂ -NH-CH(C(O)-NH₂)₀₋₁-(CH₂)₀₋₂-Phe-O-alkyle, -NH-CH(C(O)-NH₂)₀₋₁-(CH₂)₀₋₂-Phe-NH₂, -NH-CH(C(O)-NH₂)₀₋₁-(CH₂)₀₋₂-Phe-NH-C(O)CH₃ ou -NMe-CH(C(O)-NH-CH₃)-(CH₂)-Phe-OH, ou un reste de dérivé d'acide aminé lié au reste de la molécule par l'intermédiaire du groupe α-amino ;
- et l'indice n vaut 1.

6. Emploi conforme à la revendication 5, pour lequel :
- R¹ représente un groupe phényle, -phényl-butyl, -phényl-propyl,
- Phe-(CH₂)₂-CH, -benzyl-butyl, pyridyle, propyle ou hexyle ;
- R² représente un groupe pyridyle, isoquinolyle, quinolyle, furyle, diméthylamino-phényle ou pyrazinyle ;
- X représente un groupe phénylène, thiénylène, éthanediyle, propanediyle, pentanediyle, -CH₂-NH-, -CH₂-NH-CH₂-, -CH₂-NH-CH₂-CH₂-,
- CH₂-NH-Phe-, -CH₂-pipéridylène- ou -NH-(CH₂)₃- ;
- Z représente un reste de tyrosinamide, de thréoninamide, de sérinamide ou d'hydroxyméthyl-phénylalaninamide, ces restes de dérivés d'acides aminés étant liés au reste de la molécule par l'intermédiaire du groupe α-amino, ou un groupe de formule -NMe-CH(C(O)-NH-CH₃)-(CH₂)-Phe-OH, -NH-CH(C(O)-NH₂)-(CH₂)₂-Phe-OH, -NH-CH(PheOH)-C(O)-NH₂), -NH-(CH₂)₂-Phe-OH ou -NH-CH(C(O)-NH₂)-(CH₂)₂-Phe-O-t-Bu ;
- et l'indice n vaut 1.

7. Emploi conforme à la revendication 6, pour lequel R¹ représente un groupe 4-tertiobutyl-phényle, R² représente un groupe pyridyle, R³ représente un atome d'hydrogène, X représente un groupe propanediyle, Y représente un groupe carbonyle, Z représente un reste de tyrosinamide lié au reste de la molécule par l'intermédiaire du groupe α-amino, et l'indice n vaut 1.

8. Emploi conforme à l'une des revendications 1 à 7, pour lequel R² représente autre chose qu'un atome d'hydrogène.

9. Emploi conforme à l'une des revendications 1 à 8, pour lequel R¹ représente un groupe alkyle éventuellement porteur de substituant(s), aryle carbocyclique éventuellement porteur de substituant(s), ou aralkyle éventuellement porteur de substituant(s).

10. Emploi conforme à l'une des revendications 1 à 8, pour lequel R² représente un groupe aryle carbocyclique éventuellement porteur de substituant(s), ou hétéroaromatique ou hétéroalicyclique, éventuellement porteur de substituant(s).

11. Emploi conforme à l'une des revendications 1 à 10, pour lequel X représente un groupe alcanediyle, éventuellement porteur de substituant(s).

12. Emploi conforme à l'une des revendications 1 à 10, pour lequel X représente un groupe aryle carbocyclique éventuellement porteur de substituant(s), ou hétéroaromatique éventuellement porteur de substituant(s).

13. Emploi conforme à l'une des revendications 1 à 10, pour lequel X représente un groupe aryle carbocyclique éventuellement porteur de substituant(s), ou hétéroalcanediyle éventuellement porteur de substituant(s).

14. Emploi conforme à l'une des revendications 1 à 11, pour lequel X représente un groupe -CH₂CH₂CH₂CH₂- ou -CH₂CH₂CH₂CH₂CH₂-.

15. Emploi conforme à l'une des revendications 1 à 10, pour lequel X représente un groupe phényle éventuellement porteur de substituant(s), phénoxy éventuellement porteur de substituant(s), ou phénylamino éventuellement porteur de substituant(s).

16. Emploi conforme à l'une des revendications 1 à 15, pour lequel Z représente un fragment phénolique.

17. Emploi conforme à l'une des revendications 1 à 15, pour lequel Z représente un reste de tyrosine.

18. Emploi conforme à l'une des revendications 1 à 17, pour lequel le dérivé est choisi dans l'ensemble formé par les suivants :
N-{5-[1-(4-tertiobutyl-phényl)-3-pyridin-2-yl-1H-pyrazol-5-yl]pentanoyl}tyrosinamide
N-{5-[1-(4-tertiobutyl-phényl)-3-pyridin-3-yl-1H-pyrazol-5-yl]pentanoyl}tyrosinamide
N-{5-[1-(4-tertiobutyl-phényl)-5-pyridin-3-yl-1H-pyrazol-3-yl]pentanoyl}tyrosinamide
N-{5-[1-(4-tertiobutyl-phényl)-3-pyridin-4-yl-1H-pyrazol-5-yl]pentanoyl}tyrosinamide
N-{5-[1-(4-tertiobutyl-phényl)-5-pyridin-4-yl-1H-pyrazol-3-yl]pentanoyl}tyrosinamide
N-{5-[1-(4-tertiobutyl-phényl)-3-pyridin-3-yl-1H-pyrazol-5-yl]pentanoyl}-N,N-diméthyl-tyrosinamide
N-{3-[1-(4-tertiobutyl-phényl)-5-pyridin-3-yl-1H-pyrazol-3-yl]propanoyl}tyrosinamide
N-{3-[1-(4-tertiobutyl-phényl)-3-pyridin-3-yl-1H-pyrazol-5-yl]propanoyl}tyrosinamide
N-[4-(1-butyl-3-isoquinoléin-3-yl-1H-pyrazol-5-yl)benzoyl]tyrosinamide
N-{5-[1-(4-isopropyl-phényl)-3-pyridin-3-yl-1H-pyrazol-5-yl]pentanoyl}tyrosinamide
N-{6-[1-(4-isopropyl-phényl)-3-pyridin-2-yl-1H-pyrazol-5-yl]hexanoyl}tyrosinamide
N-{6-[1-(4-tertiobutyl-phényl)-3-pyridin-2-yl-1H-pyrazol-5-yl]hexanoyl}-3-hydroxyphénylalaninamide
N-[1-aminocarbonyl-3-(4-hydroxyphényl)propyl]-5-[1-(4-tertiobutyl-phényl)-3-pyridin-2-yl-1H-pyrazol-5-yl]pentanamide
N-[5-(1-butyl-3-isoquinoléin-3-yl-1H-pyrazol-5-yl)pentanoyl]tyrosinamide
N-{6-[1-(4-tertiobutyl-phényl)-3-pyridin-2-yl-1H-pyrazol-5-yl]hexanoyl}sérinamide
N-{6-[1-(4-isopropyl-phényl)-3-pyridin-2-yl-1H-pyrazol-5-yl]hexanoyl}sérinamide
N-{6-[1-(4-isopropyl-phényl)-3-pyridin-2-yl-1H-pyrazol-5-yl]hexanoyl}thréoninamide
N-{5-[1-(4-isopropyl-phényl)-3-pyridin-2-yl-1H-pyrazol-5-yl]pentanoyl}tyrosinamide
6-[1-(4-tertiobutyl-phényl)-3-pyridin-2-yl-1H-pyrazol-5-yl]-N-[2-(4-hydroxyphényl)éthyl]hexanamide
N-{6-[1-(4-tertiobutyl-phényl)-3-pyridin-2-yl-1H-pyrazol-5-yl]hexanoyl}tyrosinamide
N-{5-[1-(4-tertiobutyl-phényl)-3-isoquinoléin-3-yl-1H-pyrazol-5-yl]pentanoyl}tyrosinamide
N-{6-[1-(4-tertiobutyl-phényl)-3-isoquinoléin-3-yl-1H-pyrazol-5-yl]hexanoyl}tyrosinamide
N-{6-[1-(4-tertiobutyl-phényl)-3-pyridin-2-yl-1H-pyrazol-5-yl]hexanoyl}-N-méthyl-tyrosinamide
N-{6-[1-(4-tertiobutyl-phényl)-3-pyridin-2-yl-1H-pyrazol-5-yl]hexanoyl}-4-(hydroxyméthyl)-phénylalaninamide
4-amino-N-{6-[1-(4-tertiobutyl-phényl)-3-pyridin-2-yl-1H-pyrazol-5-yl]-hexanoyl}phénylalaninamide
4-(acétylamino)-N-{6-[1-(4-tertiobutyl-phényl)-3-pyridin-2-yl-1H-pyrazol-5-yl]hexanoyl}phénylalaninamide
4-(aminocarbonyl)-N-{6-[1-(4-tertiobutyl-phényl)-3-pyridin-2-yl-1H-pyrazol-5-yl]hexanoyl}phénylalaninamide
N-butyl-N-{6-[1-(4-tertiobutyl-phényl)-3-pyridin-2-yl-1H-pyrazol-5-yl]-hexanoyl}tyrosinamide
N-{6-[1-(4-tertiobutyl-phényl)-3-pyridin-2-yl-1H-pyrazol-5-yl]hexanoyl}thréoninamide
N-[2-amino-1-(4-hydroxyphényl)-2-oxo-éthyl]-6-[1-(4-tertiobutyl-phényl)-3-pyridin-2-yl-1H-pyrazol-5-yl]hexanamide
N-({5-[1-(4-isopropyl-phényl)-3-pyridin-2-yl-1H-pyrazol-5-yl]thién-2-yl}carbonyl)tyrosinamide
N-[2-amino-1-(4-hydroxyphényl)-2-oxo-éthyl]-4-[1-(4-tertiobutyl-phényl)-3-pyridin-2-yl-1H-pyrazol-5-yl]benzamide
4-[1-(4-tertiobutyl-phényl)-3-pyridin-2-yl-1H-pyrazol-5-yl]-N-[2-(4-hydroxyphényl)éthyl]benzamide
N-{3-[1-(4-tertiobutyl-benzyl)-3-isoquinoléin-3-yl-1H-pyrazol-5-yl]benzoyl}tyrosinamide
N-{5-[1-(4-tertiobutyl-phényl)-3-pyridin-4-yl-1H-pyrazol-5-yl]pentanoyl}tyrosinamide
N-{3-[1-(4-tertiobutyl-benzyl)-3-pyridin-3-yl-1H-pyrazol-5-yl]benzoyl}tyrosinamide
N-{3-[1-(4-tertiobutyl-phényl)-3-isoquinoléin-3-yl-1H-pyrazol-5-yl]benzoyl}tyrosinamide
N-[4-(1-butyl-3-isoquinoléin-3-yl-1H-pyrazol-5-yl)benzoyl]tyrosinamide
N-{3-[1-(4-tertiobutyl-benzyl)-3-quinoléin-3-yl-1H-pyrazol-5-yl]benzoyl}tyrosinamide
N-{3-[3-isoquinoléin-3-yl-1-(4-propyl-phényl)1H-pyrazol-5-yl]benzoyl}tyrosinamide
N-{5-[1-(4-tertiobutyl-phényl)-3-pyridin-3-yl-1H-pyrazol-5-yl]pentanoyl}tyrosinamide
N-{3-[1-(4-tertiobutyl-phényl)-3-pyridin-3-yl-1H-pyrazol-5-yl]benzoyl}tyrosinamide
N-{4-[1-(4-tertiobutyl-phényl)-3-pyridin-3-yl-1H-pyrazol-5-yl]benzoyl}tyrosinamide
N-{5-[1-(4-tertiobutyl-benzyl)-3-pyridin-4-yl-1H-pyrazol-5-yl]pentanoyl}tyrosinamide
N- {3-[1-(4-tertiobutyl-benzyl)-3-pyridin-4-yl-1H-pyrazol-5-yl]benzoyl}tyrosinamide
N-{5-[1-(4-tertiobutyl-benzyl)-3-pyridin-3-yl-1H-pyrazol-5-yl]pentanoyl}tyrosinamide
N- {5-[1-(4-tertiobutyl-phényl)-3-pyridin-4-yl-1H-pyrazol-5-yl]pentanoyl}tyrosinamide
N-[3-(1-butyl-3-isoquinoléin-3-yl-1H-pyrazol-5-yl)benzoyl]tyrosinamide
N-(4-{[1-(4-tertiobutyl-phényl)-3-pyridin-4-yl-1H-pyrazol-5-yl]amino}butanoyl)tyrosinamide
N-((3-[1-(4-tertiobutyl-phényl)-3-pyridin-4-yl-1H-pyrazol-5-yl]propoxy)-acétyl)tyrosinamide
4-[2-({3-[1-(4-tertiobutyl-phényl)-3-pyridin-4-yl-1H-pyrazol-5-yl]propyl}amino)éthyl]phénol
N-{3-[1-(4-tertiobutyl-phényl)-3-pyridin-4-yl-1H-pyrazol-5-yl]propyl}tyrosinamide
N-acétyl-N-{3-[1-(4-tertiobutyl-phényl)-3-pyridin-4-yl-1H-pyrazol-5-yl]-propyl}tyrosinamide
3-[(4- ([1-(4-tertiobutyl-phényl)-3-pyridin-4-yl-1H-pyrazol-5-yl]amino)-butanoyl)amino]-4-(4-hydroxyphényl)butanamide
N-(4-{[1-(4-tertiobutyl-phényl)-3-(1-méthyl-1,2,3,6-tétrahydropyridin-4-yl)-1H-pyrazol-5-yl]amino}butanoyl)tyrosinamide
N-(3-{[1-(4-tertiobutyl-phényl)-3-pyridin-4-yl-1H-pyrazol-5-yl] amino}propyl)tyrosinamide
N-acétyl-N-(3-{[1-(4-tertiobutyl-phényl)-3-pyridin-4-yl-1H-pyrazol-5-yl]amino}propyl)tyrosinamide
N-(4-{[1-(4-tertiobutyl-phényl)-3-(1-oxydopyridin-4-yl)-1H-pyrazol-5-yl]-amino}butanoyl)tyrosinamide
4-{[1-(4-tertiobutyl-phényl)-3-pyridin-4-yl-1H-pyrazol-5-yl] amino}-N-[2-(4-hydroxyphényl)éthyl]butanamide
4-{2-[(4-{[1-(4-tertiobutyl-phényl)-3-pyridin-4-yl-1H-pyrazol-5-yl]amino}butyl)amino]éthyl}phénol
N-(4-{[1-(4-tertiobutyl-phényl)-3-pyridin-4-yl-1H-pyrazol-5-yl]amino}butanoyl)-N-(2-hydroxyéthyl)tyrosinamide
N-(4- {[1-(4-tertiobutyl-phényl)-3-pyridin-4-yl-1H-pyrazol-5-yl]amino}butanoyl)-N-[2-(1-méthyl-pyrrolidin-2-yl)éthyl]tyrosinamide
N-(4-{[1-(4-tertiobutyl-phényl)-3-pyridin-3-yl-1H-pyrazol-5-yl]amino}butanoyl)tyrosinamide
N-(4-{[1-(4-tertiobutyl-phényl)-3-(1-méthyl-1,2,5,6-tétrahydropyridin-3-yl)-1H-pyrazol-5-yl]amino}butanoyl)tyrosinamide
(3R)-3-[(4- {[1-(4-tertiobutyl-phényl)-3-pyridin-3-yl-1H-pyrazol-5-yl]-amino}butanoyl)amino]-4-(4-hydroxyphényl)butanamide
N-(3-{[1-(4-tertiobutyl-phényl)-3-pyridin-3-yl-1H-pyrazol-5-yl]amino}propyl)tyrosinamide
N-acétyl-N-(3-{[1-(4-tertiobutyl-phényl)-3-pyridin-3-yl-1H-pyrazol-5-yl]-amino}propyl)tyrosinamide
N-(4-{[1-(4-tertiobutyl-phényl)-3-(1-oxydopyridin-3-yl)-1H-pyrazol-5-yl]-amino }butanoyl)tyrosinamide
4-{[1-(4-tertiobutyl-phényl)-3-pyridin-3-yl-1H-pyrazol-5-yl]amino}-N-[2-(4-hydroxyphényl)éthyl]butanamide
4-{2-[(4-{[1-(4-tertiobutyl-phényl)-3-pyridin-3-yl-1H-pyrazol-5-yl]amino}butyl)amino]éthyl}phénol
N-(4-{[1-(4-tertiobutyl-phényl)-3-pyridin-3-yl-1H-pyrazol-5-yl]amino}butanoyl)-N-(2-hydroxyéthyl)tyrosinamide
N-(4-{[1-(4-tertiobutyl-phényl)-3-pyridin-3-yl-1H-pyrazol-5-yl]amino}butanoyl)-N-[2-(1-méthyl-pyrrolidin-2-yl)éthyl]tyrosinamide
N-(4-{[1-(4-tertiobutyl-phényl)-3-pyridin-3-yl-1H-pyrazol-5-yl]oxy}butanoyl)tyrosinamide
et les isomères optiques, racémiques et formes tautomères de tous ces composés, ainsi que leurs sels pharmacologiquement admissibles.

19. Emploi conforme à l'une des revendications 1 à 17, pour lequel le dérivé est du [1-carbamyl-2-(4-hydroxyphényl)éthyl]amide d'acide 5-[2-(4-tertiobutyl-phényl)-5-pyridin-3-yl-2H-pyrazol-3-yl]pentanoïque ou du [1-carbamyl-2-(4-hydroxyphényl)éthyl]amide d'acide 5-[2-(4-tertiobutyl-phényl)-5-pyridin-4-yl-2H-pyrazol-3-yl]pentanoïque.

20. Emploi conforme à l'une des revendications 1 à 19, le trouble à traiter étant un trouble de la spermatogenèse.

21. Emploi conforme à l'une des revendications 1 à 19, le trouble ou la maladie étant la fonte musculaire du sida, une dystrophie musculaire ou une cachexie.
